# EUROPEAN PATENT SPECIFICATION

(11) **EP 2 473 024 B1**
(45) Date of publication and mention of the grant of the patent: **12.04.2017**
(21) Application number: 10814450.2
(22) Date of filing: 01.09.2010
(51) Int. Cl.: A01H 5/00, C12Q 1/04, C12N 15/82, C12N 15/52

(54) **HERBICIDE-TOLERANT PLANTS**
HERBIZIDTOLERANTE PFLANZEN
VÉGÉTAUX TOLÉRANT LES HERBICIDES

(30) Priority: 01.09.2009 US 238906 P; 16.07.2010 US 365298 P
(43) Date of publication of application: 11.07.2012
(73) Proprietor: BASF Agrochemical Products, B.V., 6035 EA Arnhem (NL)
(72) Inventor: MANKIN, Scots, L., Raleigh, NC 27612 (US); WENCK, Allan, R., Durham, NC 27705 (US); HONG, Haiping, Cary, NC 27519 (US)
(74) Representative: Reitstötter Kinzebach
(86) International application number: PCT/US2010/047575
(87) International publication number: WO 2011/028836

(56) References cited:
- WO-A1-94/08016
- WO-A2-2008/089061
- US-A- 5 801 233
- US-A- 5 910 626
- US-A1- 2006 039 943
- US-A1- 2007 074 303
- US-A1- 2008 234 130
- DELYE CHRISTOPHE ET AL: "Molecular bases for sensitivity to acetyl-coenzyme a carboxylase inhibitors in black-grass", PLANT PHYSIOLOGY (ROCKVILLE), vol. 137, no. 3, March 2005 (2005-03), pages 794-806, XP002688872, ISSN: 0032-0889
- DELYE C ET AL: "SNP markers for black-grass (Alopecurus myosuroides Huds.) genotypes resistant to acetyl CoA-carboxylase inhibiting herbicides", THEORETICAL AND APPLIED GENETICS, SPRINGER, BERLIN, DE, vol. 104, no. 6-7, 1 May 2002 (2002-05-01) , pages 1114-1120, XP002344973, ISSN: 0040-5752, DOI: 10.1007/S00122-001-0852-6
- DELYE CHRISTOPHE ET AL: "PCR-based detection of resistance to acetyl-CoA carboxylase-inhibiting herbicides in black-grass (Alopecurus myosuroides Huds) and ryegrass (Lolium rigidum Gaud)", PEST MANAGEMENT SCIENCE, vol. 58, no. 5, May 2002 (2002-05), pages 474-478, XP002344971, ISSN: 1526-498X
- LIU YU-WEN ET AL: "In vitro induction of phosphinothricin tolerance in rice (Oryza sativa)", PLANT PROTECTION BULLETIN (TAICHUNG), vol. 47, no. 1, March 2005 (2005-03), pages 47-58, XP002688874, ISSN: 0577-750X
- FLORENTINA RADUCANU: "Preliminary results regarding in vitro screening for roundup resistance in sunflower", ROMANIAN AGRICULTURAL RESEARCH, no. 21 2004, pages 39-47, XP002688875, Retrieved from the Internet: URL:http://www.incda-fundulea.ro/rar/nr21/ 21.7.pdf [retrieved on 2012-12-06]
- MULWA RICHARD M S ET AL: "Biotechnology approaches to developing herbicide tolerance/selectivity in crops", AFRICAN JOURNAL OF BIOTECHNOLOGY, vol. 5, no. 5, March 2006 (2006-03), pages 396-404, ISSN: 1684-5315

## Description

### BACKGROUND OF THE INVENTION

Rice is one of the most important food crops in the world, particularly in Asia. Rice is a cereal grain produced by plants in the genus *Oryza.* The two most frequently cultivated species are *Oryza sativa* and *Oryza glaberrima*, with *O*. *sativa* being the most frequently cultivated domestic rice. In addition to the two domestic species, the genus *Oryza* contains more than 20 wild species. One of these wild species, *Oryza rufipogon* ("red rice" also referred to as *Oryza sativa* subsp. *rufipogon*) presents a major problem in commercial cultivation. Red rice produces red coated seeds. After harvest, rice seeds are milled to remove their hull. After milling, domestic rice is white while wild red rice appears discolored. The presence of discolored seeds reduces the value of the rice crop. Since red rice belongs to the same species as cultivated rice (*Oryza sativa*), their genetic makeup is very similar. This genetic similarity has made herbicidal control of red rice difficult.

Domestic rice tolerant to imidazolinone herbicides have been developed and are currently marketed under the tradename CLEARFIELD^{®}. Imidazolinone herbicides inhibit a plant's acetohydroxyacid synthase (AHAS) enzyme. When cultivating CLEARFIELD^{®} rice, it is possible to control red rice and other weeds by application of imidazolinone herbicides. Unfortunately, imidazolinone herbicide-tolerant red rice and weeds have developed.

Acetyl-Coenzyme A carboxylase (ACCase; EC 6.4.1.2) enzymes synthesize malonyl-CoA as the start of the de novo fatty acid synthesis pathway in plant chloroplasts. ACCase in grass chloroplasts is a multifunctional, nuclear-genome-encoded, very large, single polypeptide, transported into the plastid via an N-terminal transit peptide. The active form in grass chloroplasts is a homomeric protein, likely a homodimer.

ACCase enzymes in grasses are inhibited by three classes of herbicidal active ingredients. The two most prevalent classes are aryloxyphenoxypropanoates ("FOPs") and cyclohexanediones ("DIMs"). In addition to these two classes, a third class phenylpyrazolines ("DENs") has been described.

A number of ACCase-inhibitor-tolerance (AIT) mutations have been found in monocot weed species exhibiting tolerance toward one or more DIM or FOP herbicides. Further, an AIT maize has been marketed by BASF. All such mutations are found in the carboxyltransferase domain of the ACCase enzyme, and these appear to be located in a substrate binding pocket, altering access to the catalytic site. Single-nucleotide polymorphisms (SNPs) of *Alopecurus myosuroides* ACCase which are associated with sensitivity to certain ACCase inhibitors are described in Délye et al. (Plant Physiology 137:794-806, 2005) and Délye et al. (Theor Appl Genet 104:1114-1120, 2002). A PCR-based method for detecting isoleucine-to-leucine substitution in ACCase of *Lolium rigidum* Gaud and *Alopecurus myesuroides* Huds is described in Délye et al. (Pest Manag Sci 58:474-478, 2002). A sorghum line that exhibits tolerance to ACCase-inhibiting herbicides is described in WO 2008/089061. WO 94/08016 describes a herbicide-tolerant cyanobacterium ACCase.

DIMs and FOPs are important herbicides and it would be advantageous if rice could be provided that exhibits tolerance to these classes of herbicide. Currently, these classes of herbicide are of limited value in rice agriculture. In some cases, herbicide-tolerance-inducing mutations create a severe fitness penalty in the tolerant plant. Therefore, there remains a need in the art for an AIT rice that also exhibits no fitness penalty. This need and others are met by the present invention.

### BRIEF SUMMARY OF THE INVENTION

The present invention relates to a method for screening for herbicide-tolerant mutants of a monocot plastidic ACCase comprising: (a) providing plant cells or tissue from a monocot plant; and (b) culturing said plant cells or tissue in a tissue culture environment in the presence of cycloxydim; wherein the cycloxydim-tolerant plant cells or tissues recovered after step (b) are cycloxydim-tolerant due to a mutation in the monocot plastidic ACCase thereof, that was not present in the monocot plastidic ACCase prior to the culturing in step (b). The present invention further relates to a method for screening for herbicide-tolerant mutants of a monocot plastidic ACCase comprising: (a) providing monocot host cells or tissue deficient in an endogenous plastidic ACCase activity, said host cells or tissue comprising an exogenous monocot plastidic ACCase; and (b) culturing said host cells or tissue in a tissue culture environment in the presence of cycloxydim; wherein the exogenous monocot plastidic ACCase of cycloxydim-tolerant plant cells or tissues recovered after step (b) is cycloxydim-tolerant due to a mutation therein that was not present in the exogenous monocot plastidic ACCase prior to the culturing in step (b). Further, described herein are herbicide-tolerant plants and methods of producing and treating herbicide-tolerant plants. A rice plant described herein can be tolerant to at least one herbicide that inhibits acetyl-Coenzyme A carboxylase activity at levels of herbicide that would normally inhibit the growth of a rice plant. Typically, an herbicide-tolerant rice plant described herein expresses an acetyl-Coenzyme A carboxylase (ACCase) in which the amino acid sequence differs from an amino acid sequence of an acetyl-Coenzyme A carboxylase of a wild-type rice plant. By convention, mutations within monocot ACCase amino acid residues are typically referred to in reference to their position in the *Alopecurus myosuroides* (blackgrass) plastidic monomeric ACCase sequence (Genbank CAC84161.1) and denoted with an *(Am).* Examples of amino acid positions at which an acetyl-Coenzyme A carboxylase of an herbicide-tolerant plant described herein differs from the acetyl-Coenzyme A carboxylase of the corresponding wild-type plant include, but are not limited to, one or more of the following positions: 1,781(Am), 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081*(Am)*, 2,088*(Am)*, 2,095*(Am)*, 2,096*(Am)*, or 2,098(Am). Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. A rice plant described herein may express an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

Further, methods of producing herbicide-tolerant plants and plants produced by such methods are described herein. An example of a plant produced by said methods is an herbicide-tolerant rice plant which is tolerant to at least one herbicide that inhibits acetyl-Coenzyme A carboxylase activity at levels of herbicide that would normally inhibit the growth of said plant, wherein the herbicide-tolerant plant is produced by: a) obtaining cells from a plant that is not tolerant to the herbicide; b) contacting the cells with a medium comprising one or more acetyl-Coenzyme A carboxylase inhibitors; and c) generating an herbicide-tolerant plant from the cells. Herbicide-tolerant plants produced by methods describe herein include, but are not limited to, herbicide-tolerant plants generated by performing a), b) and c) above and progeny of a plant generated by performing a), b), and c) above. Cells used to practice methods of this type can be in the form of a callus.

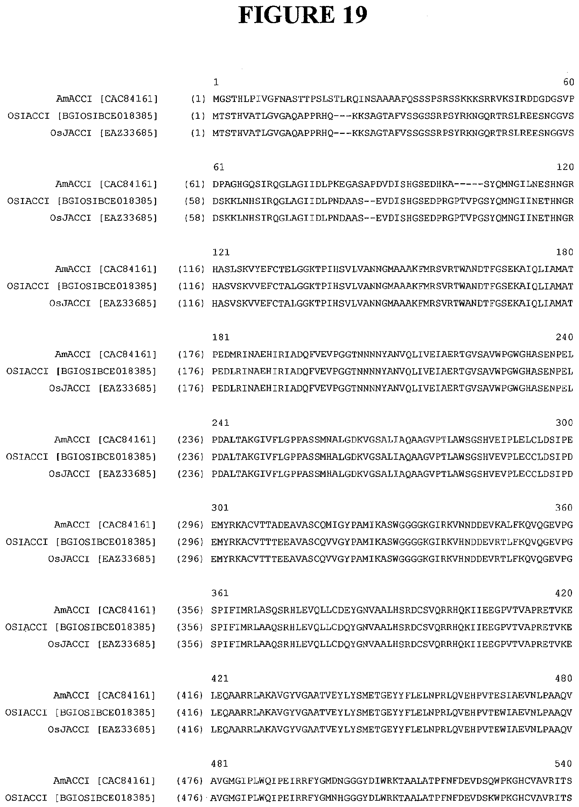

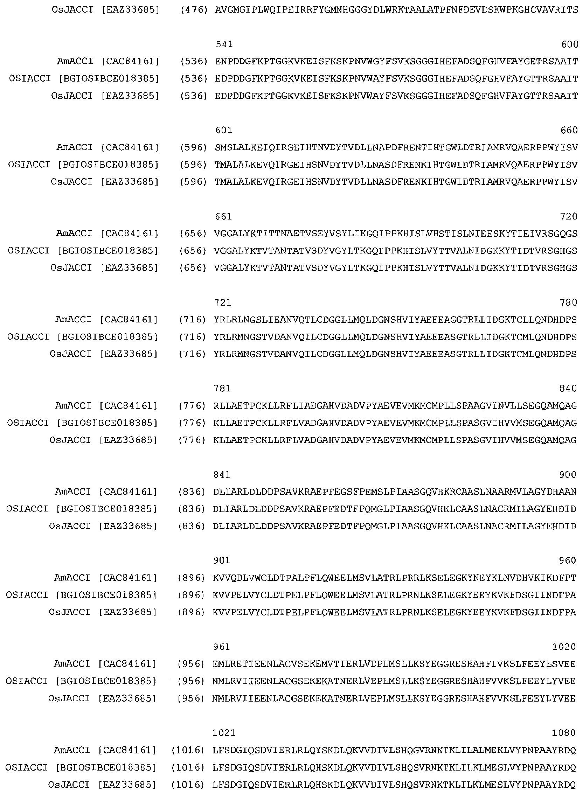

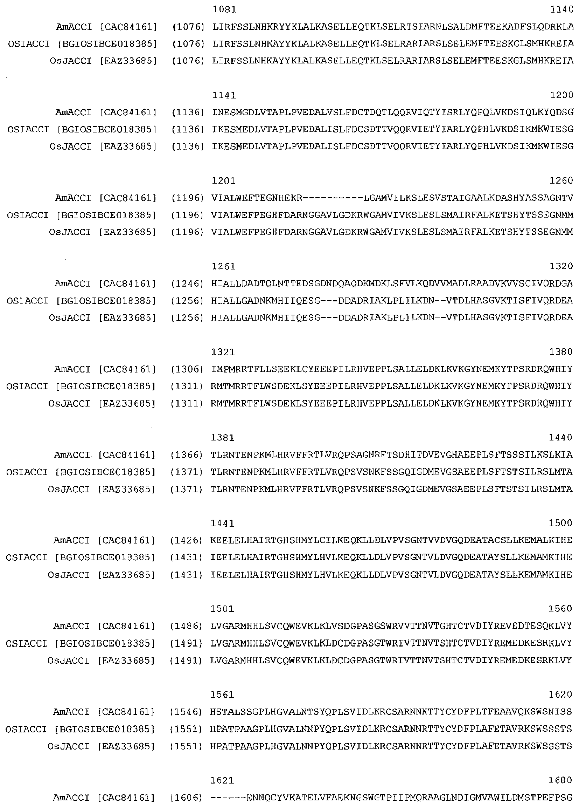

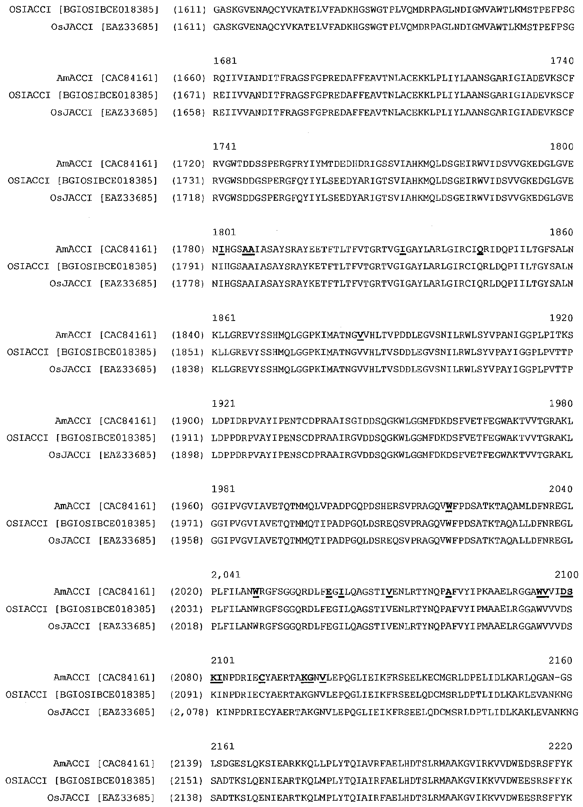

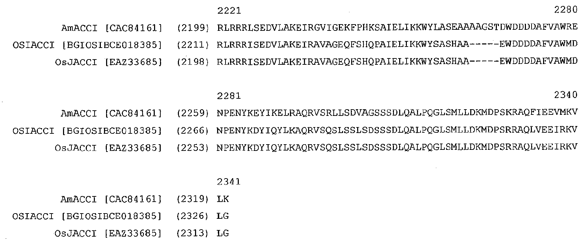

Further, plants expressing acetyl-Coenzyme A carboxylase enzymes comprising defined amino acid sequences are described herein. For example, such rice plant may be a rice plant, wherein one or more of the genomes of said rice plant encode a protein comprising a modified version of one or both of SEQ ID NOs: 2 and 3, wherein the sequence is modified such that the encoded protein comprises one or more of the following: the amino acid at position *1,781(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Figure 19 below provides an alignment of the *Alopecurus myosuroides* acetyl-Coenzyme A carboxylase sequence (SEQ ID NO:1), the *Oryza sativa* Indical acetyl-Coenzyme A carboxylase sequence (SEQ ID NO:2) and the *Oryza sativa* Japonica acetyl-Coenzyme A carboxylase sequence (SEQ ID NO:3) with examples of positions where the wild type sequences may differ with the herein described sequences indicated.

Further described herein are seeds deposited in an acceptable depository in accordance with the Budapest Treaty, cells derived from such seeds, plants grown from such seeds and cells derived from such plants, progeny of plants grown from such seed and cells derived from such progeny. The growth of plants produced from deposited seed and progeny of such plants will typically be tolerant to acetyl-Coenzyme A carboxylase-inhibiting herbicides at levels of herbicide that would normally inhibit the growth of a corresponding wild-type plant. A rice plant grown from a seed produced from a plant of any one of lines OsHPHI2, OsARWI1 OsARWI3, OsARWI8, or OsHPHN1, a representative sample of seed of each line having been deposited with American Type Culture Collection (ATCC) under Patent Deposit Designation Number PTA-10267, PTA-10568, PTA-10569, PTA-10570, or PTA-10571, respectively, is also described herein. Further, mutants, recombinants, and/or genetically engineered derivatives are described herein which are prepared from a plant of any one of lines OsHPHI2, OsARWI1 OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively, as well as any progeny of the plant grown or bred from a plant of any one of lines OsHPHI2, OsARWI1 OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively, wherein said plants or progeny have the herbicide tolerance characteristics of the plant grown from a a plant of any one of lines OsHPHI2, OsARWI1 OsARWI3, OsARWI8, or OsHPHN1, a representative sample of seed of each line having been deposited with ATCC under Patent Deposit Designation Number PTA-10267, PTA-10568, PTA-10569, PTA-10570, or PTA-10571, respectively. Further, cells cultured from such seeds and plants and their progeny produced from the cultured cells are described herein.

An herbicide-tolerant plant described herein may be a member of the species *O*. *sativa.* Herbicide-tolerant plants described herein are typically tolerant to aryloxyphenoxypropionate herbicides, cyclohexanedione herbicides, phenylpyrazoline herbicides or combinations thereof at levels of herbicide that would normally inhibit the growth of a corresponding wild-type plant, for example, a rice plant. An herbicide-tolerant plant is described herein that is not a GMO-plant. Further, an herbicide-tolerant plant that is mutagenized, is described herein, for example, a mutagenized rice plant. Further, cells derived from the plants and seeds of the herbicide-tolerant plants described above are described herein.

Further, methods for controlling growth of weeds are described herein, for example a method of controlling growth of weeds in vicinity to rice plants. Such methods may comprise applying to the weeds and rice plants an amount of an acetyl-Coenzyme A carboxylase-inhibiting herbicide that inhibits naturally occurring acetyl-Coenzyme A carboxylase activity, wherein said rice plants comprise altered acetyl-Coenzyme A carboxylase activity such that said rice plants are tolerant to the applied amount of herbicide. Methods described herein may be practiced with any herbicide that interferes with acetyl-Coenzyme A carboxylase activity including, but not limited to, aryloxyphenoxypropionate herbicides, cyclohexanedione herbicides, phenylpyrazoline herbicides or combinations thereof.

Further, a method for controlling growth of weeds in vicinity to rice plants is described herein. One example of such methods may comprise applying one or more herbicides to the weeds and to the rice plants at levels of herbicide that would normally inhibit the growth of a rice plant, wherein at least one herbicide inhibits acetyl-Coenzyme A carboxylase activity. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

Further, a method for controlling growth of weeds is described herein. One example of such methods may comprise (a) crossing an herbicide-tolerant rice plant with other rice germplasm, and harvesting the resulting hybrid rice seed; (b) planting the hybrid rice seed; and (c) applying one or more acetyl-Coenzyme A carboxylase-inhibiting herbicides to the hybrid rice and to the weeds in vicinity to the hybrid rice at levels of herbicide that would normally inhibit the growth of a rice plant. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

Further, a method for selecting herbicide-tolerant rice plants is described herein. One example of such methods may comprise (a) crossing an herbicide-tolerant rice plant with other rice germplasm, and harvesting the resulting hybrid rice seed; (b) planting the hybrid rice seed; (c) applying one or more herbicides to the hybrid rice at levels of herbicide that would normally inhibit the growth of a rice plant, wherein at least one of the herbicides inhibits acetyl-Coenzyme A carboxylase; and (d) harvesting seeds from the rice plants to which herbicide has been applied. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

Further, a method for growing herbicide-tolerant rice plants is described herein. One example of such a method comprises (a) planting rice seeds; (b) allowing the rice seeds to sprout; (c) applying one or more herbicides to the rice sprouts at levels of herbicide that would normally inhibit the growth of a rice plant, wherein at least one of the herbicides inhibits acetyl-Coenzyme A carboxylase. Such methods may be practiced with any herbicide that inhibits acetyl-Coenzyme A carboxylase activity. Suitable examples of herbicides that may be used in the practice of methods of controlling weeds include, but are not limited to, aryloxyphenoxypropionate herbicides, cyclohexanedione herbicides, phenylpyrazoline herbicides or combinations thereof.

Further, a seed of an herbicide-tolerant rice plant is described herein. Such seed may be used to grow herbicide-tolerant rice plants, wherein a plant grown from the seed is tolerant to at least one herbicide that inhibits acetyl-Coenzyme A carboxylase activity at levels of herbicide that would normally inhibit the growth of a rice plant. Examples of herbicides to which plants grown from seeds described herein would be tolerant include but are not limited to, aryloxyphenoxypropionate herbicides, cyclohexanedione herbicides, phenylpyrazoline herbicides or combinations thereof.

Further, a seed of a rice plant is described herein, wherein a plant grown from the seed expresses an acetyl-Coenzyme A carboxylase (ACCase) in which the amino acid sequence differs from an amino acid sequence of an acetyl-Coenzyme A carboxylase of a wild-type rice plant at one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041(Am), 2,049*(Am)*, 2,059*(Am)*, 2,074*(Am),* 2,075*(Am)*, 2,078*(Am)*, 2,079*(Am)*, 2,080(Am), 2,081*(Am)*, 2,088*(Am)*, 2,095*(Am)*, 2,096*(Am)*, or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position *2,081(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. A plant grown from the seed may express an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

Further, seeds of specific herbicide-tolerant cultivars are described herein. One example of such seeds is a seed of rice cultivar Indical, wherein a representative sample of seed of said cultivar was deposited under ATCC Accession No. PTA-10267, PTA-10568, PTA-10569, or PTA-10570. Another example of such seeds are those of an herbicide-tolerant Nipponbare cultivar, wherein a representative sample of seed of said cultivar was deposited under ATCC Accession No. PTA-10571. Further, a rice plant, or a part thereof, produced by growing the seeds as well as a tissue culture of cells produced from the seed are described herein. Tissue cultures of cells may be produced from a seed directly or from a part of a plant grown from a seed, for example, from the leaves, pollen, embryos, cotyledons, hypocotyls, meristematic cells, roots, root tips, pistils, anthers, flowers and/or stems. Further, plants and their progeny that have been generated from tissue cultures of cells are described herein. Such plants will typically have all the morphological and physiological characteristics of cultivar Indical.

Further, methods for producing rice seed are described herein. Such methods may comprise crossing an herbicide-tolerant rice plant with other rice germplasm; and harvesting the resulting hybrid rice seed, wherein the herbicide-tolerant rice plant is tolerant to aryloxyphenoxypropionate herbicides, cyclohexanedione herbicides, phenylpyrazoline herbicides or combinations thereof at levels of herbicide that would normally inhibit the growth of a rice plant.

Further, methods of producing F1 hybrid rice seed are described herein. Such methods may comprise crossing an herbicide-tolerant rice plant with a different rice plant; and harvesting the resultant F1 hybrid rice seed, wherein the herbicide-tolerant rice plant is tolerant to aryloxyphenoxypropionate herbicides, cyclohexanedione herbicides, phenylpyrazoline herbicides or combinations thereof at levels of herbicide that would normally inhibit the growth of a rice plant.

Further, methods of producing F1 hybrid plants are described herein. Such methods may comprise crossing an herbicide-tolerant plant with a different plant; and harvesting the resultant F1 hybrid seed and growing the resultant F1 hybrid plant, wherein the herbicide-tolerant plant is tolerant to aryloxyphenoxypropionate herbicides, cyclohexanedione herbicides, phenylpyrazoline herbicides or combinations thereof at levels of herbicide that would normally inhibit the growth of a plant.

Further, methods of producing herbicide-tolerant rice plants that may also comprise a transgene are described herein. One example of such a method may comprise transforming a cell of a rice plant with a transgene, wherein the transgene encodes an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods described herein, for example, the cell may be in the form of a callus. The transgene may comprise a nucleic acid sequence encoding an amino acid sequence comprising a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Further, plants produced by such methods are described herein. Another example of a method of producing an herbicide-tolerant plant comprising a transgene may comprise transforming a cell of a rice plant with a transgene encoding an enzyme that confers herbicide tolerance, wherein the cell was produced from a rice plant or seed thereof expressing an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods described herein, for example, the cell may be in the form of a callus. Further, herbicide-tolerant plants produced by such methods are described herein.

Further, methods of producing recombinant plants are described herein. An example of a method for producing a recombinant rice plant may comprise transforming a cell of a rice plant with a transgene, wherein the cell was produced from a rice plant expressing an acetyl-Coenzyme A carboxylase enzyme that confers tolerance to at least one herbicide is selected from the group consisting of aryloxyphenoxypropionate herbicides, cyclohexanedione herbicides, phenylpyrazoline herbicides or combinations thereof. Any suitable cell may be used in the practice of the methods described herein, for example, the cell may be in the form of a callus. A transgene for use in the methods described herein may comprise any desired nucleic acid sequence, for example, the transgene may encode a protein. In one example, the transgene may encode an enzyme, for example, an enzyme that modifies fatty acid metabolism and/or carbohydrate metabolism. Examples of suitable enzymes include but are not limited to, fructosyltransferase, levansucrase, alpha-amylase, invertase and starch branching enzyme or encoding an antisense of stearyl-ACP desaturase. Further described herein are recombinant plants produced by methods as described herein.

Methods as described herein may be used to produce a plant, e.g., a rice plant, having any desired traits. An example of such a method may comprise: (a) crossing a rice plant that is tolerant to aryloxyphenoxypropionate herbicides, cyclohexanedione herbicides, phenylpyrazoline herbicides or combinations thereof at levels of herbicide that would normally inhibit the growth of a rice plant with a plant of another rice cultivar that comprises the desired trait to produce progeny plants; (b) selecting one or more progeny plants that have the desired trait to produce selected progeny plants; (c) crossing the selected progeny plants with the herbicide-tolerant plants to produce backcross progeny plants; (d) selecting for backcross progeny plants that have the desired trait and herbicide tolerance; and (e) repeating steps (c) and (d) three or more times in succession to produce selected fourth or higher backcross progeny plants that comprise the desired trait and herbicide tolerance. Any desired trait may be introduced using the methods described herein. Examples of traits that may be desired include, but are not limited to, male sterility, herbicide tolerance, drought tolerance insect resistance, modified fatty acid metabolism, modified carbohydrate metabolism and resistance to bacterial disease, fungal disease or viral disease. An example of a method for producing a male sterile rice plant may comprise transforming a rice plant tolerant to at least one herbicide that inhibits acetyl-Coenzyme A carboxylase activity at levels of herbicide that would normally inhibit the growth of a rice plant with a nucleic acid molecule that confers male sterility. Further, male sterile plants produced by such methods are described herein.

Further, compositions comprising plant cells, for example, cells from a rice plant are described herein. One example of such a composition comprises one or more cells of a rice plant; and an aqueous medium, wherein the medium comprises a compound that inhibits acetyl-Coenzyme A carboxylase activity. The cells may be derived from a rice plant tolerant to aryloxyphenoxypropionate herbicides, cyclohexanedione herbicides, phenylpyrazoline herbicides or combinations thereof at levels of herbicide that would normally inhibit the growth of a rice plant. Any compound that inhibits acetyl-Coenzyme A carboxylase activity may be used in the compositionsdescribed herein, for example, one or more of aryloxyphenoxypropionate herbicides, cyclohexanedione herbicides, phenylpyrazoline herbicides and combinations thereof.

Further, nucleic acid molecules encoding all or a portion of an acetyl-Coenzyme A carboxylase enzyme are described herein, for example a recombinant, mutagenized, synthetic, and/or isolated nucleic acid molecule encoding a rice acetyl-Coenzyme A carboxylase (ACCase) in which the amino acid sequence differs from an amino acid sequence of an acetyl-Coenzyme A carboxylase of a wild-type rice plant at one or more of the following positions: 1,781*(Am)*, 1,785*(Am)*, 1,786*(Am)*, 1,811*(Am)*, 1,824*(Am)*, 1,864(Am), 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am),* 2,080*(Am)*, 2,081*(Am)*, 2,088*(Am)*, 2,095*(Am)*, 2,096*(Am)*, or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027*(Am)* is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. A nucleic acid molecule as described herein may encode an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine. Further, a recombinant, mutagenized, synthetic, and/or isolated nuceleic acid encoding a protein comprising all or a portion of a modified version of one or both of SEQ ID NOs: 2 and 3 is described herein, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is Arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

Further, an herbicide-tolerant, BEP clade plant is described herein. Typically such a plant is one having increased tolerance to an ACCase-inhibitor (ACCI) as compared to a wild-type variety of the plant. Such plants may be produced by a process comprising either:
(I) the steps of
   (a) providing BEP clade plant cells having a first, zero or non-zero level of ACCI tolerance;
   (b) growing the cells in contact with a medium to form a cell culture;
   (c) contacting cells of said culture with an ACCI;
   (d) growing ACCI-contacted cells from step (c) to form a culture containing cells having a level of ACCI tolerance greater than the first level of step (a); and
   (e) generating, from ACCI-tolerant cells of step (d), a plant having a level of ACCI tolerance greater than that of a wild-type variety of the plant; or
(II) the steps of
   (f) providing a first, herbicide-tolerant, BEP clade plant having increased tolerance to an ACCase-inhibitor (ACCI) as compared to a wild-type variety of the plant, said herbicide-tolerant plant having been produced by a process comprising steps (a)-(e); and
   (g) producing from the first plant a second, herbicide-tolerant, BEP clade plant that retains the increased herbicide tolerance characteristics of the first plant; thereby obtaining an herbicide-tolerant, BEP clade plant.

The herbicide-tolerant BEP clade plant described herein may be a BET subclade plant.

The herbicide-tolerant BET subclade plant described herein may be a BET crop plant.

The herbicide-tolerant plant described herein may be a member of the Bambusoideae - Ehrhartoideae subclade. Any suitable medium for growing plant cells may be used in the practice of the invention. In some embodiments, the medium may comprise a mutagen while in other embodiments the medium does not comprise a mutagen. In some embodiments, the herbicide-tolerant plant may be a member of the subfamily Ehrhartoideae. Any suitable cells may be used in the practice of the methods of the invention, for example, the cells may be in the form of a callus. In some embodiments, the herbicide-tolerant may be a member of the genus *Oryza,* for example, may be a member of the species *O*. *sativa.*

Further, herbicide-tolerant BEP clade plants produced by the above method ar described herein. Such herbicide-tolerant plants may express an acetyl-Coenzyme A carboxylase (ACCase) in which the amino acid sequence differs from an amino acid sequence of an acetyl-Coenzyme A carboxylase of a corresponding wild-type BEP clade plant at one or more of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am)*, 2,079*(Am)*, 2,080*(Am)*, 2,081(Am), 2,088*(Am)*, 2,095*(Am)*, 2,096*(Am)*, or 2,098*(Am)*. Examples of differences at these amino acid positions include, but are not limited to, one or more of the following: the amino acid at position 1,781*(Am)* is other than isoleucine; the amino acid at position 1,785*(Am)* is other than alanine; the amino acid at position 1,786*(Am)* is other than alanine; the amino acid at position 1,811*(Am)* is other than isoleucine; the amino acid position 1,824*(Am)* is other than glutamine; the amino acid position 1,864*(Am)* is other than valine; the amino acid at position 1,999*(Am)* is other than tryptophan; the amino acid at position 2,027(Am) is other than tryptophan; the amino acid position 2,039*(Am)* is other than glutamic acid; the amino acid at position 2,041*(Am)* is other than isoleucine; the amino acid at position 2,049*(Am)* is other than valine; the amino acid position 2,059*(Am)* is other than an alanine; the amino acid at position 2,074*(Am)* is other than tryptophan; the amino acid at position 2,075*(Am)* is other than valine; the amino acid at position 2,078*(Am)* is other than aspartate; the amino acid position at position 2,079*(Am)* is other than serine; the amino acid at position 2,080*(Am)* is other than lysine; the amino acid position at position 2,081*(Am)* is other than isoleucine; the amino acid at position 2,088*(Am)* is other than cysteine; the amino acid at position 2,095*(Am)* is other than lysine; the amino acid at position 2,096*(Am)* is other than glycine; or the amino acid at position 2,098*(Am)* is other than valine. The an herbicide-tolerant BEP clade plant may expresses an acetyl-Coenzyme A carboxylase enzyme comprising an amino acid sequence that comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine; the amino acid at position 2,039*(Am)* is glycine; the amino acid at position 2,041*(Am)* is asparagine; the amino acid at position 2049*(Am)* is phenylalanine; the amino acid at position 2,059*(Am)* is valine; the amino acid at position 2,074*(Am)* is leucine; the amino acid at position 2,075*(Am)* is leucine, isoleucine or methionine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is Arginine, or tryptophan; the amino acid at position 2,095*(Am)* is glutamic acid; the amino acid at position 2,096*(Am)* is alanine, or serine; or the amino acid at position 2,098*(Am)* is alanine, glycine, proline, histidine, or serine.

In one case, the rice plants may be tolerant to ACCase inhibitors by virtue of having only one substitution in its plastidic ACCase as compared to the corresponding wild-type ACCase. In yet another case, the rice plants may be tolerant to ACCase inhibitors by virtue of having two or more substitutions in its plastidic ACCase as compared to the corresponding wild-type ACCase.

In one case , the rice plants may be tolerant to ACCase inhibitors, by virtue of having two or more substitution in its plastidic ACCase as compared to the corresponding wild-type ACCase, wherein the substitutions are at amino acid positions selected from the group consisting of 1,781*(Am)*, 1,785*(Am)*, 1,786*(Am)*, 1,811*(Am)*, 1,824*(Am)*, 1,864*(Am)*, 1,999(Am), 2,027*(Am)*, 2,039*(Am)*, 2,041*(Am)*, 2,049*(Am)*, 2,059*(Am)*, 2,074*(Am)*, 2,075(*Am*), 2,078*(Am)*, 2,079*(Am)*, 2,080*(Am)*, 2,081*(Am)*, 2,088*(Am)*, 2,095*(Am)*, 2,096(*Am*), or 2,098*(Am)*.

The rice plants described herein may comprise plastidic ACCase that is not transgenic. Alternatively, the plants may comprise a rice plastidic ACCase that is transgenic.

A method for controlling growth of weeds within the vicinity of a rice plant as described herein, may comprise applying to the weeds and rice plants an amount of an acetyl-Coenzyme A carboxylase-inhibiting herbicide that inhibits naturally occurring acetyl-Coenzyme A carboxylase activity, wherein said rice plants comprise altered acetyl-Coenzyme A carboxylase activity such that said rice plants are tolerant to the applied amount of herbicide.

Methods for producing seed may comprise: (i) planting seed produced from a plant described herein, (ii) growing plants from the seed and (ii) harvesting seed from the plants.

Further, herbicide-tolerant BEP clade plants are described herein which are produced by the process of (a) crossing or back-crossing a plant grown from a seed of an herbicide-tolerant BEP clade plant produced as described above with other germplasm; (b) growing the plants resulting from said crossing or back-crossing in the presence of at least one herbicide that normally inhibits acetyl-Coenzyme A carboxylase, at levels of the herbicide that would normally inhibit the growth of a plant; and (c) selecting for further propagation plants resulting from said crossing or back-crossing, wherein the plants selected are plants that grow without significant injury in the presence of the herbicide.

Further, a recombinant, mutagenized, synthetic, and/or isolated nucleic acid molecule is described herein that comprises a nucleotide sequence encoding a mutagenized acetyl-Coenzyme A carboxylase of a plant in the BEP clade of the Family Poaceae, in which the amino acid sequence of the mutagenized acetyl-Coenzyme A carboxylase differs from an amino acid sequence of an acetyl-Coenzyme A carboxylase of the corresponding wild-type plant at one or more of the following positions: 1,781*(Am)*, 1,785*(Am),* 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049(*Am*), 2,059*(Am)*, 2,074*(Am)*, 2,075*(Am)*, 2,078*(Am)*, 2,079*(Am)*, 2,080*(Am)*, 2,081(*Am*), 2,088*(Am)*, 2,095*(Am)*, 2,096*(Am)*, or 2,098*(Am)*. Such a nucleic acid molecule may b produced by a process comprising either:
(I) the steps of
   (a) providing BEP clade plant cells having a first, zero or non-zero level of ACCase-inhibitor (ACCI) tolerance;
   (b) growing the cells in contact with a medium to form a cell culture;
   (c) contacting cells of said culture with an ACCI;
   (d) growing ACCI-contacted cells from step (c) to form a culture containing cells having a level of ACCI tolerance greater than the first level of step (a); and
   (e) generating, from ACCI-tolerant cells of step (d), a plant having a level of ACCI tolerance greater than that of a wild-type variety of the plant; or
(II) the steps of
   (f) providing a first, herbicide-tolerant, BEP clade plant having increased tolerance to an ACCase-inhibitor (ACCI) as compared to a wild-type variety of the plant, said herbicide-tolerant plant having been produced by a process comprising steps (a)-(e); and
   (g) producing from the first plant a second, herbicide-tolerant, BEP clade plant that retains the increased herbicide tolerance characteristics of the first plant;
thereby obtaining an herbicide-tolerant, BEP clade plant; and isolating a nucleic acid from the herbicide-tolerant BEP clade plant.

In one embodiment, the invention encompasses methods of screening, isolating, identifying, and/or characterizing herbicide tolerant mutations in monocot plastidic ACCases. In one embodiment, the invention encompasses the use of calli, or plant cell lines. In other embodiments, the invention encompasses performing the culturing of plant material or cells in a tissue culture environment. In yet other embodiments, the invention encompasses the presence of a nylon membrane in the tissue culture environment. In other embodiments, the tissue culture environment comprises liquid phase media while in other embodiments, the environment comprises semi-solid media. In yet other embodiments, the invention encompasses culturing plant material in the presence of herbicide (e.g., cycloxydim) in liquid media followed by culturing in semi-solid media with herbicide. In yet other embodiments, the invention encompasses culturing plant material in the presence of herbicide in semi-solid media followed by culturing in liquid media with herbicide.

In some embodiments, the invention encompasses the direct application of a lethal dose of herbicide (e.g., cycloxydim). In other embodiment, the invention encompasses the step-wise increase in herbicide dose, starting with a sub-lethal dose. In other embodiments, the invention encompasses at least one, at least two, at least three, at least four, at least five, at least six, at least seven, at least eight, or more herbicides in one step, or concurrently.

In other embodiments, the mutational frequency is determined by the number of mutant herbicide-tolerant clones as a fraction of the number of the individual calli used in the experiment. In some embodiments, the invention encompasses a mutational frequency of at least 0.03% or higher. In some embodiments, the invention encompasses mutational frequencies of at least 0.03%, at least 0.05%, at least 0.10%, at least 0.15%, at least 0.20%, at least 0.25%, at least 0.30%, at least 0.35%, at least 0.40% or higher. In other embodiments, the invention encompasses mutational frequencies that are at least 2 fold, at least 3 fold, at least 4 fold, at least 5 fold, at least 6 fold, at least 7 fold, at least 8 fold, at least 9 fold, at least 10 fold or higher than other methods of screening, isolating, identifying, and/or characterizing herbicide tolerant mutations in monocot plastidic ACCases.

In some embodiments, the methods of the invention encompass identifying the herbicide tolerant mutation(s) in the ACCase. In further embodiments, the invention comprises recapitulating the herbicide tolerant mutation(s) in monocot plant cells.

Further, an isolated cell or tissue said cell or tissue of plant origin is described herein that has: a) a deficiency in ACCase activity derived from a host ACCase (i.e., endogenous) gene; and b) an ACCase activity from a monocot-derived plastidic ACCase gene.

### Monocot sources of ACCase

Further described herein are plastidic ACCases or portions thereof from the monocot family of plants as described herein.

In other embodiments, the invention encompasses screening for herbicide-tolerant mutants of monocot plastidic ACCase in host plant cells.

In other embodiments, the invention encompasses the use of prepared host cells to screen for herbicide-tolerant mutants of monocot plastidic ACCase. In some embodiments, the invention provides a host cell which is devoid of plastidic ACCase activity. In other embodiments, the host cells used in the invention express a monocot plastidic ACCase which is herbicide sensitive.

In other embodiments, methods of the invention comprise host cells deficient in ACCase activity due to a mutation of the genomic plastidic ACCase gene which include a single point mutation, multiple point mutations, a partial deletion, a partial knockout, a complete deletion and a complete knockout. In another embodiment, genomic plastidic ACCase activity is reduced or ablated using other molecular biology techniques such as RNAi, siRNA or antisense RNA. Such molecular biology techniques are well known in the art. In yet other embodiments, genomic ACCase derived activity may be reduced or ablated by a metabolic inhibitor of ACCase.

In some embodiments, the host cell is a monocot plant host cell.

Further, a method of making a transgenic plant cell is described herein that comprises: a) isolating a cell having a monocot plant origin; b) inactivating at least one copy of a genomic ACCase gene; c) providing a monocot-derived plastidic ACCase gene to said cell; d) isolating the cell comprising the monocot-derived plastidic ACCase gene; and optionally; e) inactivating at least additional copy of a genomic ACCase gene and wherein said cell is deficient in ACCase activity provided by the genomic ACCase gene.

The cycloxydim-tolerant mutational frequency may be greater than 0.03 %.

In one embodiment, the present invention provides a method for screening, wherein cycloxydim-tolerant plant cells or tissues are also tolerant to other ACCase inhibitors.

In one embodiment, the present invention provides a method for screening, wherein the cycloxydim-tolerant plant cells or tissues comprise only one mutation not present in the monocot plastidic ACCase prior to culturing in the presence of the herbicide.

In one embodiment, the present invention provides a method for screening, wherein the cycloxydim-tolerant plant cells or tissues comprise two or more mutations not present in the monocot plastidic ACCase prior to culturing in the presence of the herbicide.

In one embodiment, the present invention provides a method for screening, wherein the cycloxydim is present at a sub-lethal dose.

In one embodiment, the present invention provides a method for screening, wherein the culturing in the presence of cycloxydim is performed in step-wise or gradual increase in cycloxydim concentrations.

In one embodiment, the present invention provides a method for screening, wherein the method comprises culturing of cells on a membrane. In a preferred embodiment, the present invention provides a method for screening comprises culturing of cells on a nylon membrane.

In one embodiment, the present invention provides a method for screening cycloxydim-tolerant plant cells, wherein the culturing of cells is in liquid media or semi-solid media.

In one embodiment, the present invention provides a method for screening, wherein the method further comprises identification of the at least one mutation not present in the exogenous monocot plastidic ACCase prior to culturing in the presence of the cycloxidim.

In one embodiment, the present invention provides a method for screening, wherein said monocot is rice.

In one embodiment, the present invention provides a method for screening, wherein said exogenous monocot plastidic ACCase is from rice.

### BRIEF DESCRIPTION OF THE DRAWINGS

Figure 1 is a bar graph showing relative growth rice calli derived from *Oryza sativa* subsp. *indica* grown in the presence of difference selection levels of herbicide. Figure 1A shows the results obtained with tepraloxydim, Figure 1B shows the results obtained with sethoxydim, and Figure 1C shows the results obtained with cycloxydim.
Figure 2 is a diagram of the selection process used to produce herbicide-tolerant rice plants.
Figure 3 shows photographs of plants taken one week after treatment with herbicide.
Figure 4 shows photographs of plants taken two weeks after treatment with herbicide.
Figure 5 provides the amino acid sequence of acetyl-coenzyme A carboxylase from *Alopecurus myosuroides* (GenBank accession number CAC84161).
Figure 6 provides the mRNA encoding acetyl-coenzyme A carboxylase from *Alopecurus myosuroides* (GenBank accession number AJ310767 region: 157..7119) (SEQ ID NO:4).
Figure 7A provides the genomic nucleotide sequence for *Oryza sativa* Indica & Japonica acetyl-Coenzyme A carboxylase gene (SEQ ID NO:5).
Figure 7B provides the nucleotide sequence encoding *Oryza sativa* Indica & Japonica acetyl-Coenzyme A carboxylase (SEQ ID NO:6).
Figure 7C provides the amino acid sequence of *Oryza sativa* Indica acetyl-Coenzyme A carboxylase (SEQ ID NO:3).
Figure 8A provides the nucleotide sequence encoding *Zea mays* acetyl-Coenzyme A carboxylase (SEQ ID NO:11).
Figure 8B provides the amino acid sequence of *Zea mays* acetyl-Coenzyme A carboxylase (SEQ ID NO:12).
Figure 9A provides the nucleotide sequence encoding *Zea mays* acetyl-Coenzyme A carboxylase (SEQ ID NO:13).
Figure 9B provides the amino acid sequence of *Zea mays* acetyl-Coenzyme A carboxylase (SEQ ID NO:14).
Figure 10A provides the nucleotide sequence encoding *Triticum aestivum* acetyl-Coenzyme A carboxylase (SEQ ID NO:15).
Figure 10B provides the amino acid sequence of *Triticum aestivum* acetyl-Coenzyme A carboxylase (SEQ ID NO:16).
Figure 11A provides the nucleotide sequence encoding *Setaria italica* acetyl-Coenzyme A carboxylase (SEQ ID NO:17).
Figure 11B provides the amino acid sequence of *Setaria italica* acetyl-Coenzyme A carboxylase (SEQ ID NO:18).
Figure 12A provides the nucleotide sequence encoding *Setaria italica* acetyl-Coenzyme A carboxylase (SEQ ID NO:19).
Figure 12B provides the amino acid sequence of *Setaria italica* acetyl-Coenzyme A carboxylase (SEQ ID NO:20).
Figure 13A provides the nucleotide sequence encoding *Setaria italica* acetyl-Coenzyme A carboxylase (SEQ ID NO:21).
Figure 13B provides the amino acid sequence of *Setaria italica* acetyl-Coenzyme A carboxylase (SEQ ID NO:22).
Figure 14A provides the nucleotide sequence encoding *Alopecurus myosuroides* acetyl-Coenzyme A carboxylase (SEQ ID NO:23).
Figure 14B provides the amino acid sequence of *Alopecurus myosuroides* acetyl-Coenzyme A carboxylase (SEQ ID NO:24).
Figure 15A provides the nucleotide sequence encoding *Aegilops tauschii* acetyl-Coenzyme A carboxylase (SEQ ID NO:25).
Figure 15B provides the amino acid sequence of *Aegilops tauschii* acetyl-Coenzyme A carboxylase (SEQ ID NO:26).
Figure 16 provides a comparison of single and double mutants.
Figure 17 provides a graph showing results for mutant rice versus various ACCase inhibitors.
Figure 18 provides *Alopecurus myosuroides* acetyl-Coenzyme A carboxylase amino acid sequence (GenBank accession no. CAC84161). Amino acids that may be altered in the acetyl-Coenzyme A carboxylase enzymes described herein are indicated in bold double underline.
Figure 19 provides amino acid sequence of wild-type *Oryza sativa* acetyl-Coenzyme A carboxylases aligned with *Alopecurus myosuroides* acetyl-Coenzyme A carboxylase with some critical residues denoted.

### DETAILED DESCRIPTION OF THE INVENTION

### Definitions

As used herein, "tolerant" or "herbicide-tolerant" indicates a plant or portion thereof capable of growing in the presence of an amount of herbicide that normally causes growth inhibition in a non-tolerant (e.g., a wild-type) plant or portion thereof. Levels of herbicide that normally inhibit growth of a non-tolerant plant are known and readily determined by those skilled in the art. Examples include the amounts recommended by manufacturers for application. The maximum rate is an example of an amount of herbicide that would normally inhibit growth of a non-tolerant plant.

As used herein, "recombinant" refers to an organism having genetic material from different sources.

As used herein, "mutagenized" refers to an organism having an altered genetic material as compared to the genetic material of a corresponding wild-type organism, wherein the alterations in genetic material were induced and/or selected by human action. Examples of human action that can be used to produce a mutagenized organism include, but are not limited to, tissue culture of plant cells (e.g., calli) in sub-lethal concentrations of herbicides (e.g., acetyl-Coenzyme A carboxylase inhibitors such as cycloxydim or sethoxydim), treatment of plant cells with a chemical mutagen and subsequent selection with herbicides (e.g., acetyl-Coenzyme A carboxylase inhibitors such as cycloxydim or sethoxydim); or by treatment of plant cells with x-rays and subsequent selection with herbicides (e.g., acetyl-Coenzyme A carboxylase inhibitors such as cycloxydim or sethoxydim). Any method known in the art may be used to induce mutations. Methods of inducing mutations may induce mutations in random positions in the genetic material or may induce mutations in specific locations in the genetic material (i.e., may be directed mutagenesis techniques).

As used herein, a "genetically modified organism" (GMO) is an organism whose genetic characteristics have been altered by insertion of genetic material from another source organism or progeny thereof that retain the inserted genetic material. The source organism may be of a different type of organism (e.g., a GMO plant may contain bacterial genetic material) or from the same type of organism (e.g., a GMO plant may contain genetic material from another plant). As used herein, recombinant and GMO are considered synonyms and indicate the presence of genetic material from a different source whereas mutagenized indicates altered genetic material from a corresponding wild-type organism but no genetic material from another source organism.

As used herein, "wild-type" or "corresponding wild-type plant" means the typical form of an organism or its genetic material, as it normally occurs, as distinguished from mutagenized and/or recombinant forms.

For the present invention, the terms "herbicide-tolerant" and "herbicide-resistant" are used interchangeably and are intended to have an equivalent meaning and an equivalent scope. Similarly, the terms "herbicide-tolerance" and "herbicide-resistance" are used interchangeably and are intended to have an equivalent meaning and an equivalent scope. Similarly, the terms "tolerant" and "resistant" are used interchangeably and are intended to have an equivalent meaning and an equivalent scope.

As used herein in regard to herbicides useful in various embodiments hereof, terms such as auxinic herbicide, AHAS inhibitor, acetyl-Coenzyme A carboxylase (ACCase) inhibitor, PPO inhibitor, EPSPS inhibitor, imidazolinone, sulfonylurea, and the like, refer to those agronomically acceptable herbicide active ingredients (A.I.) recognized in the art. Similarly, terms such as fungicide, nematicide, pesticide, and the like, refer to other agronomically acceptable active ingredients recognized in the art.

When used in reference to a particular mutant enzyme or polypeptide, terms such as herbicide tolerant (HT) and herbicide tolerance refer to the ability of such enzyme or polypeptide to perform its physiological activity in the presence of an amount of an herbicide A.I. that would normally inactivate or inhibit the activity of the wild-type (non-mutant) version of said enzyme or polypeptide. For example, when used specifically in regard to an AHAS enzyme, or AHASL polypeptide, it refers specifically to the ability to tolerate an AHAS-inhibitor. Classes of AHAS-inhibitors include sulfonylureas, imidazolinones, triazolopyrimidines, sulfonylaminocarbonyltriazolinones, and pyrimidinyloxy[thio]benzoates.

As used herein, "descendant" refers to any generation plant.

As used herein, "progeny" refers to a first generation plant.

### Plants

Herbicide-tolerant monocotyledonous plants of the grass family Poaceae may be used. The family Poaceae may be divided into two major clades, the clade containing the subfamilies Bambusoideae, Ehrhartoideae, and Pooideae (the BEP clade) and the clade containing the subfamilies Panicoideae, Arundinoideae, Chloridoideae, Centothecoideae, Micrairoideae, Aristidoideae, and Danthonioideae (the PACCMAD clade). The subfamily Bambusoideae includes tribe *Oryzeae.* Plants of the BEP clade, in particular plants of the subfamilies Bambusoideae and Ehrhartoideae may be used. The plants used in the invention are typically tolerant to at least one herbicide that inhibits acetyl-Coenzyme A carboxylase activity as a result of expressing an acetyl-Coenzyme A carboxylase enzyme as described below. The BET clade includes subfamilies Bambusoideae, Ehrhartoideae, and group Triticodae and no other subfamily Pooideae groups. BET crop plants are plants grown for food or forage that are members of BET subclade, for example barley, corn, etc.

Further, commerially important herbicide-tolerant monocots, including Sugarcane (Saccharum spp.), as well as Turfgrasses, e.g., Poa pratensis (Bluegrass), Agrostis spp. (Bentgrass), Lolium spp. (Ryegrasses), Festuca spp. (Fescues), Zoysia spp. (Zoysia grass), Cynodon spp. (Bermudagrass), Stenotaphrum secundatum (St. Augustine grass), Paspalum spp. (Bahiagrass), Eremochloa ophiuroides (Centipedegrass), Axonopus spp. (Carpetgrass), Bouteloua dactyloides (Buffalograss), and Bouteloua var. spp. (Grama grass) may be used.

In one embodiment, herbicide-tolerant plants of the Bambusoideae subfamily are used. Such plants are typically tolerant to one or more herbicides that inhibit acetyl-Coenzyme A carboxylase activity. Examples of herbicide-tolerant plants of the subfamily Bambusoideae include, but are not limited to, those of the genera Arundinaria, Bambusa, Chusquea, Guadua, and Shibataea.

In one embodiment, herbicide-tolerant plants of the Ehrhartoideae subfamily are used. Such plants are typically tolerant to one or more herbicides that inhibit acetyl-Coenzyme A carboxylase activity. Examples of herbicide-tolerant plants of the subfamily Ehrhartoideae include, but are not limited to, those of the genera *Erharta*, *Leersia*, *Microlaena*, *Oryza*, and *Zizania.*

In one embodiment, herbicide-tolerant plants of the Pooideae subfamily are used. Such plants are typically tolerant to one or more herbicides that inhibit acetyl-Coenzyme A carboxylase activity. Examples of herbicide-tolerant plants of the subfamily Ehrhartoideae include, but are not limited to, those of the genera *Triticeae*, *Aveneae*, and *Poeae.*

In one embodiment, the herbicide-tolerant plants used in the invention are rice plants. Two species of rice are most frequently cultivated, *Oryza sativa* and *Oryza glaberrima.* Numerous subspecies of *Oryza sativa* are commercially important including *Oryza sativa* subsp. *indica*, *Oryza sativa* subsp. *japonica*, *Oryza sativa* subsp. *javanica*, *Oryza sativa* subsp. *glutinosa* (glutinous rice), *Oryza sativa* Aromatica group (e.g., basmati), and *Oryza sativa* (Floating rice group). The present invention may use herbicide-tolerant plants in all of the aforementioned species and subspecies.

In one embodiment, the herbicide-tolerant plants used in the invention are wheat plants. Two species of wheat are most frequently cultivated, *Triticum Triticum aestivum, and Triticum turgidum.* Numerous other species are commercially important including, but not limited to, *Triticum timopheevii, Triticum monococcum, Triticum zhukovskyi* and *Triticum urartu* and hybrids thereof. Herbicide-tolerant plants in all of the aforementioned species and subspecies may be used. Examples of T. aestivum subspecies which may be used in the present invention are aestivum (common wheat), compactum (club wheat), macha (macha wheat), vavilovi (vavilovi wheat), spelta and sphaecrococcum (shot wheat). Examples of T. turgidum subspecies which may be used in the present invention are turgidum, carthlicum, dicoccon, durum, paleocolchicuna, polonicum, turanicum and dicoccoides. Examples of T. monococcum subspecies which may be used in the present invention are monococcum (einkorn) and aegilopoides. In one embodiment of the present invention, the wheat plant is a member of the Triticum aestivum species, and more particularly, the CDC Teal cultivar.

In one embodiment, herbicide-tolerant plants used in the invention are barley plants. Two species of barley are most frequently cultivated, *Hordeum vulgare* and *Hordeum arizonicum.* Numerous other species are commercially important including, but not limited, *Hordeum_bogdanii, Hordeum_brachyantherum, Hordeum brevisubulatum, Hordeum bulbosum, Hordeum comosum, Hordeum depressum, Hordeum intercedens, Hordeum jubatum, Hordeum marinum, Hordeum marinum, Hordeum parodii, Hordeum pusillum, Hordeum_secalinum*, and *Hordeum spontaneum.* The present invention encompasses the use of herbicide-tolerant plants in all of the aforementioned species and subspecies.

In one embodiment, herbicide-tolerant plants used in the invention are rye plants. Commercially important species include, but are not limited *to*,*Secale sylvestre, Secale strictum, Secale cereale, Secale vavilovii, Secale africanum, Secale ciliatoglume, Secale ancestrale,* and *Secale montanum.* The present invention encompasses the use of herbicide-tolerant plants in all of the aforementioned species and subspecies.

In one embodiment, herbicide-tolerant plants used in the invention are turf plants. Numerous commercially important species of Turf grass include *Zoysia japonica, Agrostris palustris, Poa pratensis, Poa annua, Digitaria sanguinalis, Cyperus rotundus, Kyllinga brevifolia, Cyperus amuricus, Erigeron canadensis, Hydrocotyle sibthorpioides, Kummerowia striata*, *Euphorbia humifusa*, and *Viola arvensis.* The present invention encompasses the use of herbicide-tolerant plants in all of the aforementioned species and subspecies.

In addition to being able to tolerate herbicides that inhibit acetyl-Coenzyme A carboxylase activity, plants used in the invention may also be able to tolerate herbicides that work on other physiological processes. For example, plants used in the invention may be tolerant to acetyl-Coenzyme A carboxylase inhibitors and also tolerant to other herbicides, for example, enzyme inhibitors. Examples of other enzyme inhibitors to which plants used in the invention may be tolerant include, but are not limited to, inhibitors of 5-enolpyruvylshikimate-3-phosphate synthase (EPSPS) such as glyphosate, inhibitors of acetohydroxyacid synthase (AHAS) such as imidazolinones, sulfonylureas and sulfonamide herbicides, and inhibitors of glutamine synthase such as glufosinate. In addition to enzyme inhibitors, plants used in the invention may also be tolerant of herbicides having other modes of action, for example, auxinic herbicides such as 2,4-D or dicamba, chlorophyll/carotenoid pigment inhibitors such as hydroxyphenylpyruvate dioxygenase (HPPD) inhibitors or phytoene desaturase (PDS) inhibitors, protoporphyrinogen-IX oxidase inhibitors, cell membrane destroyers, photosynthetic inhibitors such as bromoxynil or ioxynil, cell division inhibitors, root inhibitors, shoot inhibitors, and combinations thereof. Thus, plants used in the invention tolerant to acetyl-Coenzyme A carboxylase inhibitors can be made resistant to multiple classes of herbicides.

For example, plants used in the invention tolerant to acetyl-Coenzyme A carboxylase inhibitors, such as "dims" (*e*.*g*., cycloxydim, sethoxydim, clethodim, or tepraloxydim), "fops" (*e*.*g*., clodinafop, diclofop, fluazifop, haloxyfop, or quizalofop), and "dens" (such as pinoxaden), in some embodiments, may be auxinic-herbicide tolerant, tolerant to EPSPS inhibitors, such as glyphosate; to PPO inhibitors, such as pyrimidinedione, such as saflufenacil, triazolinone, such as sulfentrazone, carfentrazone, flumioxazin, diphenylethers, such as acifluorfen, fomesafen, lactofen, oxyfluorfen, N-phenylphthalamides, such as flumiclorac, CGA-248757, and/or to GS inhibitors, such as glufosinate. In addition to these classes of inhibitors, plants used in the invention tolerant to acetyl-Coenzyme A carboxylase inhibitors may also be tolerant to herbicides having other modes of action, for example, chlorophyll/carotenoid pigment inhibitors, cell membrane disruptors, photosynthesis inhibitors, cell division inhibitors, root inhibitors, shoot inhibitors, and combinations thereof. Such tolerance traits may be expressed, *e*.*g*., as mutant EPSPS proteins, or mutant glutamine synthetase proteins; or as mutant native, inbred, or transgenic aryloxyalkanoate dioxygenase (AAD or DHT), haloarylnitrilase (BXN), 2,2-dichloropropionic acid dehalogenase (DEH), glyphosate-N-acetyltransferase (GAT), glyphosate decarboxylase (GDC), glyphosate oxidoreductase (GOX), glutathione-S-transferase (GST), phosphinothricin acetyltransferase (PAT or bar), or cytochrome P450 (CYP450) proteins having an herbicide-degrading activity. Plants tolerant to acetyl-Coenzyme A carboxylase inhibitors hereof can also be stacked with other traits including, but not limited to, pesticidal traits such as *Bt* Cry and other proteins having pesticidal activity toward coleopteran, lepidopteran, nematode, or other pests; nutrition or nutraceutical traits such as modified oil content or oil profile traits, high protein or high amino acid concentration traits, and other trait types known in the art.

Furthermore, plants are also described herein that, in addition to being able to tolerate herbicides that inhibit acetyl-Coenzyme A carboxylase activity, are by the use of recombinant DNA techniques capable to synthesize one or more insecticidal proteins, especially those known from the bacterial genus *Bacillus*, particularly from *Bacillus thuringiensis*, such as δ-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda).

Furthermore, plants are also described herein that are, e.g., by the use of recombinant DNA techniques and/or by breeding and/or otherwise selected for such traits, able to synthesize one or more proteins to increase the resistance or tolerance of those plants to bacterial, viral or fungal pathogens. The methods for producing such genetically modified plants are generally known to the person skilled in the art. The plants produced as described herein can also be stacked with other traits including, but not limited to, disease resistance, enhanced mineral profile, enhanced vitamin profile, enhanced oil profile (e.g., high oleic acid content), amino acid profile (e.g, high lysine corn), and other trait types known in the art.

Furthermore, plants are also described herein that are, e.g., by the use of recombinant DNA techniques and/or by breeding and/or by other means of selection, able to synthesize one or more proteins to increase the productivity (e.g. bio mass production, grain yield, starch content, oil content or protein content), tolerance to drought, salinity or other growth-limiting environmental factors or tolerance to pests and fungal, bacterial or viral pathogens of those plants.

Furthermore, plants are also described herein that contain, e.g., by the use of recombinant DNA techniques and/or by breeding and/or by other means of selection, a modified amount of substances of content or new substances of content, specifically to improve human or animal nutrition. Furthermore, plants are also covered that contain by the use of recombinant DNA techniques a modified amount of substances of content or new substances of content, specifically to improve raw material production.

Furthermore, plants are also described herein which are, *e.g.* by the use of recombinant DNA techniques and/or by breeding and/or otherwise selected for such traits, altered to contain increased amounts of vitamins and/or minerals, and/or improved profiles of nutraceutical compounds.

Plants described herein which are tolerant to acetyl-Coenzyme A carboxylase inhibitors, relative to a wild-type plant, may comprise an increased amount of, or an improved profile of, a compound selected from the group consisting of: glucosinolates (*e*.*g*., glucoraphanin (4-methylsulfinylbutyl-glucosinolate), sulforaphane, 3-indolylmethyl-glucosinolate (glucobrassicin), 1-methoxy-3-indolylmethyl-glucosinolate (neoglucobrassicin)); phenolics (*e.g.,* flavonoids (*e.g.,* quercetin, kaempferol), hydroxycinnamoyl derivatives (*e*.*g*., 1,2,2'-trisinapoylgentiobiose, 1,2-diferuloylgentiobiose, 1,2'-disinapoyl-2-feruloylgentiobiose, 3-O-caffeoyl-quinic (neochlorogenic acid)); and vitamins and minerals (e.g., vitamin C, vitamin E, carotene, folic acid, niacin, riboflavin, thiamine, calcium, iron, magnesium, potassium, selenium, and zinc).

Plants described herein which are tolerant to acetyl-Coenzyme A carboxylase inhibitors, relative to a wild-type plant, may comprise an increased amount of, or an improved profile of, a compound selected from the group consisting of: progoitrin; isothiocyanates; indoles (products of glucosinolate hydrolysis); glutathione; carotenoids such as beta-carotene, lycopene, and the xanthophyll carotenoids such as lutein and zeaxanthin; phenolics comprising the flavonoids such as the flavonols (*e*.*g*. quercetin, rutin), the flavans/tannins (such as the procyanidins comprising coumarin, proanthocyanidins, catechins, and anthocyanins); flavones; phytoestrogens such as coumestans, lignans, resveratrol, isoflavones *e*.*g*., genistein, daidzein, and glycitein; resorcyclic acid lactones; organosulphur compounds; phytosterols; terpenoids such as carnosol, rosmarinic acid, glycyrrhizin and saponins; chlorophyll; chlorphyllin, sugars, anthocyanins, and vanilla.

Plants described herein which are tolerant to acetyl-Coenzyme A carboxylase inhibitors, relative to a wild-type plant, may comprise an increased amount of, or an improved profile of, a compound selected from the group consisting of: vincristine, vinblastine, taxanes (*e*.*g*., taxol (paclitaxel), baccatin III, 10-desacetylbaccatin III, 10-desacetyl taxol, xylosyl taxol, 7-epitaxol, 7-epibaccatin III, 10-desacetylcephalomannine, 7-epicephalomannine, taxotere, cephalomannine, xylosyl cephalomannine, taxagifine, 8-benxoyloxy taxagifine, 9-acetyloxy taxusin, 9-hydroxy taxusin, taiwanxam, taxane Ia, taxane Ib, taxane Ic, taxane Id, GMP paclitaxel, 9-dihydro 13-acetylbaccatin III, 10-desacetyl-7-epitaxol, tetrahydrocannabinol (THC), cannabidiol (CBD), genistein, diadzein, codeine, morphine, quinine, shikonin, ajmalacine, serpentine, and the like.

In addition to the herbicide-tolerant plants described herein, progeny of such plantsas well as seeds and cells derived from said plants are described herein.

Plants hereof can be used to produce plant products. Thus, a method for preparing a descendant seed comprises planting a seed of a capable of producing a plant hereof, growing the resulting plant, and harvesting descendant seed thereof. Such a method can further comprise applying an ACCase-inhibiting herbicide composition to the resulting plant. Similarly, a method for producing a derived product from a plant hereof can comprise processing a plant part thereof to obtain a derived product. Such a method can be used to obtain a derived product that is any of, e.g., fodder, feed, seed meal, oil, or seed-treatment-coated seeds. Seeds, treated seeds, and other plant products obtained by such methods are useful products that can be commercialized.

Further described herein are production of food products, consumer products, industrial products, and veterinary products from any of the plants described herein.

### Acetyl-Coenzyme A carboxylase Enzymes

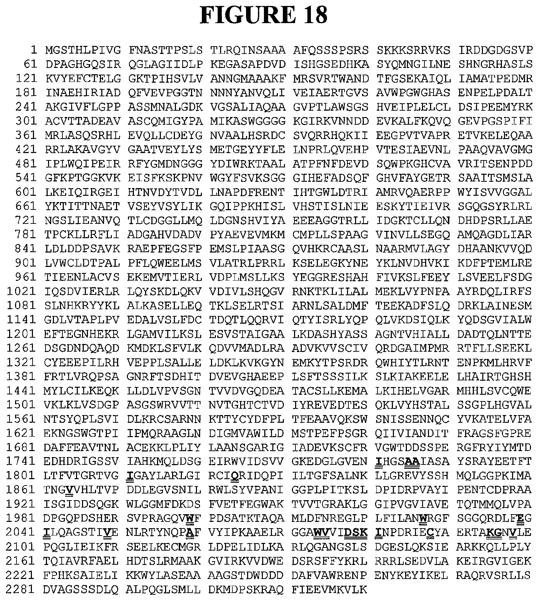

Plants expressing acetyl-Coenzyme A carboxylase enzymes with amino acid sequences that differ from the amino acid sequence of the acetyl-Coenzyme A carboxylase enzyme found in the corresponding wild-type plant are described herein. For ease of understanding, the amino acid numbering system used herein will be the numbering system used for the acetyl-Coenzyme A carboxylase from *Alopecurus myosuroides* [Huds.] (also referred to as black grass). The mRNA sequence encoding the A. *myosuroides* acetyl-Coenzyme A carboxylase is available at GenBank accession number AJ310767 and the protein sequence is available at GenBank accession no. CAC84161. The number of the amino acid referred to will be followed with *(Am)* to indicate the amino acid in the *Alopecurus myosuroides* sequence to which the amino acid corresponds. Figure 18 provides *Alopecurus myosuroides* acetyl-Coenzyme A carboxylase amino acid sequence (GenBank accession no. CAC84161). Amino acids that may be altered in the acetyl-Coenzyme A carboxylase enzymes described herein are indicated in bold double underline, and Figure 19 depicts the amino acid sequence of wild-type *Oryza sativa* acetyl-Coenzyme A carboxylases aligned with *Alopecurus myosuroides* acetyl-Coenzyme A carboxylase with some critical residues denoted.

In one example, the acetyl-Coenzyme A carboxylase differs from the corresponding wild-type acetyl-Coenzyme A carboxylase at amino acid position 1,781*(Am)*. Wild-type A. *myosuroides* acetyl-Coenzyme A carboxylase has an isoleucine at position 1,781*(Am)* (I1781). The 1,781*(Am)* ACCase mutants may have an amino acid other than isoleucine at this position. Suitable examples of amino acids that may be found at this position in the acetyl-Coenzyme A carboxylase enzymes include, but are not limited to, leucine (I1781L), valine (I1781V), threonine (I1781T) and alanine (I1781A). In one example , the acetyl-Coenzyme A carboxylase enzyme will have a leucine at position 1,781*(Am)*.

In one example, the acetyl-Coenzyme A carboxylase differs from the corresponding wild-type acetyl-Coenzyme A carboxylase at amino acid position 1,785*(Am)*. Wild-type A. *myosuroides* acetyl-Coenzyme A carboxylase has an alanine at position 1,785*(Am)* (A1785). The 1,785*(Am)* ACCase mutants may have an amino acid other than alanine at this position. Suitable examples of amino acids that may be found at this position in the acetyl-Coenzyme A carboxylase enzymes include, but are not limited to, glycine (A1785G). In one example, the acetyl-Coenzyme A carboxylase enzyme will have a glycine at position 1,785*(Am)*.

In one example, the acetyl-Coenzyme A carboxylase differs from the corresponding wild-type acetyl-Coenzyme A carboxylase at amino acid position 1,786*(Am)*. Wild-type *A*. *myosuroides* acetyl-Coenzyme A carboxylase has an alanine at position 1,786*(Am)* (A1786). The 1,786*(Am)* ACCase mutants may have an amino acid other than alanine at this position. Suitable examples of amino acids that may be found at this position in the acetyl-Coenzyme A carboxylase enzymes include, but are not limited to, proline (A1786P). In one example, the acetyl-Coenzyme A carboxylase enzyme will have a proline at position 1,786*(Am)*.

In one example, the acetyl-Coenzyme A carboxylase differs from the corresponding wild-type acetyl-Coenzyme A carboxylase at amino acid position 1,811*(Am)*. Wild-type A. *myosuroides* acetyl-Coenzyme A carboxylase has an isoleucine at position 1,811*(Am)* (I1811). The 1,811*(Am)* ACCase mutants may have an amino acid other than isoleucine at this position. Suitable examples of amino acids that may be found at this position in the acetyl-Coenzyme A carboxylase enzymes include, but are not limited to, asparagine (I1811N). In one example, the acetyl-Coenzyme A carboxylase enzyme will have an asparagine at position *1,811(Am).*

In one example, the acetyl-Coenzyme A carboxylase differs from the corresponding wild-type acetyl-Coenzyme A carboxylase at amino acid position 1,824*(Am)*. Wild-type A. *myosuroides* acetyl-Coenzyme A carboxylase has a glutamine at position 1,824*(Am)* (Q1824). The 1,824*(Am)* ACCase mutants may have an amino acid other than glutamine at this position. Suitable examples of amino acids that may be found at this position in the acetyl-Coenzyme A carboxylase enzymes include, but are not limited to, proline (Q1824P). In one example, the acetyl-Coenzyme A carboxylase enzyme will have a proline at position 1,824*(Am)*.

In one example, the acetyl-Coenzyme A carboxylase differs from the corresponding wild-type acetyl-Coenzyme A carboxylase at amino acid position 1,864*(Am)*. Wild-type A. *myosuroides* acetyl-Coenzyme A carboxylase has a valine at position 1,864*(Am)* (V1864). The 1,864*(Am)* ACCase mutants may have an amino acid other than valine at this position. Suitable examples of amino acids that may be found at this position in the acetyl-Coenzyme A carboxylase enzymes include, but are not limited to, phenylalanine (V1864F). In one example, the acetyl-Coenzyme A carboxylase enzyme will have a phenylalanine at position 1,864*(Am)*.

In one example, the acetyl-Coenzyme A carboxylase differs from the corresponding wild-type acetyl-Coenzyme A carboxylase at amino acid position 1,999*(Am)*. Wild-type A. *myosuroides* acetyl-Coenzyme A carboxylase has a tryptophan at position 1,999*(Am)* (W1999). The 1,999*(Am)* ACCase mutants may have an amino acid other than tryptophan at this position. Suitable examples of amino acids that may be found at this position in the acetyl-Coenzyme A carboxylase enzymes include, but are not limited to, cysteine (W1999C) and glycine (W1999G). In one example, the acetyl-Coenzyme A carboxylase enzyme will have a glycine at position 1,999*(Am)*.

In one example, the acetyl-Coenzyme A carboxylase differs from the corresponding wild-type acetyl-Coenzyme A carboxylase at amino acid position 2,027*(Am)*. Wild-type A. *myosuroides* acetyl-Coenzyme A carboxylase has a tryptophan at position 2,027*(Am)*(W2027). The 2,027*(Am)* ACCase mutants may have an amino acid other than tryptophan at this position. Suitable examples of amino acids that may be found at this position in the acetyl-Coenzyme A carboxylase enzymes include, but are not limited to, cysteine (W2027C) and arginine (W2027R). In one example, the acetyl-Coenzyme A carboxylase enzyme will have a cysteine at position 2,027*(Am)*.

In one example, an acetyl-Coenzyme A carboxylase differs from the corresponding wild-type acetyl-Coenzyme A carboxylase at amino acid position 2,039*(Am)*. Wild-type *A*. *myosuroides* acetyl-Coenzyme A carboxylase has a glutamic acid at position 2,039*(Am)* (E2039). The 2,039*(Am)* ACCase mutants may have an amino acid other than glutamic acid at this position. Suitable examples of amino acids that may be found at this position in the acetyl-Coenzyme A carboxylase enzymes include, but are not limited to, glycine (E2039G). In one example, the acetyl-Coenzyme A carboxylase enzyme will have an glycine at position 2,039*(Am)*.

In one example, the acetyl-Coenzyme A carboxylase differs from the corresponding wild-type acetyl-Coenzyme A carboxylase at amino acid position 2,041*(Am)*. Wild-type A. *myosuroides* acetyl-Coenzyme A carboxylase has an isoleucine at position 2,041*(Am)* (I2041). The 2,041*(Am)* ACCase mutants may have an amino acid other than isoleucine at this position. Suitable examples of amino acids that may be found at this position in the acetyl-Coenzyme A carboxylase enzymes include, but are not limited to, asparagine (I2041N), or valine (I2041V). In one example, the acetyl-Coenzyme A carboxylase enzyme will have an asparagine at position 2,041*(Am)*.

In one example, the acetyl-Coenzyme A carboxylase differs from the corresponding wild-type acetyl-Coenzyme A carboxylase at amino acid position 2,049*(Am)*. Wild-type A. *myosuroides* acetyl-Coenzyme A carboxylase has an valine at position 2,049*(Am)* (V2049). The 2,049*(Am)* ACCase mutants may have an amino acid other than valine at this position. Suitable examples of amino acids that may be found at this position in the acetyl-Coenzyme A carboxylase enzymes include, but are not limited to, phenylalanine (V2049F), isoleucine (V2049I) and leucine (V2049L). In one example, the acetyl-Coenzyme A carboxylase enzyme will have an phenylalanine at position 2,049*(Am)*.

In one example, the acetyl-Coenzyme A carboxylase differs from the corresponding wild-type acetyl-Coenzyme A carboxylase at amino acid position 2,059*(Am)*. Wild-type *A*. *myosuroides* acetyl-Coenzyme A carboxylase has an alanine at position 2,059*(Am)* (A2059). The 2,059*(Am)* ACCase mutants may have an amino acid other than an alanine at this position. Suitable examples of amino acids that may be found at this position in the acetyl-Coenzyme A carboxylase enzymes include, but are not limited to, valine (A2059V). In one example, the acetyl-Coenzyme A carboxylase enzyme will have an valine at position 2,059*(Am)*.

In one example, the acetyl-Coenzyme A carboxylase differs from the corresponding wild-type acetyl-Coenzyme A carboxylase at amino acid position 2074*(Am)*. Wild-type A. *myosuroides* acetyl-Coenzyme A carboxylase has a tryptophan at position 2074*(Am)* (W2074). The 2,074*(Am)* ACCase mutants may have an amino acid other than tryptophan at this position. Suitable examples of amino acids that may be found at this position in the acetyl-Coenzyme A carboxylase enzymes include, but are not limited to, leucine (W2074L). In one example, the acetyl-Coenzyme A carboxylase enzyme will have a leucine at 2074*(Am)*.

In one example, the acetyl-Coenzyme A carboxylase differs from the corresponding wild-type acetyl-Coenzyme A carboxylase at amino acid position 2,075*(Am)*. Wild-type A. *myosuroides* acetyl-Coenzyme A carboxylase has a valine at position 2,075*(Am)* (V2075). The 2,075*(Am)* ACCase mutants may have an amino acid other than valine at this position. Suitable examples of amino acids that may be found at this position in the acetyl-Coenzyme A carboxylase enzymes include, but are not limited to, methionine (V2075M), leucine (V2075L) and isoleucine (V2075I). In one example, the acetyl-Coenzyme A carboxylase enzyme will have a leucine at position 2,075*(Am)*. In some examples, the acetyl-Coenzyme A carboxylase enzyme will have a valine at position 2075*(Am)* and an additional valine immediately after position 2075*(Am)* and before the valine at position 2076*(Am)*, i.e., may have three consecutive valines where the wild-type enzyme has two.

In one example, the acetyl-Coenzyme A carboxylase differs from the corresponding wild-type acetyl-Coenzyme A carboxylase at amino acid position 2,078*(Am).* Wild-type A. *myosuroides* acetyl-Coenzyme A carboxylase has an aspartate at position 2,078*(Am)* (D2078). The 2,078*(Am)* ACCase mutants may have an amino acid other than aspartate at this position. Suitable examples of amino acids that may be found at this position in the acetyl-Coenzyme A carboxylase enzymes include, but are not limited to, lysine (D2,078K), glycine (D2078G), or threonine (D2078T). In one example, the acetyl-Coenzyme A carboxylase enzyme will have a glycine at position 2,078*(Am)*.

In one example, the acetyl-Coenzyme A carboxylase differs from the corresponding wild-type acetyl-Coenzyme A carboxylase at amino acid position 2,079*(Am)*. Wild-type A. *myosuroides* acetyl-Coenzyme A carboxylase has a serine at position 2,079*(Am)* (S2079). The 2,079*(Am)* ACCase mutants may have an amino acid other than serine at this position. Suitable examples of amino acids that may be found at this position in the acetyl-Coenzyme A carboxylase enzymes include, but are not limited to, phenylalanine (S2079F). In one example, the acetyl-Coenzyme A carboxylase enzyme will have a phenylalanine at position 2,079*(Am)*.

In one example, the acetyl-Coenzyme A carboxylase differs from the corresponding wild-type acetyl-Coenzyme A carboxylase at amino acid position 2,080*(Am)*. Wild-type *A*. *myosuroides* acetyl-Coenzyme A carboxylase has a lysine at position 2,080*(Am)* (K2080). The 2,080*(Am)* ACCase mutants may have an amino acid other than lysine at this position. Suitable examples of amino acids that may be found at this position in the acetyl-Coenzyme A carboxylase enzymes include, but are not limited to, glutamic acid (K2080E). In one example, the acetyl-Coenzyme A carboxylase enzyme will have a glutamic acid at position 2,080*(Am)*. In another example, the acetyl-Coenzyme A carboxylase enzymes will typically have a deletion of this position (Δ2080).

In one example, the acetyl-Coenzyme A carboxylase differs from the corresponding wild-type acetyl-Coenzyme A carboxylase at amino acid position *2,081(Am).* Wild-type A. *myosuroides* acetyl-Coenzyme A carboxylase has a isoleucine at position 2,081*(Am)* (I2081). The 2,081*(Am)* ACCase mutants may have an amino acid other than isoleucine at this position. In one example, the acetyl-Coenzyme A carboxylase enzymes will typically have a deletion of this position (Δ2081).

In one example, the acetyl-Coenzyme A carboxylase differs from the corresponding wild-type acetyl-Coenzyme A carboxylase at amino acid position 2,088*(Am)*. Wild-type *A*. *myosuroides* acetyl-Coenzyme A carboxylase has a cysteine at position 2,088*(Am)* (C2088). The 2,088*(Am)* ACCase mutants may have an amino acid other than cysteine at this position. Suitable examples of amino acids that may be found at this position in the acetyl-Coenzyme A carboxylase enzymes include, but are not limited to, arginine (C2088R), tryptophan (C2088W), phenylalanine (C2088F), glycine (C2088G), histidine (C2088H), lysine (C2088K), serine (C2088S), threonine (C2088T), leucine (C2088L) or valine (C2088V). In one example, the acetyl-Coenzyme A carboxylase enzyme will have an arginine at position 2,088*(Am)*.

In one example, the acetyl-Coenzyme A carboxylase differs from the corresponding wild-type acetyl-Coenzyme A carboxylase at amino acid position 2,095*(Am)*. Wild-type A. *myosuroides* acetyl-Coenzyme A carboxylase has a lysine at position 2,095*(Am)* (K2095). The 2,095*(Am)* ACCase mutants may have an amino acid other than lysine at this position. Suitable examples of amino acids that may be found at this position in the acetyl-Coenzyme A carboxylase enzymes include, but are not limited to, glutamic acid (K2095E). In one example, the acetyl-Coenzyme A carboxylase enzyme will have a glutamic acid at position 2,095*(Am)*.

In one example, the acetyl-Coenzyme A carboxylase differs from the corresponding wild-type acetyl-Coenzyme A carboxylase at amino acid position 2,096*(Am).* Wild-type *A*. *myosuroides* acetyl-Coenzyme A carboxylase has a glycine at position 2,096*(Am)* (G2096). The 2,096*(Am)* ACCase mutants may have an amino acid other than glycine at this position. Suitable examples of amino acids that may be found at this position in the acetyl-Coenzyme A carboxylase enzymes include, but are not limited to, alanine (G2096A), or serine (G2096S). In one example, the acetyl-Coenzyme A carboxylase enzyme will have an alanine at position 2,096*(Am)*.

In one example, the acetyl-Coenzyme A carboxylase differs from the corresponding wild-type acetyl-Coenzyme A carboxylase at amino acid position 2,098*(Am).* Wild-type *A*. *myosuroides* acetyl-Coenzyme A carboxylase has a valine at position 2,098*(Am)* (V2098). The 2,098*(Am)* ACCase mutants may have an amino acid other than valine at this position. Suitable examples of amino acids that may be found at this position in the acetyl-Coenzyme A carboxylase enzymes include, but are not limited to, alanine (V2098A), glycine (V2098G), proline (V2098P), histidine (V2098H), serine (V2098S) or cysteine (V2098C). In one example, the acetyl-Coenzyme A carboxylase enzyme will have an alanine at position 2,098*(Am)*.

The acetyl-Coenzyme A carboxylase of an herbicide-tolerant plant described herein may differ from the acetyl-Coenzyme A carboxylase of the corresponding wild-type plant at only one of the following positions: 1,781*(Am)*, 1,785*(Am)*, 1,786*(Am)*, 1,811*(Am)*, 1,824(*Am*), 1,864*(Am)*, 1,999*(Am)*, 2,027*(Am)*, 2,039*(Am)*, 2,041*(Am)*, 2,049*(Am)*, 2,059(*Am*), 2,074*(Am)*, 2,075*(Am)*, 2,078*(Am)*, 2,079*(Am)*, 2,080*(Am)*, 2,081*(Am)*, 2,088(*Am*), 2,095*(Am)*, 2,096*(Am)*, or 2,098*(Am)*. In one example, the acetyl-Coenzyme A carboxylase of an herbicide-tolerant plant described herein will differ at only one of the following positions: 2,078*(Am)*, 2,088*(Am)*, or 2,075*(Am)*. In a preferred example, the acetyl-Coenzyme A carboxylase of an herbicide-tolerant plant described herein will differ at only one of the following positions:2,039(*Am*), 2,059*(Am)*, 2,080*(Am)*, or 2,095*(Am)*. In a more preferred example, the acetyl-Coenzyme A carboxylase of a herbicide-tolerant plant described herein will differ at only one of the following positions: 1,785*(Am)*, 1,786*(Am)*, 1,811*(Am)*, 1,824*(Am)*, 1,864*(Am)*, 2,041*(Am)*, 2,049*(Am)*, 2,074*(Am)*, 2,079*(Am)*, 2,081*(Am),* 2,096*(Am),* or 2,098*(Am)*. In a most preferred example, the acetyl-Coenzyme A carboxylase of an herbicide-tolerant plant described herein will differ at only one of the following positions: 1,781*(Am)*, 1,999*(Am)*, 2,027*(Am)*, 2,041*(Am)*, or 2,096*(Am)*.

In one example, the acetyl-Coenzyme A carboxylase enzymes will have only one of the following substitutions: an isoleucine at position 2,075*(Am),* glycine at position 2,078*(Am)*, or arginine at position 2,088*(Am).* In a preferred example, the acetyl-Coenzyme A carboxylase enzymes will have only one of the following substitutions: a glycine at position 2,039*(Am)*, valine at position 2,059*(Am)*, methionine at position 2,075*(Am)*, duplication of position 2,075*(Am)* (i.e., an insertion of valine between 2,074*(Am)* and 2,075*(Am),* or an insertion of valine between position 2,075*(Am)* and 2,076*(Am)*), deletion of amino acid position 2,080*(Am)*, glutamic acid at position 2,080*(Am)*, deletion of position 2,081*(Am)*, or glutamic acid at position 2,095*(Am)*. In a more preferred example, the acetyl-Coenzyme A carboxylase enzymes will have only one of the following substitutions: a glycine at position 1,785*(Am),* a proline at position 1,786*(Am),* an asparagine at position 1,811*(Am)*, a leucine at position 2,075*(Am)*, a methionine at position 2,075*(Am)*, a threnonine at position 2,078*(Am)*, a deletion at position 2,080*(Am)*, a deletion at position 2,081*(Am)*, a tryptophan, phenylalanine, glycine, histidine, lysine, leucine, serine, threonine, or valine at position 2,088*(Am)*, a serine at position 2,096*(Am)*, an alanine at position 2,096*(Am)*, an alanine at position 2,098*(Am)*, a glycine at position 2,098*(Am),* an histidine at position 2,098*(Am)*, a proline at position 2,098*(Am)*, or a serine at position 2,098*(Am)*. In a most preferred example, the acetyl-Coenzyme A carboxylase enzymes will have only one of the following substitutions: a leucine at position 1,781*(Am)*, a threonine at position 1,781*(Am)*, a valine at position 1,781*(Am),* an alanine at position 1,781*(Am)*, a glycine at position 1,999*(Am)*, a cysteine or arginine at position 2,027*(Am)*, an arginine at position 2,027*(Am)*, an asparagine at position 2,041*(Am)*, a valine at position 2,041*(Am)*, an alanine at position 2,096*(Am)*, and a serine at position 2,096*(Am)*.

Nucleic acids encoding the acetyl-Coenzyme A carboxylase polypeptide having only one of the following substitutions: isoleucine at position 2,075*(Am)*, glycine at position 2,078*(Am)*, or arginine at position 2,088*(Am)* can be used transgenically. A monocot plant cell can be transformed with an expression vector construct comprising the nucleic acid encoding Acetyl-Coenzyme A carboxylase polypeptide having only one of the following substitutions: isoleucine at position 2,075*(Am)*, glycine at position 2,078*(Am)*, or arginine at position 2,088*(Am)*.

Rice plants are described herein which comprise nucleic acids encoding Acetyl-Coenzyme A carboxylase polypeptides having a substitution at only one amino acid position as described above.

BEP clade plants are described herein which comprise nucleic acids encoding Acetyl-Coenzyme A carboxylase polypeptides having a substitution at only one amino acid position as described above.

BET subclade plants are described herein which comprise nucleic acids encoding Acetyl-Coenzyme A carboxylase polypeptides having a substitution at only one amino acid position as described above.

BET crop plants are described herein which comprise nucleic acids encoding Acetyl-Coenzyme A carboxylase polypeptides having a substitution at only one amino acid position as described above.

Monocot plants are described herein which comprise nucleic acids encoding Acetyl-Coenzyme A carboxylase polypeptides having a substitution at only one amino acid position as described above.

Monocot plants are described herein which comprise nucleic acids encoding Acetyl-Coenzyme A carboxylase polypeptides having a substitution at amino acid position 1,781*(Am)*, wherein the amino acid at position 1,781*(Am)* differs from that of wild type and is not leucine.

Monocot plants are described herein which comprise nucleic acids encoding Acetyl-Coenzyme A carboxylase polypeptides having a substitution at amino acid position 1,999*(Am)*, wherein the amino acid at position 1,999*(Am)* differs from that of wild type and is not cysteine.

In Monocot plants are described herein which comprise nucleic acids encoding Acetyl-Coenzyme A carboxylase polypeptides having a substitution at amino acid position 2,027 *(Am)*, wherein the amino acid at position 2,027*(Am)* differs from that of wild type and is not cysteine.

In Monocot plants are described herein which comprise nucleic acids encoding Acetyl-Coenzyme A carboxylase polypeptides having a substitution at amino acid position 2,041*(Am)*, wherein the amino acid at position 2,041*(Am)* differs from that of wild type and is not valine or asparagine.

Monocot plants are described herein which comprise nucleic acids encoding Acetyl-Coenzyme A carboxylase polypeptides having a substitution at amino acid position 2,096*(Am)*, wherein the amino acid at position 2,096*(Am)* differs from that of wild type and is not alanine.

Acetyl-Coenzyme A carboxylase enzymes with an amino acid sequence that differs in more than one amino acid position from that of the acetyl-Coenzyme A carboxylase enzyme found in the corresponding wild-type plant are described herein. For example, the acetyl-Coenzyme A carboxylase may differ in 2, 3, 4, 5, 6, or 7 positions from that of the acetyl-Coenzyme A carboxylase enzyme found in the corresponding wild-type plant.

In one example, the acetyl-Coenzyme A carboxylase differs from the corresponding wild-type acetyl-Coenzyme A carboxylase at amino acid position 1,781*(Am)* and at one or more additional amino acid positions. Acetyl-Coenzyme A carboxylase enzymes described herein will typically have a leucine, a threonine, a valine, or an alanine at position 1,781*(Am)*. In addition, the enzymes will also comprise one or more of a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine, or an additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine tryptophan, phenylalanine, glycine, histidine, lysine, serine, threonine, or valine at position 2,088*(Am),* a glutamic acid at position 2,095*(Am)*, an alanine or serine at position 2,096*(Am)*, and an alanine, glycine, proline, histidine, cysteine, or serine at position 2,098*(Am).* In one example, the acetyl-Coenzyme A carboxylase will have a leucine, a threonine, a valine, or an alanine at position 1,781*(Am)* and a glycine at position 1,785*(Am)*. In one example, the acetyl-Coenzyme A carboxylase will have a leucine, a threonine, a valine, or an alanine at position 1,781*(Am)* and a proline at position 1,786*(Am)*. In one example, the acetyl-Coenzyme A carboxylase will have a leucine, a threonine, a valine, or an alanine at position 1,781*(Am)* and an asparagine at position 1,811*(Am)*. In one example, the acetyl-Coenzyme A carboxylase will have a leucine, a threonine, a valine, or an alanine at position 1,781*(Am)* and a proline at position 1824*(Am)*. In one example, the acetyl-Coenzyme A carboxylase will have a leucine, a threonine, a valine, or an alanine at position 1,781*(Am)* and a phenylalanine at position 1864*(Am)*. In one example, the acetyl-Coenzyme A carboxylase will have a leucine, a threonine, a valine, or an alanine at position 1,781*(Am)* and a cysteine or glycine at position 1,999*(Am)*. In one example, the acetyl-Coenzyme A carboxylase will have a leucine, a threonine, a valine, or an alanine at position 1,781*(Am)* and a cysteine or an arginine at position 2,027*(Am)*. In one example, the acetyl-Coenzyme A carboxylase will have a leucine, a threonine, a valine, or an alanine at position 1,781*(Am)* and a glycine at position 2039*(Am)*. In one example, the acetyl-Coenzyme A carboxylase will have a leucine, a threonine, a valine, or an alanine at position 1,781*(Am)* and an asparagine at position 2,041*(Am)*. In one example, the acetyl-Coenzyme A carboxylase will have a leucine, a threonine, a valine, or an alanine at position 1,781*(Am)* and a phenylalanine, leucine or isoleucine at position 2,049*(Am)*. In one example, the acetyl-Coenzyme A carboxylase will have a leucine, a threonine, a valine, or an alanine at position 1,781*(Am)* and a valine at position 2059*(Am).* In one example, the acetyl-Coenzyme A carboxylase will have a leucine, a threonine, a valine, or an alanine at position 1,781*(Am)* and a leucine at position 2,074*(Am)*. In one example, the acetyl-Coenzyme A carboxylase will have a leucine, a threonine, a valine, or an alanine at position 1,781*(Am)* and a leucine, isoleucine methionine, or additional valine at position 2,075*(Am)*. In one example, the acetyl-Coenzyme A carboxylase will have a leucine, a threonine, a valine, or an alanine at position 1,781*(Am)* and a glycine or threonine at position 2,078*(Am)*. In one example, the acetyl-Coenzyme A carboxylase will have a leucine, a threonine, a valine, or an alanine at position 1,781*(Am)* and a phenylalanine at position 2079*(Am)*. In one example, the acetyl-Coenzyme A carboxylase will have a leucine, a threonine, a valine, or an alanine at position 1,781*(Am)* and a glutamic acid or a deletion at position 2080*(Am)*. In one example, the acetyl-Coenzyme A carboxylase will have a leucine, a threonine, a valine, or an alanine at position 1,781*(Am)* and a deletion at position 2081*(Am).* In one example, the acetyl-Coenzyme A carboxylase will have a leucine, a threonine, a valine, or an alanine at position 1,781*(Am)* and an arginine, tryptophan, phenylalanine, glycine, histidine, lysine, serine, threonine, or valine at position 2,088*(Am)*. In one example, the acetyl-Coenzyme A carboxylase will have a leucine, a threonine, a valine, or an alanine at position 1,781*(Am)* and a glutamic acid at position 2,095*(Am)*. In one example, the acetyl-Coenzyme A carboxylase will have a leucine, a threonine, a valine, or an alanine at position 1,781*(Am)* and an alanine or serine at position 2,096*(Am).* In one example, the acetyl-Coenzyme A carboxylase will have a leucine, a threonine, a valine, or an alanine at position 1,781*(Am)* and an alanine, glycine, proline, histidine, cysteine, or serine at position 2,098*(Am).* In one example, the acetyl-Coenzyme A carboxylase will have a leucine, a threonine, a valine, or an alanine at position 1,781*(Am),* a cysteine or arginine at position 2,027*(Am)*, and an asparagine at position 2,041(Am). In one example, the acetyl-Coenzyme A carboxylase will have a leucine, a threonine, a valine, or an alanine at position 1,781*(Am)*, a cysteine or arginine at position 2,027(Am), an asparagine at position 2,041*(Am),* and an alanine at position 2,096*(Am)*.

In one example, the acetyl-Coenzyme A carboxylase differs from the corresponding wild-type acetyl-Coenzyme A carboxylase at amino acid position 1,785*(Am)* and at one or more additional amino acid positions. Acetyl-Coenzyme A carboxylase enzymes described herein will typically have an glycine at position 1,785*(Am)*. In addition, the enzymes will also comprise one or more of a leucine, threonine, a valine, or alanine at position 1,781*(Am)*, a proline at position 1,786*(Am)*, an asparagine at position 1,811*(Am)*, a proline at position 1,824*(Am)*, a phenylalanine at position 1,864*(Am),* a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am),* a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am),* a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have a glycine at position 1,785*(Am)* and a leucine, a threonine, a valine, or an alanine at position 1,781*(Am)*. In one example, the acetyl-Coenzyme A carboxylase will have a glycine at position 1,785*(Am)* and a proline at position 1,786*(Am)*. In one example, the acetyl-Coenzyme A carboxylase will have a glycine at position 1,785*(Am)* and an asparagine at position 1,811*(Am)*. In one example, the acetyl-Coenzyme A carboxylase will have a glycine at position 1,785*(Am)* and a proline at position 1,824*(Am)*. In one example, the acetyl-Coenzyme A carboxylase will have a glycine at position 1,785*(Am)* and a phenylalanine at position 1,864*(Am)*. In one example, the acetyl-Coenzyme A carboxylase will have a glycine at position 1,785*(Am)* and a cysteine or glycine at position 1,999*(Am)*. In one example, the acetyl-Coenzyme A carboxylase will have a glycine at position 1,785*(Am)* and a cysteine or an arginine at position 2,027*(Am)*. In one example, the acetyl-Coenzyme A carboxylase will have a glycine at position 1,785*(Am)* and a glycine at position 2,039*(Am)*. In one example, the acetyl-Coenzyme A carboxylase will have a glycine at position 1,785*(Am)* and an asparagine at position 2,041*(Am)*. In one example, the acetyl-Coenzyme A carboxylase will have a glycine at position 1,785*(Am)* and a phenylalanine, isoleucine or leucine at position 2,049*(Am).* In one example, the acetyl-Coenzyme A carboxylase will have a glycine at position 1,785*(Am)* and a valine at position 2,059*(Am)*. In one example, the acetyl-Coenzyme A carboxylase will have a glycine at position 1,785*(Am)* and a leucine at position 2,074(*Am*). In one example, the acetyl-Coenzyme A carboxylase will have a glycine at position 1,785*(Am)* and a leucine, isoleucine, methionine or additional valine at position 2,075(*Am*). In one example, the acetyl-Coenzyme A carboxylase will have a glycine at position 1,785*(Am)* and a glycine or threonine at position 2,078*(Am)*. In one example, the acetyl-Coenzyme A carboxylase will have a glycine at position 1,785*(Am)* and a phenylalanine at position 2,079*(Am)*. In one example, the acetyl-Coenzyme A carboxylase will have a glycine at position 1,785*(Am)* and a glutamic acid or deletion at position 2,080(*Am*). In one example, the acetyl-Coenzyme A carboxylase will have a glycine at position 1,785*(Am)* and a deletion at position 2,081*(Am)*. In one example, the acetyl-Coenzyme A carboxylase will have a glycine at position 1,785*(Am)* and an arginine, tryptophan, phenylalanine, glycine, histidine, lysine, leucine, serine, threonine, or valine at position 2,088*(Am)*. In one example, the acetyl-Coenzyme A carboxylase will have a glycine at position 1,785*(Am)* and a glutamic acid at position 2,095*(Am)*. In one example, the acetyl-Coenzyme A carboxylase will have a glycine at position 1,785*(Am)* and an alanine or serine at position 2,096*(Am)*. In one example, the acetyl-Coenzyme A carboxylase will have a glycine at position 1,785*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 1,786*(Am)* and at one or more additional amino acid positions. Acetyl-Coenzyme A carboxylase enzymes described herein will typically have a proline at position 1,786*(Am).* In addition, the enzymes will also comprise one or more of a leucine, threonine, a valine, or alanine at position 1,781*(Am)*, a glycine at position 1,785*(Am)*, an asparagine at position 1,811*(Am),* a proline at position 1,824*(Am)*, a phenylalanine at position 1,864*(Am)*, a cysteine or glycine at position 1,999*(Am),* a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid or deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have a proline at position 1,786*(Am)* and a leucine, a threonine, a valine, or an alanine at position 1,781*(Am)*. In one example, the acetyl-Coenzyme A carboxylase will have a proline at position 1,786*(Am)* and a glycine at position 1,785*(Am)*. In one example, the acetyl-Coenzyme A carboxylase will have a proline at position 1,786*(Am)* and an asparagine at position 1,811*(Am).* In one example, the acetyl-Coenzyme A carboxylase will have a proline at position 1,786*(Am)* and a proline at position 1,824(*Am*). In one example, the acetyl-Coenzyme A carboxylase will have a proline at position 1,786*(Am)* and phenylalanine at position 1,864*(Am)*. In one example, the acetyl-Coenzyme A carboxylase will have a proline at position 1,786*(Am)* and a cysteine or glycine at position 1,999*(Am)*. In one example, the acetyl-Coenzyme A carboxylase will have a proline at position 1,786*(Am)* and a cysteine or an arginine at position 2,027*(Am)*. In one example, the acetyl-Coenzyme A carboxylase will have a proline at position 1,786*(Am)* and a glycine at position 2,039*(Am)*. In one example, the acetyl-Coenzyme A carboxylase will have a proline at position 1,786*(Am)* and an asparagine at position 2,041*(Am)*. In one example, the acetyl-Coenzyme A carboxylase will have a proline at position 1,786*(Am)* and phenylalanine, isoleucine or leucine at position 2,049*(Am)* In one example, the acetyl-Coenzyme A carboxylase will have a proline at position 1,786*(Am)* and a valine at position 2,059*(Am)*. In one example, the acetyl-Coenzyme A carboxylase will have a proline at position 1,786*(Am)* and a leucine at position 2,074*(Am)*. In one example, the acetyl-Coenzyme A carboxylase will have a proline at position 1,786*(Am)* and a leucine, isoleucine, methionine or additional valine at position 2,075*(Am)*. In one example, the acetyl-Coenzyme A carboxylase will have a proline at position 1,786*(Am)* and a glycine or threonine at position 2,078*(Am)*. In one example, the acetyl-Coenzyme A carboxylase will have a proline at position 1,786*(Am)* and a phenylalanine at position 2,079*(Am)*. In one example, the acetyl-Coenzyme A carboxylase will have a proline at position 1,786*(Am)* and a glutamic acid or deletion at position 2,080*(Am)*. In one example, the acetyl-Coenzyme A carboxylase will have a proline at position 1,786*(Am)* and a deletion at position 2,081*(Am)*. In one example, the acetyl-Coenzyme A carboxylase will have a proline at position 1,786*(Am)* and an arginine, tryptophan, phenylalanine, glycine, histidine, lysine, leucine, serine, threonine, or valine at position 2,088*(Am)*. In one example, the acetyl-Coenzyme A carboxylase will have a proline at position 1,786*(Am)* and a glutamic acid at position 2,095*(Am)*. In one example, the acetyl-Coenzyme A carboxylase will have a proline at position 1,786*(Am)* and an alanine or serine at position 2,096*(Am)*. In one example, the acetyl-Coenzyme A carboxylase will have a proline at position 1,786*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 1,811*(Am)* and at one or more additional amino acid positions. Acetyl-Coenzyme A carboxylase enzymes described herein will typically have an asparagine at position 1,811*(Am)*. In addition, the enzymes will also comprise one or more of a leucine, threonine, a valine, or alanine at position 1,781*(Am)*, a glycine at position 1,785*(Am)*, a proline at position 1,786*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have an asparagine at position 1,811*(Am)* and a leucine, a threonine, a valine, or an alanine at position 1,781(*Am*). In one example, the acetyl-Coenzyme A carboxylase will have an asparagine at position 1,811*(Am)* and a glycine at position 1,785*(Am)*. In one example, the acetyl-Coenzyme A carboxylase of will have an asparagine at position 1,811*(Am)* and a proline at position 1,786*(Am)*. In one example, the acetyl-Coenzyme A carboxylase will have an asparagine at position 1,811*(Am)* and a proline at position 1,824*(Am)*. In one example, the acetyl-Coenzyme A carboxylase will have an asparagine at position 1,811*(Am)* and phenylalanine at position 1,864*(Am)*. In one example, the acetyl-Coenzyme A carboxylase will have an asparagine at position 1,811*(Am)* and a cysteine or glycine at position 1,999*(Am)*. In one example, the acetyl-Coenzyme A carboxylase will have an asparagine at position 1,811*(Am)* and a cysteine or an arginine at position 2,027*(Am)*. In one example, the acetyl-Coenzyme A carboxylase will have an asparagine at position 1,811*(Am)* and a glycine at position 2,039*(Am)*. In one example, the acetyl-Coenzyme A carboxylase will have an asparagine at position 1,811*(Am)* and an asparagine at position 2,041*(Am)*. In one example, the acetyl-Coenzyme A carboxylase will have an asparagine at position 1,811*(Am)* and phenylalanine, isoleucine or leucine at position 2,049*(Am)*. In one example, the acetyl-Coenzyme A carboxylase will have an asparagine at position 1,811*(Am)* and a valine at position 2,059*(Am)*. In one example, the acetyl-Coenzyme A carboxylase will have an asparagine at position 1,811*(Am)* and a leucine at position 2,074*(Am)*. In one example, the acetyl-Coenzyme A carboxylase will have an asparagine at position 1,811*(Am)* and a leucine, isoleucine, methionine or additional valine at position 2,075*(Am)*. In one example, the acetyl-Coenzyme A carboxylase will have an asparagine at position 1,811*(Am)* and a glycine or threonine at position 2,078*(Am)*. In one example, the acetyl-Coenzyme A carboxylase will have an asparagine at position 1,811*(Am)* and a phenylalanine at position 2,079*(Am)*. In one example, the acetyl-Coenzyme A carboxylase will have an asparagine at position 1,811*(Am)* and a glutamic acid or deletion at position 2,080*(Am)*. In one example, the acetyl-Coenzyme A carboxylase of will have an asparagine at position 1,811*(Am)* and a deletion at position 2,081(*Am*). In one example, the acetyl-Coenzyme A carboxylase will have an asparagine at position 1,811*(Am)* and an arginine, tryptophan, phenylalanine, glycine, histidine, lysine, leucine, serine, threonine, or valine at position 2,088*(Am)*. In one example, the acetyl-Coenzyme A carboxylase will have an asparagine at position 1,811*(Am)* and a glutamic acid at position 2,095*(Am)*. In one example, the acetyl-Coenzyme A carboxylase will have an asparagine at position 1,811*(Am)* and an alanine or serine at position 2,096*(Am)*. In one example, the acetyl-Coenzyme A carboxylase will have an asparagine at position 1,811*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 1,824*(Am)* and at one or more additional amino acid positions. Acetyl-Coenzyme A carboxylase enzymes described herein will typically have a proline at position 1,824*(Am)*. In addition, the enzymes will also comprise one or more of a leucine, threonine, a valine, or alanine at position 1,781*(Am),* a glycine at position 1,785*(Am)*, a proline at position 1,786*(Am)*, an asparagine at position 1,811*(Am),* a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 1,864*(Am)* and at one or more additional amino acid positions. Acetyl-Coenzyme A carboxylase enzymes described herein will typically have a phenylalanine at position 1,864*(Am)*. In addition, the enzymes will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a cysteine or glycine at position 1,999*(Am),* a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 1,999*(Am)* and at one or more additional amino acid positions. Acetyl-Coenzyme A carboxylase enzymes described herein will typically have a cysteine or glycine at position 1,999*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have a cysteine or glycine at position 1,999*(Am)* and a leucine, a threonine, a valine, or an alanine at position 1,781*(Am)*. In one example, the acetyl-Coenzyme A carboxylase will have a cysteine or glycine at position 1,999*(Am)* and a glycine at position 1,785*(Am).* In example one, the acetyl-Coenzyme A carboxylase will have a cysteine or glycine at position 1,999*(Am)* and a proline at position 1,786*(Am)*. In one example, the acetyl-Coenzyme A carboxylase will have a cysteine or glycine at position 1,999*(Am)* and have an asparagine at position 1,811*(Am)*. In one example, the acetyl-Coenzyme A carboxylase will have a cysteine or glycine at position 1,999*(Am)* and a proline at position 1,824*(Am)*. In one example, the acetyl-Coenzyme A carboxylase will have a cysteine or glycine at position 1,999*(Am)* and phenylalanine at position 1,864*(Am)*. In one example, the acetyl-Coenzyme A carboxylase will have a cysteine or glycine at position 1,999*(Am)* and a cysteine or an arginine at position 2,027*(Am)*. In one example, the acetyl-Coenzyme A carboxylase will have a cysteine or glycine at position 1,999*(Am)* and a glycine at position 2,039*(Am)*. In one example, the acetyl-Coenzyme A carboxylase will have a cysteine or glycine at position 1,999*(Am)* and an asparagine at position 2,041*(Am)*. In one example, the acetyl-Coenzyme A carboxylase will have a cysteine or glycine at position 1,999*(Am)* and a phenylalanine, isoleucine or leucine at position 2,049*(Am)*. In one example, the acetyl-Coenzyme A carboxylase will have a cysteine or glycine at position 1,999*(Am)* and a cysteine or a valine at position 2,059*(Am)*. In one example, the acetyl-Coenzyme A carboxylase will have a cysteine or glycine at position 1,999*(Am)* and a leucine at position 2,074*(Am).* In one example, the acetyl-Coenzyme A carboxylase will have a cysteine or glycine at position 1,999*(Am)* and a leucine, isoleucine, methionine or additional valine at position 2,075*(Am)*. In one example, the acetyl-Coenzyme A carboxylase will have a cysteine or glycine at position 1,999*(Am)* and a glycine or threonine at position 2,078*(Am)*. In one example, the acetyl-Coenzyme A carboxylase will have a cysteine or glycine at position 1,999*(Am)* and a phenylalanine at position 2,079*(Am)*. In one example, the acetyl-Coenzyme A carboxylase will have a cysteine or glycine at position 1,999*(Am)* and a glutamic acid or deletion at position 2,080*(Am)*. In one example, the acetyl-Coenzyme A carboxylase will have a cysteine or glycine at position 1,999*(Am)* and a deletion at position 2,081*(Am)*. In one example, the acetyl-Coenzyme A carboxylase will have a cysteine or glycine at position 1,999*(Am*) and an arginine, tryptophan, phenylalanine, glycine, histidine, lysine, leucine, serine, threonine, or valine at position 2,088*(Am)*. In one example, the acetyl-Coenzyme A carboxylase will have a cysteine or glycine at position 1,999*(Am)* and a glutamic acid at position 2,095*(Am)*. In one example, the acetyl-Coenzyme A carboxylase will have a cysteine or glycine at position 1,999*(Am)* and an alanine or serine at position 2,096*(Am)*. In one example, the acetyl-Coenzyme A carboxylase will have a cysteine or glycine at position 1,999*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,027*(Am)* and at one or more additional amino acid positions. Acetyl-Coenzyme A carboxylase enzymes described herein will typically have a cysteine or arginine at position 2,027*(Am)*. In addition, the enzymes will also comprise one or more of a leucine, threonine, a valine, or alanine at position 1,781*(Am),* a glycine at position 1,785*(Am)*, a proline at position 1,786*(Am)*, an asparagine at position 1,811(*Am*), a proline at position 1,824*(Am)*, a phenylalanine at position 1,864*(Am)*, a cysteine or glycine at position 1,999*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position *2,081(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have a cysteine or arginine at position 2,027*(Am)* and a leucine, a threonine, a valine, or an alanine at position 1,781*(Am)*. In one example, the acetyl-Coenzyme A carboxylase will have a cysteine or arginine at position 2,027*(Am)* and a glycine at position 1,785*(Am)*. In one example, the acetyl-Coenzyme A carboxylase will have a cysteine or arginine at position 2,027*(Am)* and a proline at position 1,786*(Am)*. In one example, the acetyl-Coenzyme A carboxylase will have a cysteine or arginine at position 2,027*(Am)* and have an asparagine at position 1,811*(Am)*. In one example, the acetyl-Coenzyme A carboxylase will have a cysteine or arginine at position 2,027*(Am)* and have a proline at position 1,824*(Am)*. In one example, the acetyl-Coenzyme A carboxylase will have a cysteine or arginine at position 2,027*(Am)* and have a phenylalanine at position 1,864*(Am)*. In one example, the acetyl-Coenzyme A carboxylase will have a cysteine or arginine at position 2,027*(Am)* and a cysteine or glycine at position 1,999*(Am)*. In one example, the acetyl-Coenzyme A carboxylase will have a cysteine or arginine at position 2,027*(Am)* and have a glycine at position 2,039*(Am)*. In one example, the acetyl-Coenzyme A carboxylase will have a cysteine or arginine at position 2,027*(Am)* and an asparagine at position 2,041*(Am)*. In one example, the acetyl-Coenzyme A carboxylase will have a cysteine or arginine at position 2,027*(Am)* and a phenylalanine, isoleucine or leucine at position 2,049*(Am)*. In one example, the acetyl-Coenzyme A carboxylase will have a cysteine or arginine at position 2,027*(Am)* and have a valine at position 2,059*(Am)*. In one example, the acetyl-Coenzyme A carboxylase will have a cysteine or arginine at position 2,027*(Am)* and a leucine at position 2,074*(Am)*. In one example, the acetyl-Coenzyme A carboxylase will have a cysteine or arginine at position 2,027*(Am)* and a leucine, isoleucine, methionine or additional valine at position 2,075*(Am)*. In one example, the acetyl-Coenzyme A carboxylase will have a cysteine or arginine at position 2,027*(Am)* and a glycine or threonine at position 2,078*(Am)*. In one example, the acetyl-Coenzyme A carboxylase will have a cysteine or arginine at position 2,027*(Am)* and a phenylalanine at position 2,079*(Am)*. In one example, the acetyl-Coenzyme A carboxylase will have a cysteine or arginine at position 2,027*(Am)* and a glutamic acid or deletion at position 2,080*(Am)*. In one example, the acetyl-Coenzyme A carboxylase will have a cysteine or arginine at position 2,027*(Am)* and a deletion at position 2,081*(Am)*. In one example, the acetyl-Coenzyme A carboxylase will have a cysteine or arginine at position 2,027*(Am)* and an arginine, tryptophan, phenylalanine, glycine, histidine, lysine, leucine, serine, threonine, or valine at position 2,088*(Am)*. In one example, the acetyl-Coenzyme A carboxylase will have a cysteine or arginine at position 2,027*(Am)* and a glutamic acid at position 2,095*(Am)*. In one example, the acetyl-Coenzyme A carboxylase will have a cysteine or arginine at position 2,027*(Am)* and an alanine or serine at position 2,096(*Am*). In one example, the acetyl-Coenzyme A carboxylase will have a cysteine or arginine at position 2,027*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,039(*Am*) and at one or more additional amino acid positions. Acetyl-Coenzyme A carboxylase enzymes described herein will typically have a glycine at position 2,039*(Am)*. In addition, the enzymes will also comprise one or more of a leucine, threonine, a valine, or alanine at position *1,781(Am),* a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am)*, a glycine or threonine at position 2,078*(Am),* a phenylalanine at position 2,079*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,041*(Am)* and at one or more additional amino acid positions. Acetyl-Coenzyme A carboxylase enzymes described herein will typically have an asparagine at position 2,041*(Am)*. In addition, the enzymes will also comprise one or more of a leucine, threonine, a valine, or alanine at position 1,781*(Am)*, a glycine at position 1,785*(Am)*, a proline at position 1,786*(Am)*, an asparagine at position *1,811(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2041*(Am)*, a phenylalanine, isoleucine or leucine at position 2,049*(Am)*, a valine at position 2,059(*Am*), a leucine at position 2,074*(Am)*, a leucine, isoleucine, methionine or additional valine at position 2,075*(Am),* a glycine or threonine at position 2,078(*Am*), a phenylalanine at position 2,079*(Am)*, a glutamic acid at position 2,080(*Am*), a deletion at position 2,080*(Am)*, a deletion at position *2,081(Am),* an arginine, tryptophan, phenylalanine, glycine, histidine, lysine, leucine, serine, threonine, or valine at position 2,088(*Am*), a glutamic acid at position 2,095(*Am*), an alanine or serine at position 2,096(*Am*), and an alanine, glycine, proline, histidine, cysteine or serine at position 2,098(*Am*). In one example, the acetyl-Coenzyme A carboxylase will have an asparagine at position 2,041*(Am)* and a leucine, a threonine, a valine, or an alanine at position 1,781*(Am)*. In one example, the acetyl-Coenzyme A carboxylase will have an asparagine at position 2,041*(Am)* and a glycine at position 1,785*(Am)*. In one example, the acetyl-Coenzyme A carboxylase will have an asparagine at position 2,041*(Am)* and a proline at position 1,786*(Am)*. In one example, the acetyl-Coenzyme A carboxylase will have an asparagine at position 2,041*(Am)* and have an asparagine at position *1,811(Am).* In one example, the acetyl-Coenzyme A carboxylase will have an asparagine at position 2,041*(Am)* and a proline at position 1824*(Am)*. In one example, the acetyl-Coenzyme A carboxylase will have an asparagine at position 2,041*(Am)* and a phenylalanine at position 1864*(Am)*. In one example, the acetyl-Coenzyme A carboxylase will have an asparagine at position 2,041*(Am)* and a cysteine or glycine at position 1,999*(Am)*. In one example, the acetyl-Coenzyme A carboxylase will have an asparagine at position 2,041*(Am)* and a cysteine or arginine at position 2,027*(Am)*. In one example, the acetyl-Coenzyme A carboxylase will have an asparagine at position 2,041*(Am)* and a glycine at position 2039*(Am)*. In one example, the acetyl-Coenzyme A carboxylase will have an asparagine at position 2,041*(Am)* and an asparagine at position 2,041*(Am)*. In one example, the acetyl-Coenzyme A carboxylase will have an asparagine at position 2,041*(Am)* and a phenylalanine, isoleucine or leucine at position 2,049(*Am*) In one example, the acetyl-Coenzyme A carboxylase will have an asparagine at position 2,041*(Am)* and a valine at position 2,059*(Am)*.. In one example, the acetyl-Coenzyme A carboxylase will have an asparagine at position 2,041*(Am)* and a leucine at position 2,074*(Am)*. In one example, the acetyl-Coenzyme A carboxylase will have an asparagine at position 2,041*(Am)* and a leucine, isoleucine, methionine or additional valine at position 2,075*(Am)*. In one example, the acetyl-Coenzyme A carboxylase will have an asparagine at position 2,041*(Am)* and a glycine or threonine at position 2,078(*Am*). In one example, the acetyl-Coenzyme A carboxylase will have an asparagine at position 2,041*(Am)* and a phenylalanine at position 2079(*Am*). In one example, the acetyl-Coenzyme A carboxylase will have an asparagine at position 2,041*(Am)* and a glutamic acid or a deletion at position 2080*(Am)*. In one example, the acetyl-Coenzyme A carboxylase will have an asparagine at position 2,041*(Am)* and a deletion at position 2081(*Am*). In one example, the acetyl-Coenzyme A carboxylase will have an isoleucine at position 2,041*(Am)* and an arginine, tryptophan, phenylalanine, glycine, histidine, lysine, leucine, serine, threonine, or valine at position 2,088*(Am)*. In one example, the acetyl-Coenzyme A carboxylase will have an isoleucine at position 2,041*(Am)* and a glutamic acid at position 2,095*(Am)*. In one example, the acetyl-Coenzyme A carboxylase will have an isoleucine at position 2,041*(Am)* and an alanine or serine at position 2,096*(Am)*. In one example, the acetyl-Coenzyme A carboxylase will have an isoleucine at position 2,041*(Am)* and an alanine, glycine, proline, histidine, cysteine, or serine at position 2,098*(Am).*

In one example, the acetyl-Coenzyme A carboxylase differs from the corresponding wild-type acetyl-Coenzyme A carboxylase at amino acid position 2,049*(Am)* and at one or more additional amino acid positions. Acetyl-Coenzyme A carboxylase enzymes described herein will typically have a phenylalanine, isoleucine or leucine at position 2,049*(Am)*. In addition, the enzymes will also comprise one or more of a leucine, threonine, a valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864(*Am*), a cysteine or glycine at position 1,999*(Am)*, a cysteine or arginine at position 2,027*(Am)*, a glycine at position 2,039*(Am)*, an asparagine at position 2,041*(Am)*, a valine at position 2,059*(Am)*, a leucine at position 2,074*(Am)*, a leucine, isoleucine, methionine or additional valine at position 2,075(*Am*), a glycine or threonine at position 2,078*(Am)*, a phenylalanine at position 2,079(*Am*), a glutamic acid at position 2,080(*Am*), a deletion at position 2,080*(Am)*, a deletion at position *2,081(Am),* an arginine, tryptophan, phenylalanine, glycine, histidine, lysine, leucine, serine, threonine, or valine at position 2,088*(Am)*, a glutamic acid at position 2,095(*Am*), an alanine or serine at position 2,096(*Am*), and an alanine, glycine, proline, histidine, cysteine, or serine at position 2,098(*Am*). In one example, the acetyl-Coenzyme A carboxylase will have a phenylalanine, isoleucine or leucine at position 2,049*(Am)* and a leucine, a threonine, a valine, or an alanine at position 1,781*(Am)*. In one example, the acetyl-Coenzyme A carboxylase will have a phenylalanine, isoleucine or leucine at position 2,049*(Am)* and a glycine at position 1,785*(Am)*. In one example, the acetyl-Coenzyme A carboxylase will have a phenylalanine, isoleucine or leucine at position 2,049(*Am*) and a proline at position 1,786(Am). In one example, the acetyl-Coenzyme A carboxylase will have a phenylalanine, isoleucine or leucine at position 2,049*(Am)* and have an asparagine at position 1,811*(Am).* In one example, the acetyl-Coenzyme A carboxylase will have a phenylalanine, isoleucine or leucine at position 2,049*(Am)* and a proline at position 1824(*Am*). In one example, the acetyl-Coenzyme A carboxylase will have a phenylalanine, isoleucine or leucine at position 2,049(*Am*) and a phenylalanine at position 1864(*Am*). In one example, the acetyl-Coenzyme A carboxylase will have a phenylalanine, isoleucine or leucine at position 2,049(*Am*) and a cysteine or glycine at position 1,999*(Am)*. In one example, the acetyl-Coenzyme A carboxylase will have a phenylalanine, isoleucine or leucine at position 2,049*(Am)* and a cysteine or an arginine at position 2,027*(Am)*. In one example, the acetyl-Coenzyme A carboxylase will have a phenylalanine, isoleucine or leucine at position 2,049*(Am)* and a glycine at position 2039(*Am*). In one example, the acetyl-Coenzyme A carboxylase will have a phenylalanine, isoleucine or leucine at position 2,049*(Am)* and an asparagine at position 2,041*(Am)*. In one example, the acetyl-Coenzyme A carboxylase will have a phenylalanine, isoleucine or leucine at position 2,049*(Am)* and a valine at position 2059(*Am*). In one example, the acetyl-Coenzyme A carboxylase will have a phenylalanine, isoleucine or leucine at position 2,049(*Am*) and a leucine at position 2,074*(Am)*. In one example, the acetyl-Coenzyme A carboxylase will have a phenylalanine, isoleucine or leucine at position 2,049(*Am*) and a leucine, isoleucine methionine, or additional valine at position 2,075*(Am)*. In one example, the acetyl-Coenzyme A carboxylase will have a phenylalanine, isoleucine or leucine at position 2,049(*Am*) and a glycine or threonine at position 2,078*(Am)*. In one example, the acetyl-Coenzyme A carboxylase will have a phenylalanine, isoleucine or leucine at position 2,049*(Am)* and a phenylalanine at position 2079(*Am*). In one example, the acetyl-Coenzyme A carboxylase will have a phenylalanine, isoleucine or leucine at position 2,049*(Am)* and a glutamic acid or a deletion at position 2080(*Am*). In one example, the acetyl-Coenzyme A carboxylase will have a phenylalanine, isoleucine or leucine at position 2,049(*Am*) and a deletion at position 2081(*Am*). In one example, the acetyl-Coenzyme A carboxylase will have a phenylalanine, isoleucine or leucine at position 2,049*(Am)* and an arginine, tryptophan, phenylalanine, glycine, histidine, lysine, serine, threonine, or valine at position 2,088*(Am)*. In one example, the acetyl-Coenzyme A carboxylase will have a phenylalanine, isoleucine or leucine at position 2,049*(Am)* and a glutamic acid at position 2,095*(Am)*. In one example, the acetyl-Coenzyme A carboxylase will have a phenylalanine, isoleucine or leucine at position 2,049*(Am)* and an alanine or serine at position 2,096*(Am)*. In one example, the acetyl-Coenzyme A carboxylase will have a phenylalanine, isoleucine or leucine at position 2,049*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,059*(Am)* and at one or more additional amino acid positions. Acetyl-Coenzyme A carboxylase enzymes described herein will typically have a valine at position 2,059*(Am)*. In addition, the enzymes will also comprise one or more of a leucine, threonine, a valine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a leucine at position 2,074*(Am)*, a leucine, isoleucine, methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position *2,081(Am),* an arginine or tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,074*(Am)* and at one or more additional amino acid positions. Acetyl-Coenzyme A carboxylase enzymes described herein will typically have a leucine at position 2,074*(Am)*. In addition, the enzymes will also comprise one or more of a leucine, threonine, a valine, or alanine at position 1,781*(Am),* a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027(Am), a glycine at position 2,039(*Am*), an asparagine at position 2,041*(Am)*, a phenylalanine, isoleucine or leucine at position 2,049(*Am*), a valine at position 2,059*(Am)*, a leucine, isoleucine, methionine or additional valine at position 2,075(*Am*), a glycine or threonine at position 2,078(*Am*), a phenylalanine at position 2,079*(Am)*, a glutamic acid at position 2,080(*Am*), a deletion at position 2,080(*Am*), a deletion at position *2,081(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have a leucine at position 2,074*(Am)* and a leucine, a threonine, a valine, or an alanine at position 1,781*(Am).* In one example, the acetyl-Coenzyme A carboxylase will have a leucine at position 2,074*(Am)* and a glycine at position 1,785*(Am)*. In one example, the acetyl-Coenzyme A carboxylase will have a leucine at position 2,074*(Am)* and a proline at position 1,786*(Am)*. In one example, the acetyl-Coenzyme A carboxylase will have a leucine at position 2,074*(Am)* and have an asparagine at position 1,811*(Am).* In one example, the acetyl-Coenzyme A carboxylase will have a leucine at position 2,074(*Am*) and a proline at position 1824*(Am)*. In one example, the acetyl-Coenzyme A carboxylase will have a leucine at position 2,074(*Am*) and a phenylalanine at position 1864(*Am*). In one example, the acetyl-Coenzyme A carboxylase will have a leucine at position 2,074*(Am)* and a cysteine or glycine at position 1,999*(Am)*. In one example, the acetyl-Coenzyme A carboxylase will have a leucine at position 2,074*(Am)* and a cysteine or an arginine at position 2,027*(Am)*. In one example, the acetyl-Coenzyme A carboxylase will have a leucine at position 2,074(*Am*) and a glycine at position 2039(*Am*). In one example, the acetyl-Coenzyme A carboxylase will have a leucine at position 2,074*(Am)* and an asparagine at position 2,041*(Am)*. In one example, the acetyl-Coenzyme A carboxylase will have a leucine at position 2,074*(Am)* and a phenylalanine, leucine or isoleucine at position 2,049*(Am*). In one example, the acetyl-Coenzyme A carboxylase will have a leucine at position 2,074(*Am*) and a valine at position 2059(*Am*). In one example, the acetyl-Coenzyme A carboxylase will have a leucine at position 2,074*(Am)* and a leucine, isoleucine methionine, or additional valine at position 2,075*(Am)*. In one example, the acetyl-Coenzyme A carboxylase will have a leucine at position 2,074*(Am)* and a glycine or threonine at position 2,078*(Am)*. In one example, the acetyl-Coenzyme A carboxylase will have a leucine at position 2,074(*Am*) and a phenylalanine at position 2079(*Am*). In one example, the acetyl-Coenzyme A carboxylase will have a leucine at position 2,074(*Am*) and a glutamic acid or a deletion at position 2080(*Am*). In one example, the acetyl-Coenzyme A carboxylase will have a leucine at position 2,074(*Am*) and a deletion at position 2081(*Am*). In one example, the acetyl-Coenzyme A carboxylase will have a leucine at position 2,074*(Am)* and an arginine, tryptophan, phenylalanine, glycine, histidine, lysine, serine, threonine, or valine at position 2,088*(Am)*. In one example, the acetyl-Coenzyme A carboxylase will have a leucine at position 2,074*(Am)* and a glutamic acid at position 2,095*(Am)*. In one example, the acetyl-Coenzyme A carboxylase will have a leucine at position 2,074*(Am)* and an alanine or serine at position 2,096*(Am)*. In one example, the acetyl-Coenzyme A carboxylase will have a leucine at position 2,074*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,075*(Am)* and at one or more additional amino acid positions. Acetyl-Coenzyme A carboxylase enzymes described herein will typically have a leucine, isoleucine, methionine or additional valine at position 2,075*(Am)*. In addition, the enzymes will also comprise one or more of a leucine, threonine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have a leucine, isoleucine, methionine or additional valine at position 2,075*(Am)* and a leucine, a threonine, a valine, or an alanine at position 1,781*(Am)*. In one example, the acetyl-Coenzyme A carboxylase will have a leucine, isoleucine, methionine or additional valine at position 2,075*(Am)* and a glycine at position 1,785*(Am)*. In one example, the acetyl-Coenzyme A carboxylase will have a leucine, isoleucine, methionine or additional valine at position 2,075*(Am)* and a proline at position 1,786*(Am)*. In one example, the acetyl-Coenzyme A carboxylase will have a leucine, isoleucine, methionine or additional valine at position 2,075*(Am)* and have an asparagine at position 1,811*(Am)*. In one example, the acetyl-Coenzyme A carboxylase will have a leucine, isoleucine, methionine or additional valine at position 2,075*(Am)* and a cysteine or glycine at position 1,999*(Am)*. In one example, the acetyl-Coenzyme A carboxylase will have a leucine, isoleucine, methionine or additional valine at position 2,075*(Am)* and a cysteine or arginine at position 2,027*(Am)*. In one example, the acetyl-Coenzyme A carboxylase will have a leucine, isoleucine, methionine or additional valine at position 2,075*(Am)* and an isoleucine at position 2,041*(Am)*. In one example, the acetyl-Coenzyme A carboxylase will have a leucine, isoleucine, methionine or additional valine at position 2,075*(Am) an*d a phenylalanine, isoleucine or leucine at position 2,049*(Am)*. In one example, the acetyl-Coenzyme A carboxylase will have a leucine, isoleucine, methionine or additional valine at position 2,075*(Am)* and a leucine at position 2,074*(Am)*. In one example, the acetyl-Coenzyme A carboxylase will have a leucine, isoleucine, methionine or additional valine at position 2,075*(Am)* and a glycine or threonine at position 2,078(*Am*). In one example, the acetyl-Coenzyme A carboxylase will have a leucine, isoleucine, methionine or additional valine at position 2,075*(Am)* and an arginine or tryptophan, phenylalanine, glycine, histidine, lysine, leucine, serine, threonine, or valine at position 2,088*(Am)*. In one example, the acetyl-Coenzyme A carboxylase will have a leucine, isoleucine, methionine or additional valine at position 2,075*(Am)* and an alanine or serine at position 2,096*(Am)*. In one example, the acetyl-Coenzyme A carboxylase will have a leucine, isoleucine, methionine or additional valine at position 2,075*(Am)* and an alanine, glycine, proline, histidine, cysteine, or serine at position 2,098*(Am)*.

In one example, an acetyl-Coenzyme A carboxylase differs from the corresponding wild-type acetyl-Coenzyme A carboxylase at amino acid position 2,078*(Am)* and at one or more additional amino acid positions. Acetyl-Coenzyme A carboxylase enzymes described herein will typically have a glycine or threonine at position 2,078*(Am)*. In addition, the enzymes will also comprise one or more of a leucine, threonine, a valine, or alanine at position 1,781*(Am),* a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine, methionine or additional valine at position 2,075*(Am),* a phenylalanine at position 2,079*(Am)*, a glutamic acid at position 2,080(*Am*), a deletion at position 2,080(*Am*), a deletion at position 2,081*(Am),* an arginine, tryptophan, phenylalanine, glycine, histidine, lysine, leucine, serine, threonine, or valine at position 2,088*(Am)*, a glutamic acid at position 2,095(*Am*), an alanine or serine at position 2,096(*Am*), and an alanine, glycine, proline, histidine, cysteine or serine at position 2,098*(Am)*. In one example, the acetyl-Coenzyme A carboxylase will have a glycine or threonine at position 2,078*(Am)* and a leucine, a threonine or an alanine at position 1,781*(Am)*. In one example, the acetyl-Coenzyme A carboxylase will have a glycine or threonine at position 2,078*(Am)* and a glycine at position 1,785*(Am)*. In one example, the acetyl-Coenzyme A carboxylase will have a glycine or threonine at position 2,078*(Am)* and a proline at position 1,786*(Am)*. In one example, the acetyl-Coenzyme A carboxylase will have a glycine or threonine at position 2,078*(Am)* and an asparagine at position 1,811*(Am).* In one example, the acetyl-Coenzyme A carboxylase will have a glycine or threonine at position 2,078*(Am)* and a cysteine or glycine at position 1,999*(Am)*. In one example, the acetyl-Coenzyme A carboxylase will have a glycine or threonine at position 2,078*(Am)* and a cysteine or arginine at position 2,027*(Am)*. In one example, the acetyl-Coenzyme A carboxylase will have a glycine or threonine at position 2,078*(Am)* and an isoleucine at position 2,041*(Am)*. In one example, the acetyl-Coenzyme A carboxylase will have a glycine or threonine at position 2,078*(Am)* and a phenylalanine, isoleucine or leucine at position 2,049*(Am)*. In one example, the acetyl-Coenzyme A carboxylase will have a glycine or threonine at position 2,078*(Am)* and a leucine at position 2,074*(Am)*. In one example, the acetyl-Coenzyme A carboxylase will have a glycine or threonine at position 2,078*(Am)* and a leucine, isoleucine, methionine or additional valine at position 2,075*(Am)*. In one example, the acetyl-Coenzyme A carboxylase will have a glycine or threonine at position 2,078*(Am)* and an arginine, tryptophan, phenylalanine, glycine, histidine, lysine, leucine, serine, threonine, or valine at position 2,088*(Am)*. In one example, the acetyl-Coenzyme A carboxylase will have a glycine or threonine at position 2,078*(Am)* and an alanine or serine at position 2,096*(Am)*. In one example, the acetyl-Coenzyme A carboxylase will have a glycine or threonine at position 2,078*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,079*(Am)* and at one or more additional amino acid positions. Acetyl-Coenzyme A carboxylase enzymes described herein will typically have a phenylalanine at position 2,079*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am),* a leucine, isoleucine , methionine or additional valine at position 2,075*(Am)*, a glycine or threonine at position 2,078*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,080*(Am)* and at one or more additional amino acid positions. Acetyl-Coenzyme A carboxylase enzymes described herein will typically have a glutamic acid or a deletion at position 2,080*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,081*(Am)* and at one or more additional amino acid positions. Acetyl-Coenzyme A carboxylase enzymes described herein will typically have a deletion at position 2,081*(Am)*,. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, an arginine, tryptophan, phenylalanine, glycine, histidine, lysine, leucine, serine, threonine, or valine at position 2,088*(Am)*, a glutamic acid at position 2,095*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,088*(Am)* and at one or more additional amino acid positions. Acetyl-Coenzyme A carboxylase enzymes described herein will typically have an arginine, tryptophan, phenylalanine, glycine, histidine, lysine, leucine, serine, threonine, or valine at position 2,088*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074(*Am*), a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am*), a deletion at position 2,080*(Am)*, a deletion at position 2,081*(Am)*, a glutamic acid at position 2,095*(Am)*, an alanine or serine at position 2,096*(Am)*, and an alanine, glycine, proline, histidine, cysteine, or serine at position 2,098*(Am)*. In one example, the acetyl-Coenzyme A carboxylase will have an arginine, tryptophan, phenylalanine, glycine, histidine, lysine, leucine, serine, threonine, or valine at position 2,088*(Am)* and a leucine, a threonine, valine, or an alanine at position 1,781*(Am)*. In one example, the acetyl-Coenzyme A carboxylase will have an arginine, tryptophan, phenylalanine, glycine, histidine, lysine, leucine, serine, threonine, or valine at position 2,088*(Am)* and a glycine at position 1,785*(Am)*. In one example, the acetyl-Coenzyme A carboxylase will have an arginine, tryptophan, phenylalanine, glycine, histidine, lysine, leucine, serine, threonine, or valine at position 2,088*(Am)* and a proline at position 1,786*(Am)*. In one example, the acetyl-Coenzyme A carboxylase will have an arginine, tryptophan, phenylalanine, glycine, histidine, lysine, leucine, serine, threonine, or valine at position 2,088*(Am)* and an asparagine at position 1,811*(Am).* In one example, the acetyl-Coenzyme A carboxylase will have an arginine, tryptophan, phenylalanine, glycine, histidine, lysine, leucine, serine, threonine, or valine at position 2,088*(Am)* and a cysteine or glycine at position 1,999*(Am)*. In one example, the acetyl-Coenzyme A carboxylase will have an arginine or tryptophan, phenylalanine, glycine, histidine, lysine, leucine, serine, threonine, or valine at position 2,088*(Am)* and a cysteine or arginine at position 2,027*(Am)*. In one example, the acetyl-Coenzyme A carboxylase will have an arginine, tryptophan, phenylalanine, glycine, histidine, lysine, leucine, serine, threonine, or valine at position 2,088*(Am)* and an isoleucine at position 2,041*(Am)*. In one example, the acetyl-Coenzyme A carboxylase will have an arginine, tryptophan, phenylalanine, glycine, histidine, lysine, leucine, serine, threonine, or valine at position 2,088*(Am)* and a phenylalanine, isoleucine or leucine at position 2,049*(Am)*. In one example, the acetyl-Coenzyme A carboxylase will have an arginine, tryptophan, phenylalanine, glycine, histidine, lysine, leucine, serine, threonine, or valine at position 2,088*(Am)* and a leucine at position 2,074*(Am)*. In one example, the acetyl-Coenzyme A carboxylase will have an arginine, tryptophan, phenylalanine, glycine, histidine, lysine, leucine, serine, threonine, or valine at position 2,088*(Am)* and a leucine, isoleucine , methionine or additional valine at position 2,075*(Am)*. In one example, the acetyl-Coenzyme A carboxylase will have an arginine, tryptophan, phenylalanine, glycine, histidine, lysine, leucine, serine, threonine, or valine at position 2,088*(Am)* and a glycine or threonine at position 2,078*(Am)*. In one example, the acetyl-Coenzyme A carboxylase will have an arginine, tryptophan, phenylalanine, glycine, histidine, lysine, leucine, serine, threonine, or valine at position 2,088*(Am)* and an alanine or serine at position 2,096*(Am)*. In one example, the acetyl-Coenzyme A carboxylase will have an arginine, tryptophan, phenylalanine, glycine, histidine, lysine, leucine, serine, threonine, or valine at position 2,088(*Am*) and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,095*(Am)* and at one or more additional amino acid positions. Acetyl-Coenzyme A carboxylase enzymes described herein will typically have a glutamic acid at position 2,095*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am*), an arginine or tryptophan, phenylalanine, glycine, histidine, lysine, leucine, serine, threonine, or valine at position 2,088*(Am)*, an alanine or serine at position 2,096*(Am)*, and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,096*(Am)* and at one or more additional amino acid positions. Acetyl-Coenzyme A carboxylase enzymes described herein will typically have an alanine or serine at position 2,096*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am),* a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am),* an arginine, tryptophan, phenylalanine, glycine, histidine, lysine, leucine, serine, threonine, or valine at position 2,088*(Am)*, a glutamic acid at position 2,095*(Am)*, and an alanine, glycine, proline, histidine, cysteine, or serine at position 2,098*(Am)*. In one example, the acetyl-Coenzyme A carboxylase will have an alanine or serine at position 2,096*(Am)* and a leucine, a threonine or an alanine at position 1,781*(Am).* In one example, the acetyl-Coenzyme A carboxylase will have an alanine or serine at position 2,096*(Am)* and a glycine at position 1,785*(Am)*. In one example, the acetyl-Coenzyme A carboxylase will have an alanine or serine at position 2,096*(Am)* and a proline at position 1,786*(Am)*. In one example, the acetyl-Coenzyme A carboxylase will have an alanine or serine at position 2,096*(Am)* and an asparagine at position 1,811*(Am)*. In one example, the acetyl-Coenzyme A carboxylase will have an alanine or serine at position 2,096*(Am)* and a cysteine or glycine at position 1,999*(Am)*. In one example, the acetyl-Coenzyme A carboxylase will have an alanine or serine at position 2,096*(Am)* and a cysteine or arginine at position 2,027*(Am)*. In one example, the acetyl-Coenzyme A carboxylase will have an alanine or serine at position 2,096*(Am)* and an isoleucine at position 2,041*(Am)*. In one example, the acetyl-Coenzyme A carboxylase will have an alanine or serine at position 2,096*(Am)* and a phenylalanine, isoleucine or leucine at position 2,049*(Am)*. In one example, the acetyl-Coenzyme A carboxylase will have an alanine or serine at position 2,096*(Am)* and a leucine at position 2,074*(Am)*. In one example, the acetyl-Coenzyme A carboxylase will have an alanine or serine at position 2,096*(Am)* and a leucine, isoleucine, methionine or additional valine at position 2,075*(Am)*. In one example, the acetyl-Coenzyme A carboxylase will have an alanine or serine at position 2,096*(Am)* and a glycine or threonine at position 2,078*(Am)*. In one example, the acetyl-Coenzyme A carboxylase will have an alanine or serine at position 2,096*(Am)* and an an arginine, tryptophan, phenylalanine, glycine, histidine, lysine, leucine, serine, threonine, or valine at position 2,088*(Am)*. In one example, the acetyl-Coenzyme A carboxylase will have an alanine or serine at position 2,096*(Am)* and an alanine, glycine, proline, histidine, cysteine, or serine at position 2,098*(Am)*.

In one example, the acetyl-Coenzyme A carboxylase differs from the corresponding wild-type acetyl-Coenzyme A carboxylase at amino acid position 2,098*(Am)* and at one or more additional amino acid positions. Acetyl-Coenzyme A carboxylase enzymes described herein will typically have an alanine, glycine, proline, histidine, cysteine, or serine at position 2,098*(Am)*. In addition, the enzymes will also comprise one or more of a leucine, threonine, valine, or alanine at position 1,781*(Am)*, a glycine at position 1,785*(Am)*, a proline at position 1,786*(Am)*, an asparagine at position 1,811*(Am)*, a proline at position 1,824*(Am)*, a phenylalanine at position 1,864*(Am)*, a cysteine or glycine at position 1,999*(Am)*, a cysteine or arginine at position 2,027*(Am)*, a glycine at position 2,039*(Am)*, an asparagine at position 2,041*(Am)*, a phenylalanine, isoleucine or leucine at position 2,049*(Am)*, a valine at position 2,059*(Am)*, a leucine at position 2,074*(Am)*, a leucine, isoleucine , methionine or additional valine at position 2,075*(Am)*, a glycine or threonine at position 2,078*(Am)*, a phenylalanine at position 2,079*(Am)*, a glutamic acid at position 2,080*(Am)*, a deletion at position 2,080*(Am)*, a deletion at position 2,081*(Am)*, an arginine, tryptophan, phenylalanine, glycine, histidine, lysine, leucine, serine, threonine, or valine at position 2,088*(Am)*, a glutamic acid at position 2,095*(Am)*, and an alanine or serine at position 2,096*(Am)*. In one example, the acetyl-Coenzyme A carboxylase will have an alanine, glycine, proline, histidine, cysteine, or serine at position 2,098*(Am)* and a leucine, a threonine, valine, or an alanine at position 1,781*(Am)*. In one example, the acetyl-Coenzyme A carboxylase will have an alanine, glycine, proline, histidine, cysteine, or serine at position 2,098*(Am)* and a glycine at position 1,785*(Am)*. In one example, the acetyl-Coenzyme A carboxylase will have an alanine, glycine, proline, histidine, cysteine, or serine at position 2,098*(Am)* and a proline at position 1,786*(Am)*. In one example, the acetyl-Coenzyme A carboxylase will have an alanine, glycine, proline, histidine, cysteine, or serine at position 2,098*(Am)* and an asparagine at position 1,811*(Am)*. In one example, the acetyl-Coenzyme A carboxylase will have an alanine, glycine, proline, histidine, cysteine, or serine at position 2,098*(Am)* and a cysteine or glycine at position 1,999*(Am)*. In one example, the acetyl-Coenzyme A carboxylase will have an alanine, glycine, proline, histidine, cysteine, or serine at position 2,098*(Am)* and a cysteine or arginine at position 2,027*(Am)*. In one example, the acetyl-Coenzyme A carboxylase will have an alanine, glycine, proline, histidine, cysteine, or serine at position 2,098*(Am)* and an isoleucine at position 2,041*(Am)*. In one example, the acetyl-Coenzyme A carboxylase will have an alanine, glycine, proline, histidine, cysteine, or serine at position 2,098*(Am)* and a phenylalanine, isoleucine or leucine at position 2,049*(Am)*. In one example, the acetyl-Coenzyme A carboxylase will have an alanine, glycine, proline, histidine, cysteine, or serine at position 2,098*(Am)* and a leucine at position 2,074*(Am)*. In one example, the acetyl-Coenzyme A carboxylase will have an alanine, glycine, proline, histidine, cysteine, or serine at position 2,098*(Am)* and a leucine, isoleucine, methionine or additional valine at position 2,075*(Am)*. In one example, the acetyl-Coenzyme A carboxylase will have an alanine, glycine, proline, histidine, cysteine, or serine at position 2,098*(Am)* and a glycine or threonine at position 2,078*(Am)*. In one example, the acetyl-Coenzyme A carboxylase will have an alanine, glycine, proline, histidine, cysteine, or serine at position 2,098*(Am)* and an arginine or tryptophan, phenylalanine, glycine, histidine, lysine, leucine, serine, threonine, or valine at position 2,088*(Am)*. In one example, the acetyl-Coenzyme A carboxylase will have an alanine, glycine, proline, histidine, cysteine, or serine at position 2,098*(Am)* and an alanine or serine at position 2,096*(Am)*.

Further examples include acetyl-Coenzyme A carboxylases having an isoleucine at position 2,075*(Am)* and a glycine at position 1,999*(Am)*; acetyl-Coenzyme A carboxylases having a methionine at position 2,075*(Am)* and a glutamic acid at position 2,080*(Am)*; acetyl-Coenzyme A carboxylases having a methionine at position 2,075*(Am)* and a glutamic acid at position 2,095*(Am)*; acetyl-Coenzyme A carboxylases having a glycine at position 2,078*(Am)* and a valine at position 2,041*(Am)*; acetyl-Coenzyme A carboxylases having a glycine at position 2,078*(Am)* and a glycine at position 2,039*(Am)*; acetyl-Coenzyme A carboxylases having a glycine at position 2,078*(Am)* and an alanine at position 2,049*(Am)*; acetyl-Coenzyme A carboxylases having a glycine at position 2,078*(Am)* and a cysteine at position 2,049*(Am)*; acetyl-Coenzyme A carboxylases having a glycine at position 2,078*(Am)* and a serine at position 2,049*(Am)*; acetyl-Coenzyme A carboxylases having a glycine at position 2,078*(Am)* and a threonine at position 2,049*(Am)*; acetyl-Coenzyme A carboxylases having a glycine at position 2,078*(Am)* and a valine at position 2,059*(Am)*; acetyl-Coenzyme A carboxylases having a glycine at position 2,078*(Am)* and a phenylalanine at position 2,079*(Am)*; acetyl-Coenzyme A carboxylases having a glycine at position 2,078*(Am)* and a proline at position at position 2,079*(Am)*; and acetyl-Coenzyme A carboxylases having a glycine at position 2,078*(Am)* and a glycine at position 2,088*(Am)*.

Preferred examples include acetyl-Coenzyme A carboxylases having a leucine at position 1,781*(Am)* and a proline at position 1,824*(Am)*; acetyl-Coenzyme A carboxylases having a leucine at position 1,781*(Am)* and an arginine at position 2027*(Am)*; and acetyl-Coenzyme A carboxylases having a glycine at position 2,078*(Am)* and a proline at position 1,824*(Am)*.

More preferred examples include acetyl-Coenzyme A carboxylases having a leucine at position 1,781*(Am)* and a phenylalanine at position 2,049*(Am)*; acetyl-Coenzyme A carboxylases having an alanine at position 2,098*(Am)* and a leucine at position 2,049*(Am)*; acetyl-Coenzyme A carboxylases having an alanine at position 2,098*(Am)* and a histidine at position 2088*(Am)*; acetyl-Coenzyme A carboxylases having an alanine at position 2,098*(Am)* and a phenylalanine at position 2,088*(Am)*; acetyl-Coenzyme A carboxylases having an alanine at position 2,098*(Am)* and a lysine at position 2,088*(Am)*; acetyl-Coenzyme A carboxylases having an alanine at position 2,098*(Am)* and a leucine at position 2,088*(Am)*; acetyl-Coenzyme A carboxylases having an alanine at position 2,098*(Am)* and a threonine at position 2,088*(Am)*; acetyl-Coenzyme A carboxylases having a glycine at position 2,098*(Am)* and a glycine at position 2,088*(Am)*; acetyl-Coenzyme A carboxylases having a glycine at position 2,098*(Am)* and a histidine at position 2,088*(Am)*; acetyl-Coenzyme A carboxylases having a glycine at position 2,098*(Am)* and leucine at position 2,088*(Am)*; acetyl-Coenzyme A carboxylases having a glycine at position 2,098*(Am)* and a serine at position 2,088*(Am)*; acetyl-Coenzyme A carboxylases having a glycine at position 2,098*(Am)* and threonine at position 2,088*(Am)*; acetyl-Coenzyme A carboxylases having a glycine at position 2,098*(Am)* and a valine at position 2,088*(Am)*; acetyl-Coenzyme A carboxylases having a cysteine at position 2,098*(Am)* and a tryptophan at position 2088*(Am)*; acetyl-Coenzyme A carboxylases having a serine at position 2,098*(Am)* and a tryptophan at position 2088*(Am)*; and acetyl-Coenzyme A carboxylases having a deletion at position 2,080*(Am)* and a deletion at position 2081*(Am)*.

Most preferred examples include acetyl-Coenzyme A carboxylases having a leucine at position 1,781*(Am)* and a asparagine at position 2,041*(Am)*; acetyl-Coenzyme A carboxylases having a leucine at position 1,781*(Am)* and a cysteine at position 2,027*(Am)*; acetyl-Coenzyme A carboxylases having a leucine at position 1,781*(Am)* and a leucine at position 2,075*(Am)*; acetyl-Coenzyme A carboxylases having a leucine at position 1,781*(Am)* and a phenylalanine at position 1,864*(Am)*; acetyl-Coenzyme A carboxylases having a leucine at position 1,781*(Am)* and an alanine at position 2098*(Am)*; acetyl-Coenzyme A carboxylases having a leucine at position 1,781*(Am)* and a glycine at position 2,098*(Am)*; acetyl-Coenzyme A carboxylases having a leucine at position 1,781*(Am)* and a duplication 2,075*(Am)*; acetyl-Coenzyme A carboxylases having a glycine at position 1,999*(Am)* and a phenylalanine at position 1,864*(Am)*; acetyl-Coenzyme A carboxylases having a glycine at position 1,999*(Am)* and isoleucine at position 2,049*(Am)*; acetyl-Coenzyme A carboxylases having a glycine at position 1,999*(Am)* and leucine at position 2,075*(Am)*; and acetyl-Coenzyme A carboxylases having a glycine at position 1,999*(Am)* and alanine at position 2,098*(Am)*.

### Nucleic acid molecules:

Further, nucleic acid molecules are described herein that encode all or a portion of the acetyl-Coenzyme A carboxylase enzymes described above. Nucleic acid molecules described herein may comprise a nucleic acid sequence encoding an amino acid sequence comprising a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2049*(Am)* is phenylalanine, isoleucine or leucine; the amino acid at position 2,059*(Am)* is valine; the amino acid at position 2,074*(Am)* is leucine; the amino acid at position 2,075*(Am)* is leucine, isoleucine , methionine or additional valine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences. In some examples, the nucleic acid molecule may encode an acetyl-Coenzyme A carboxylase having multiple differences from the wild type acetyl-Coenzyme A carboxylase as described above.

In one example, the nucleic acid molecule encodes an acetyl-Coenzyme A carboxylase which differs from the acetyl-Coenzyme A carboxylase of the corresponding wild-type plant at only one of the following positions: 1,781*(Am)*, 1,785*(Am)*, 1,786*(Am)*, 1,811*(Am)*, 1,824*(Am)*, 1,864*(Am)*, 1,999*(Am)*, 2,027*(Am)*, 2,039*(Am)*, 2,041*(Am)*, 2,049*(Am)*, 2,059*(Am)*, 2,074*(Am)*, 2,075*(Am)*, 2,078*(Am*), 2,079*(Am)*, 2,080*(Am)*, 2,081*(Am)*, 2,088*(Am)*, 2,095*(Am)*, 2,096*(Am)*, or 2,098*(Am)*. In one example, the acetyl-Coenzyme A carboxylase of an herbicide-tolerant plant described herein will differ at only one of the following positions: 2,078*(Am)*, 2,088*(Am)*, or 2,075*(Am)*. In a preferred example, the acetyl-Coenzyme A carboxylase of an herbicide-tolerant plant described herein will differ at only one of the following positions: 2,039*(Am)*, 2,059*(Am)*, 2,080*(Am)*, or 2,095*(Am)*. In a more preferred example, the acetyl-Coenzyme A carboxylase of an herbicide-tolerant plant described herein will differ at only one of the following positions: 1,785*(Am)*, 1,786*(Am)*, 1,811*(Am)*, 1,824*(Am)*, 1,864*(Am)*, 2,041*(Am)*, 2,049*(Am)*, 2,074*(Am)*, 2,079*(Am)*, 2,081*(Am),* 2,096*(Am),* or 2,098*(Am).* In a most preferred example, the acetyl-Coenzyme A carboxylase of an herbicide-tolerant plant described herein will differ at only one of the following positions: 1,781*(Am)*, 1,999*(Am)*, 2,027*(Am)*, 2,041*(Am)*, or 2,096*(Am)*.

In one example, the nucleic acid molecule encodes an acetyl-Coenzyme A carboxylase having only one of the following substitutions: isoleucine at position 2,075*(Am)*, glycine at position 2,078*(Am)*, or arginine at position 2,088*(Am)*. In a preferred example, the nucleic acid molecule encodes an acetyl-Coenzyme A carboxylase having only one of the following substitutions: glycine at position 2,039*(Am)*, valine at position 2,059*(Am)*, methionine at position 2,075*(Am)*, duplication of position 2,075*(Am)* (i.e., an insertion of valine between 2,074*(Am)* and 2,075*(Am)*, or an insertion of valine between position 2,075*(Am)* and 2,076*(Am)*, deletion of amino acid position 2,088*(Am)*, glutamic acid at position 2,080*(Am)*, deletion of position 2,088*(Am)*, or glutamic acid at position 2,095*(Am)*. In a more preferred example, the nucleic acid molecule encodes an acetyl-Coenzyme A carboxylase having only one of the following substitutions: a glycine at position 1,785*(Am)*, a proline at position 1,786*(Am)*, an asparagine at position 1,811*(Am),* a leucine at position 2,075*(Am)*, a methionine at position 2,075*(Am)*, a threnonine at position 2,078*(Am)*, a deletion at position 2,080*(Am)*, a deletion at position 2,081*(Am),* a tryptophan at position 2,088*(Am)*, a serine at position 2,096*(Am)*, an alanine at position 2,096*(Am)*, an alanine at position 2,098*(Am)*, a glycine at position 2,098*(Am)*, an histidine at position 2,098*(Am)*, a proline at position 2,098(*Am*), or a serine at position 2,098(Am). In a most preferred example, the nucleic acid molecule encodes an acetyl-Coenzyme A carboxylase having only one of the following substitutions: a leucine at position 1,781(*Am*), a threonine at position 1,781(*Am*), a valine at position 1,781(*Am*), an alanine at position 1,781(*Am*), a glycine at position 1,999(*Am*), a cysteine at position 2,027(Am), an arginine at position 2,027(Am), an asparagine at position 2,041*(Am)*, a valine at position 2,041*(Am)*, an alanine at position 2,096*(Am)*, and a serine at position 2,096*(Am)*.

In one example, the nucleic acid molecule may encode an acetyl-Coenzyme A carboxylase comprising a leucine, threonine, valine, or an alanine at position 1,781*(Am)* and a cysteine or glycine at position 1,999*(Am)*. In one example, the nucleic acid molecule may encode an acetyl-Coenzyme A carboxylase comprising a leucine, threonine, valine, or an alanine at position 1,781*(Am)* and a cysteine or arginine at position 2,027*(Am)*. In one example, the nucleic acid molecule may encode an acetyl-Coenzyme A carboxylase comprising a leucine, threonine, valine, or an alanine at position 1,781*(Am)* and an asparagine at position 2,041*(Am)*. In one example, the nucleic acid molecule may encode an acetyl-Coenzyme A carboxylase comprising a leucine, threonine, valine, or an alanine at position 1,781*(Am)* and a phenylalanine, isoleucine or leucine at position 2,049*(Am)*. In one example, the nucleic acid molecule may encode an acetyl-Coenzyme A carboxylase comprising a leucine, threonine, valine, or an alanine at position 1,781*(Am)* and a leucine or isoleucine at position 2,075*(Am)*. In one example, the nucleic acid molecule may encode an acetyl-Coenzyme A carboxylase comprising a leucine, threonine, valine, or an alanine at position 1,781*(Am)* and a glycine at position 2,078*(Am)*. In one example, the nucleic acid molecule may encode an acetyl-Coenzyme A carboxylase comprising a leucine, threonine, valine, or an alanine at position 1,781*(Am)* and an arginine at position 2,088*(Am)*. In one example, the nucleic acid molecule may encode an acetyl-Coenzyme A carboxylase comprising a leucine, threonine, valine, or an alanine at position 1,781*(Am)* and an alanine at position 2,096*(Am)*. In one example, the nucleic acid molecule may encode an acetyl-Coenzyme A carboxylase comprising a leucine, threonine, valine, or an alanine at position 1,781*(Am)* and an alanine at position 2,098*(Am)*. In one example, the nucleic acid molecule may encode an acetyl-Coenzyme A carboxylase comprising a leucine, threonine, valine, or an alanine at position 1,781*(Am)*, a cysteine at position 2,027*(Am)*, and an asparagine at position 2,041*(Am)*. In one example, the nucleic acid molecule may encode an acetyl-Coenzyme A carboxylase comprising a leucine, threonine, valine, or an alanine at position 1,781*(Am)*, a cysteine at position 2,027*(Am)*, an asparagine at position 2,041*(Am)*, and an alanine at position 2,096*(Am)*.

Further examples include a nucleic acid molecule encoding an acetyl-Coenzyme A carboxylase having an isoleucine at position 2,075*(Am)* and a glycine at position 1,999*(Am)*; a nucleic acid molecule encoding an acetyl-Coenzyme A carboxylase having a methionine at position 2,075*(Am)* and a glutamic acid at position 2,080*(Am)*; a nucleic acid molecule encoding an acetyl-Coenzyme A carboxylase having a methionine at position 2,075*(Am)* and a glutamic acid at position 2,095*(Am)*; a nucleic acid molecule encoding an acetyl-Coenzyme A carboxylase having a glycine at position 2,078*(Am)* and a valine at position 2,041*(Am)*; a nucleic acid molecule encoding an acetyl-Coenzyme A carboxylase having a glycine at position 2,078*(Am)* and a glycine at position 2,039*(Am)*; a nucleic acid molecule encoding an acetyl-Coenzyme A carboxylase having a glycine at position 2,078*(Am)* and an alanine at position 2,049*(Am)*; a nucleic acid molecule encoding an acetyl-Coenzyme A carboxylase having a glycine at position 2,078*(Am)* and a cysteine at position 2,049*(Am)*; a nucleic acid molecule encoding an acetyl-Coenzyme A carboxylase having a glycine at position 2,078*(Am)* and a serine at position 2,049*(Am)*; a nucleic acid molecule encoding an acetyl-Coenzyme A carboxylase having a glycine at position 2,078*(Am)* and a threonine at position 2,049*(Am)*; a nucleic acid molecule encoding an acetyl-Coenzyme A carboxylase having a glycine at position 2,078*(Am)* and a valine at position 2,059*(Am)*; a nucleic acid molecule encoding an acetyl-Coenzyme A carboxylase having a glycine at position 2,078*(Am)* and a phenylalanine at position 2,079*(Am)*; a nucleic acid molecule encoding an acetyl-Coenzyme A carboxylase having a glycine at position 2,078*(Am)* and a proline at position at position 2,079*(Am)*; or a nucleic acid molecule encoding an acetyl-Coenzyme A carboxylase having a glycine at position 2,078*(Am)* and a glycine at position 2,088*(Am)*.

Preferred examples include a nucleic acid molecule encoding an acetyl-Coenzyme A carboxylase having a leucine at position 1,781*(Am)* and a proline at position 1,824*(Am)*; a nucleic acid molecule encoding an acetyl-Coenzyme A carboxylase having a leucine at position 1,781*(Am)* and an arginine at position 2027*(Am)*; or a nucleic acid molecule encoding an acetyl-Coenzyme A carboxylase having a glycine at position 2,078*(Am)* and a proline at position 1,824*(Am)*.

More preferred examples include a nucleic acid molecule encoding an acetyl-Coenzyme A carboxylase having a leucine at position 1,781*(Am)* and a phenylalanine at position 2,049*(Am)*; a nucleic acid molecule encoding an acetyl-Coenzyme A carboxylase having an alanine at position 2,098*(Am)* and a leucine at position 2,049*(Am)*; a nucleic acid molecule encoding an acetyl-Coenzyme A carboxylase having an alanine at position 2,098*(Am)* and a histidine at position 2088*(Am)*; a nucleic acid molecule encoding an acetyl-Coenzyme A carboxylase having an alanine at position 2,098*(Am)* and a phenylalanine at position 2,088*(Am)*; a nucleic acid molecule encoding an acetyl-Coenzyme A carboxylase having an alanine at position 2,098*(Am)* and a lysine at position 2,088*(Am)*; a nucleic acid molecule encoding an acetyl-Coenzyme A carboxylase having an alanine at position 2,098*(Am)* and a leucine at position 2,088*(Am)*; a nucleic acid molecule encoding an acetyl-Coenzyme A carboxylase having an alanine at position 2,098*(Am)* and a threonine at position 2,088*(Am)*; a nucleic acid molecule encoding an acetyl-Coenzyme A carboxylase having a glycine at position 2,098*(Am)* and a glycine at position 2,088*(Am)*; a nucleic acid molecule encoding an acetyl-Coenzyme A carboxylase having a glycine at position 2,098*(Am)* and a histidine at position 2,088*(Am)*; a nucleic acid molecule encoding an acetyl-Coenzyme A carboxylase having a glycine at position 2,098(*Am*) and leucine at position 2,088*(Am)*; a nucleic acid molecule encoding an acetyl-Coenzyme A carboxylase having a glycine at position 2,098*(Am)* and a serine at position 2,088*(Am)*; a nucleic acid molecule encoding an acetyl-Coenzyme A carboxylase having a glycine at position 2,098*(Am)* and threonine at position 2,088*(Am)*; a nucleic acid molecule encoding an acetyl-Coenzyme A carboxylase having a glycine at position 2,098*(Am)* and a valine at position 2,088*(Am)*; a nucleic acid molecule encoding an acetyl-Coenzyme A carboxylase having a cysteine at position 2,098*(Am)* and a tryptophan at position 2088*(Am)*; a nucleic acid molecule encoding an acetyl-Coenzyme A carboxylase having a serine at position 2,098*(Am)* and a tryptophan at position 2088*(Am)*; or a nucleic acid molecule encoding an acetyl-Coenzyme A carboxylase having a deletion at position 2,080*(Am)* and a deletion at position 2081*(Am).*

Most preferred examples include a nucleic acid molecule encoding an acetyl-Coenzyme A carboxylase having a leucine at position 1,781*(Am)* and a asparagine at position 2,041*(Am)*; a nucleic acid molecule encoding an acetyl-Coenzyme A carboxylase having a leucine at position 1,781*(Am)* and a cysteine at position 2,027*(Am)*; a nucleic acid molecule encoding an acetyl-Coenzyme A carboxylase having a leucine at position 1,781*(Am)* and a leucine at position 2,075*(Am)*; a nucleic acid molecule encoding an acetyl-Coenzyme A carboxylase having a leucine at position 1,781*(Am)* and a phenylalanine at position 1,864*(Am)*; a nucleic acid molecule encoding an acetyl-Coenzyme A carboxylase having a leucine at position 1,781*(Am)* and an alanine at position 2098*(Am)*; a nucleic acid molecule encoding an acetyl-Coenzyme A carboxylase having a leucine at position 1,781*(Am)* and a glycine at position 2,098*(Am)*; a nucleic acid molecule encoding an acetyl-Coenzyme A carboxylase having a leucine at position 1,781*(Am)* and a duplication 2,075*(Am)*; a nucleic acid molecule encoding an acetyl-Coenzyme A carboxylase having a glycine at position 1,999*(Am)* and a phenylalanine at position 1,864*(Am)*; a nucleic acid molecule encoding an acetyl-Coenzyme A carboxylase having a glycine at position 1,999*(Am)* and isoleucine at position 2,049*(Am)*; a nucleic acid molecule encoding an acetyl-Coenzyme A carboxylase having a glycine at position 1,999*(Am)* and leucine at position 2,075*(Am)*; or a nucleic acid molecule encoding an acetyl-Coenzyme A carboxylase having a glycine at position 1,999*(Am)* and alanine at position 2,098*(Am)*.

Rice plants are described herein which comprise nucleic acids encoding Acetyl-Coenzyme A carboxylase polypeptide having one or more substitutions as described above.

BEP clade plants are described herein which comprise nucleic acids encoding Acetyl-Coenzyme A carboxylase polypeptide having one or more substitutions as described above.

BET subclade plants are described herein which comprise nucleic acids encoding Acetyl-Coenzyme A carboxylase polypeptide having one or more substitutions as described above.

BET crop plants are described herein which comprise nucleic acids encoding Acetyl-Coenzyme A carboxylase polypeptide having one or more substitutions as described above.

Monocot plants are described herein which comprise nucleic acids encoding Acetyl-Coenzyme A carboxylase polypeptide having one or more substitutions as described above.

The nucleic acid molecule described herein may be DNA, derived from genomic DNA or cDNA, or RNA. The nucleic acid molecule described herein may be naturally occurring or may be synthetic. The nucleic acid molecule described herein may be isolated, recombinant and/or mutagenized.

In one example, the nucleic acid molecule encodes an acetyl-Coenzyme A carboxylase enzyme in which the amino acid at position 1,781*(Am)* is leucine or alanine or is complementary to such a nucleic acid molecule. Such nucleic acid molecules include, but are not limited to, genomic DNA that serves as a template for a primary RNA transcription, a plasmid molecule encoding the acetyl-Coenzyme A carboxylase, as well as an mRNA encoding such an acetyl-Coenzyme A carboxylase.

The nucleic acid molecules described herein may comprise non-coding sequences, which may or may not be transcribed. Non-coding sequences that may be included in the nucleic acid molecules include, but are not limited to, 5' and 3' UTRs, polyadenylation signals and regulatory sequences that control gene expression (e.g., promoters). The nucleic acid molecules described herein may also comprise sequences encoding transit peptides, protease cleavage sites, covalent modification sites and the like. In one example, the nucleic acid molecules encode a chloroplast transit peptide sequence in addition to a sequence encoding an acetyl-Coenzyme A carboxylase enzyme.

In another example, the nucleic acid molecules may encode an acetyl-Coenzyme A carboxylase enzyme having at least 50%, 60%, 70%, 75%, 80%, 85%, 90%, 95% or more sequence identity to a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2049*(Am)* is phenylalanine, leucine or isoleucine; the amino acid at position 2,059*(Am)* is valine; the amino acid at position 2,074*(Am)* is leucine; the amino acid at position 2,075*(Am)* is leucine, isoleucine or methionine or an additional valine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences.

As used herein, "percent (%) sequence identity" is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program BLAST available at http://blast.ncbi.nlm.nih.gov/Blast.cgi with search parameters set to default values.

Further described herein are nucleic acid molecules that hybridize to nucleic acid molecules encoding an acetyl-Coenzyme A carboxylase as described herein as well as nucleic acid molecules that hybridize to the reverse complement of nucleic acid molecules encoding an acetyl-Coenzyme A carboxylase as described herein. In one example, the nucleic acid molecules comprise nucleic acid molecules that hybridize to a nucleic acid molecule encoding one or more of a modified version of one or both of SEQ ID NOs: 2 and 3, wherein the sequence is modified such that the encoded protein comprises one or more of the following: the amino acid at position 1,781*(Am)* is leucine, threonine, valine, or alanine; the amino acid at position 1,785*(Am)* is glycine; the amino acid at position 1,786*(Am)* is proline; the amino acid at position 1,811*(Am)* is asparagine; the amino acid at position 1,824*(Am)* is proline; the amino acid at position 1,864*(Am)* is phenylalanine; the amino acid at position 1,999*(Am)* is cysteine or glycine; the amino acid at position 2,027*(Am)* is cysteine or arginine; the amino acid at position 2,039*(Am)* is glycine; the amino acid at position 2,041*(Am)* is asparagine; the amino acid at position 2049*(Am)* is phenylalanine, isoleucine or leucine; the amino acid at position 2,059*(Am)* is valine; the amino acid at position 2,074*(Am)* is leucine; the amino acid at position 2,075*(Am)* is leucine, isoleucine or methionine or an additional valine; the amino acid at position 2,078*(Am)* is glycine, or threonine; the amino acid at position 2,079*(Am)* is phenylalnine; the amino acid at position 2,080*(Am)* is glutamic acid; the amino acid at position 2,080*(Am)* is deleted; the amino acid at position 2,081*(Am)* is deleted; the amino acid at position 2,088*(Am)* is arginine, tryptophan, phenylalanine, glycine, histidine, lysine, leucine, serine, threonine, or valine; the amino acid at position 2,095*(Am)* is glutamic acid; the amino acid at position 2,096*(Am)* is alanine, or serine; or the amino acid at position 2,098*(Am)* is alanine, glycine, proline, histidine, or serine, as well as nucleic acid molecules complementary to all or a portion of the coding sequences, or the reverse complement of such nucleic acid molecules under stringent conditions. The stringency of hybridization can be controlled by temperature, ionic strength, pH, and the presence of denaturing agents such as formamide during hybridization and washing. Stringent conditions that may be used include those defined in Current Protocols in Molecular Biology, Vol. 1, Chap. 2.10, John Wiley & Sons, Publishers (1994) and Sambrook et al., Molecular Cloning, Cold Spring Harbor (1989) which relate to teaching stringent conditions.

Any of the mutants described above in a plasmid with a combinatinon of the gene of interest can be used in transformation.

Expression vectors are described herein which comprise nucleic acid molecules encoding any of the ACCase mutants described above.

The use of mutant ACCase nucleic acids and proteins encoded by such mutant ACCase nucleic acids as described above as selectable markers is described herein.

The nucleic acid molecules described herein include oligonucleotides that may be used as hybridization probes, sequencing primers, and/or PCR primers. Such oligonucleotides may be used, for example, to determine a codon sequence at a particular position in a nucleic acid molecule encoding an acetyl-Coenzyme A carboxylase, for example, by allele specific PCR. Such oligonucleotides may be from about 15 to about 30, from about 20 to about 30, or from about 20-25 nucleotides in length.

Test for double mutant ACCase genes "DBLM Assay":
(1) In a test population (of, e.g., at least 12 and preferably at least 20) whole rice plants containing 1 or 2 copies of a transgenic ACCase gene encoding an at-least-double-mutant ACCase (i.e. 1 min. and 2 max. chromosomal insertions of the transgenic ACCase gene to be tested),
   wherein the rice plants are T0 ("T-zero") regenerants
   and in parallel with a control population of such plants to be used as untreated check plants;
(2) Application to the test population at 200 L / ha spray volume of a composition comprising Tepraloxydim (AI) and 1% Crop Oil Concentrate (COC), to provide an AI application rate equivalent to 50 g/ha of Tepraloxydim (AI);
(3) Determining a phytotoxicity score for each test and check plant, based on a traditional plant injury rating system (e.g., evaluating visual evidence of herbicide burn, leaf morphology changes, wilt, yellowing, and other morphological characteristics, preferably according to a typical, at least-5-level injury rating scale);
(4) Analyzing the collected data to determine whether at least 75% of the plants in the test population exhibit an average phytotoxicity, i.e. increase in injury relative to check plants, of less than 10%; and
(5) Identifying a positive result so determined as demonstrating that the double-mutant ACCase provides an acceptable AIT.

### Herbicides

Plants, e.g., rice plants, are described herein that are tolerant of concentrations of herbicide that normally inhibit the growth of wild-type plants. The plants are typically resistant to herbicides that interfere with acetyl-Coenzyme A carboxylase activity. Any herbicide that inhibits acetyl-Coenzyme A carboxylase activity can be used in conjunction with the plants described herein. Suitable examples include, but are not limited to, cyclohexanedione herbicides, aryloxyphenoxy propionate herbicides, and phenylpyrazole herbicides. In some methods of controlling weeds and/or growing herbicide-tolerant plants, at least one herbicide is selected from the group consisting of sethoxydim, cycloxydim, tepraloxydim, haloxyfop, haloxyfop-P or a derivative of any of these herbicides. Table 1 provides a list of cyclohexanedione herbicides (DIMs, also referred to as: cyclohexene oxime cyclohexanedione oxime; and CHD) that interfere with acetyl-Coenzyme A carboxylase activity and may be used in conjunction with the herbicide-tolerant plants described herein. One skilled in the art will recognize that other herbicides in this class exist and may be used in conjunction with the herbicide-tolerant plants described herein. Also included in Table 1 is a list of aryloxyphenoxy propionate herbicides (also referred to as aryloxyphenoxy propanoate; aryloxyphenoxyalkanoate; oxyphenoxy; APP; AOPP; APA; APPA; FOP, note that these are sometime written with the suffix '-oic') that interfere with acetyl-Coenzyme A carboxylase activity and may be used in conjunction with the herbicide-tolerant plants described herein. One skilled in the art will recognize that other herbicides in this class exist and may be used in conjunction with the herbicide-tolerant plants described herein.

**Table 1**

| **ACCase Inhibitor** | **Class** | **Company** | **Examples of Synonyms and Trade Names** |
|---|---|---|---|
| alloxydim | DIM | BASF | Fervin, Kusagard, NP-48Na, BAS 9021H, Carbodimedon, Zizalon |
| butroxydim | DIM | Syngenta | Falcon, ICI-A0500, Butroxydim |
| clethodim | DIM | Valent | Select, Prism, Centurion, RE-45601, Motsa |
| Clodinafop-propargyl | FOP | Syngenta | Discover, Topik, CGA 184 927 |
| clofop | FOP | | Fenofibric Acid, Alopex |
| cloproxydim | FOP | | |
| chlorazifop | FOP | | |
| cycloxydim | DIM | BASF | Focus, Laser, Stratos, BAS 517H |
| cyhalofop-butyl | FOP | Dow | Clincher, XDE 537, DEH 112, Barnstorm |
| diclofop-methyl | FOP | Bayer | Hoegrass, Hoelon, Illoxan, HOE 23408, Dichlorfop, Illoxan |
| fenoxaprop-P-ethyl | FOP | Bayer | Super Whip, Option Super, Exel Super, HOE-46360, Aclaim, Puma S, Fusion |
| fenthiaprop | FOP | | Taifun; Joker |
| fluazifop-P-butyl | FOP | Syngenta | Fusilade, Fusilade 2000, Fusilade DX, ICI-A 0009, ICI-A 0005, SL-236, IH-773B, TF-1169, Fusion |
| haloxyfop-etotyl | FOP | Dow | Gallant, DOWCO 453EE |
| haloxyfop-methyl | FOP | Dow | Verdict, DOWCO 453ME |
| haloxyfop-P-methyl | FOP | Dow | Edge, DE 535 |
| isoxapyrifop | FOP | | |
| Metamifop | FOP | Dongbu | NA |
| pinoxaden | DEN | Syngenta | Axial |
| profoxydim | DIM | BASF | Aura, Tetris, BAS 625H, Clefoxydim |
| propaquizafop | FOP | Syngenta | Agil, Shogun, Ro 17-3664, Correct |
| quizalofop-P-ethyl | FOP | DuPont | Assure, Assure II, DPX-Y6202-3, Targa Super, NC-302, Quizafop |
| quizalofop-P-tefuryl | FOP | Uniroyal | Pantera, UBI C4874 |
| sethoxydim | DIM | BASF | Poast, Poast Plus, NABU, Fervinal, NP-55, Sertin, BAS 562H, Cvethoxydim, Rezult |
| tepraloxydim | DIM | BASF | BAS 620H, Aramo, Caloxydim |
| tralkoxydim | DIM | Syngenta | Achieve, Splendor, ICI-A0604, Tralkoxydime, Tralkoxidym |
| trifop | FOP | | |

In addition to the herbicides listed above, other ACCAse-inhibitors can be used in conjunction with the herbicide-tolerant plants described herein. For example, ACCase-inhibiting herbicides of the phenylpyrazole class, also known as DENs, can be used. An exemplary DEN is pinoxaden, which is a phenylpyrazoline-type member of this class. Herbicide compositions containing pinoxaden are sold under the brands Axial and Traxos.

The herbicidal compositions hereof comprising one or more acetyl-Coenzyme A carboxylase-inhibiting herbicides, and optionally other agronomic A.I.(s), *e.g.,* one or more sulfonylureas (SUs) selected from the group consisting of amidosulfuron, flupyrsulfuron, foramsulfuron, imazosulfuron, iodosulfuron, mesosulfuron, nicosulfuron, thifensulfuron, and tribenuron, agronomically acceptable salts and esters thereof, or one or more imidazolinones selected from the group of imazamox, imazethapyr, imazapyr, imazapic, combinations thereof, and their agriculturally suitable salts and esters, can be used in any agronomically acceptable format. For example, these can be formulated as ready-to-spray aqueous solutions, powders, suspensions; as concentrated or highly concentrated aqueous, oily or other solutions, suspensions or dispersions; as emulsions, oil dispersions, pastes, dusts, granules, or other broadcastable formats. The herbicide compositions can be applied by any means known in the art, including, for example, spraying, atomizing, dusting, spreading, watering, seed treatment, or co-planting in admixture with the seed. The use forms depend on the intended purpose; in any case, they should ensure the finest possible distribution of the active ingredients according to the invention.

Where the optional A.I. includes an herbicide from a different class to which the plant(s) hereof would normally be susceptible, the plant to be used can be selected from among those that further comprise a trait of tolerance to such herbicide. Such further tolerance traits can be provided to the plant by any method known in the art, *e.g.,* including techniques of traditional breeding to obtain a tolerance trait gene by hybridization or introgression, of mutagenesis, and/or of transformation. Such plants can be described as having "stacked" traits.

In addition, any of the above acetyl-Coenzyme A carboxylase-inhibiting herbicides can be combined with one or more herbicides of another class, for example, any of the acetohydroxyacid synthase-inhibiting herbicides, EPSP synthase-inhibiting herbicides, glutamine synthase-inhibiting herbicides, lipid- or pigment-biosynthesis inhibitor herbicides, cell-membrane disruptor herbicides, photosynthesis or respiration inhibitor herbicides, or growth regulator or growth inhibitor herbicides known in the art. Non-limiting examples include those recited in Weed Science Society of America's Herbicide Handbook, 9th Edition edited by S.A. Senseman, copy right 2007. An herbicidal composition herein can contain one or more agricultural active ingredient(s) selected from the agriculturallyacceptable fungicides, strobilurin fungicides, insecticides (including nematicides), miticides, and molluscicides. Non-limiting examples include those recited in 2009 Crop Protection Reference (www.greenbook.net), Vance Publications.

Any of the above acetyl-Coenzyme A carboxylase-inhibiting herbicides can be combined with herbicides which exhibit low damage to rice, whereby the rice tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, azimsulfuron, bensulfuron, chlorimuron, cyclosulfamuron, ethoxysulfuron, flucetosulfuron, halosulfuron, imazosulfuron, metsulfuron, orthosulfamuron, propyrisulfuron, pyrazosulfuron, bispyribac, pyrimisulfan or penoxsulam, the EPSP synthase-inhibiting herbicides glyphosate or sulfosate, the glutamine synthase-inhibiting herbicides glufosinate, glufosinate-P or bialaphos, the lipid biosynthesis inhibitor herbicides benfuresate, molinate or thiobencarb, the photosynthesis inhibitor herbicides bentazon, paraquat, prometryn or propanil, the bleacher herbicides benzobicyclone, clomazone or tefuryltrione, the auxin herbicides 2,4-D, fluroxypyr, MCPA, quinclorac, quinmerac or triclopyr, the microtubule inhibitor herbicide pendimethalin, the VLCFA inhibitor herbicides anilofos, butachlor, fentrazamide, ipfencarbazone, mefenacet, pretilachlor, acetochlor, metolachlor or S-metolachlor or the protoporphyrinogen-IX-oxidase inhibitor herbicides carfentrazone, oxadiazon, oxyfluorfen, pyraclonil or saflufenacil.

Any of the above acetyl-Coenzyme A carboxylase-inhibiting herbicides can be combined with herbicides which exhibit low damage to cereals such as wheat, barley or rye, whereby the cereals tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, amidosulfuron, chlorsulfuron, flucetosulfuron, flupyrsulfuron, iodosulfuron, mesosulfuron, metsulfuron, sulfosulfuron, thifensulfuron, triasulfuron, tribenuron, tritosulfuron, florasulam, pyroxsulam, pyrimisulfan, flucarbazone, propoxycarbazone or thiencarbazone, the EPSP synthase-inhibiting herbicides glyphosate or sulfosate, the glutamine synthase-inhibiting herbicides glufosinate, glufosinate-P or bialaphos, the lipid biosynthesis inhibitor herbicides prosulfocarb, the photosynthesis inhibitor herbicides bentazon, chlorotoluron, isoproturon, ioxynil, bromoxynil, the bleacher herbicides diflufenican, flurtamone, picolinafen or pyrasulfotole, the auxin herbicides aminocyclopyrachlor, aminopyralid, 2,4-D, dicamba, fluroxypyr, MCPA, clopyralid, MCPP, or MCPP-P, the microtubule inhibitor herbicides pendimethalin or trifluralin, the VLCFA inhibitor herbicide flufenacet, or the protoporphyrinogen-IX-oxidase inhibitor herbicides bencarbazone, carfentrazone or saflufenacil, or the herbicide difenzoquat.

Any of the above acetyl-Coenzyme A carboxylase-inhibiting herbicides can be combined with herbicides which exhibit low damage to turf, whereby the turf tolerance to such herbicides may optionally be a result of genetic modifications of the crop plants. Examples of such herbicides are the acetohydroxyacid synthase-inhibiting herbicides imazamethabenz, imazamox, imazapic, imazapyr, imazaquin, imazethapyr, flazasulfuron, foramsulfuron, halosulfuron, trifloxysulfuron, bispyribac or thiencarbazone, the EPSP synthase-inhibiting herbicides glyphosate or sulfosate, the glutamine synthase-inhibiting herbicides glufosinate, glufosinate-P or bialaphos, the photosynthesis inhibitor herbicides atrazine or bentazon, the bleacher herbicides mesotrione, picolinafen, pyrasulfotole or topramezone, the auxin herbicides aminocyclopyrachlor, aminopyralid, 2,4-D, 2,4-DB, clopyralid, dicamba, dichlorprop, dichlorprop-P, fluroxypyr, MCPA, MCPB, MCPP, MCPP-P, quinclorac, quinmerac or trichlopyr, the microtubule inhibitor herbicide pendimethalin, the VLCFA inhibitor herbicides dimethenamide, dimethenamide-P or ipfencarbazone, the protoporphyrinogen-IX-oxidase inhibitor herbicides saflufenacil or sulfentrazone, or the herbicide indaziflam.

Furthermore, any of the above acetyl-Coenzyme A carboxylase-inhibiting herbicides can be combined with safeners. Safeners are chemical compounds which prevent or reduce damage on useful plants without having a major impact on the herbicidal action of the herbicides towards unwanted plants. They can be applied either before sowings (e. g. on seed treatments, shoots or seedlings) or in the pre-emergence application or post-emergence application of the useful plant. The safeners and the aforementioned herbicides can be applied simultaneously or in succession. Suitable safeners are e. g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates. Examples of saferners are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

An herbicidal composition hereof can comprise, e.g., a combination of: auxinic herbicide(s), e.g., dicamba; AHAS-inhibitor(s), e.g., imidazolinone(s) and/or sulfonylurea(s); ACCase-inhibitor(s); EPSPS inhibitor(s), e.g., glyphosate; glutamine synthetase inhibitor(s), e.g.., glufosinate; protoporphyrinogen-IX oxidase (PPO) inhibitor(s), e.g., saflufenacil; fungicide(s), e.g., strobilurin fungicide(s) such as pyraclostrobin; and the like. An herbicidal composition hereof can comprise, e.g., a combination of auxinic herbicide(s), e.g., dicamba; a microtubule inhibitor herbicide, e.g., pendimethalin and strobilurin fungicide(s) such as pyraclostrobin(s). An herbicidal composition will be selected according to the tolerances of a plant hereof, and the plant can be selected from among those having stacked tolerance traits.

The herbicides individually and/or in combination as described herein can be used as pre-mixes or tank mixes. Such herbicides can also be incorporated into an agronomically acceptable compositions.

Those skilled in the art will recognize that some of the above mentioned herbicides and/or safeners are capable of forming geometrical isomers, for example E/Z isomers. It is possible to use both, the pure isomers and mixtures thereof, in the compositions described herein. Furthermore, some of the above mentioned herbicides and/or safeners have one or more centers of chirality and, as a consequence, are present as enantiomers or diastereomers. It is possible to use both, the pure enantiomers and diastereomers and their mixtures, in the compositions described herein. In particular, some of the aryloxyphenoxy propionate herbicides are chiral, and some of them are commonly used in enantiomerically enriched or enantiopure form, e. g. clodinafop, cyhalofop, fenoxaprop-P, fluazifop-P, haloxyfop-P, metamifop, propaquizafop or quizalofop-P. As a further example, glufosinate may be used in enantiomerically enriched or enantiopure form, also known as glufosinate-P.

Those skilled in the art will recognize that any derivative of the above mentioned herbicides and/or safeners can be used in the practice of the invention, for example agriculturally suitable salts and esters.

The herbicides and/or safeners, or the herbicidal compositions comprising them, can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in any case, they should ensure the finest possible distribution of the active ingredients described herein.

The herbicidal compositions comprise an herbicidal effective amount of at least one of the acetyl-Coenzyme A carboxylase-inhibiting herbicides and potentially other herbicides and/or safeners and auxiliaries which are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, optionally colorants and, for seed formulations, adhesives. The person skilled in the art is sufficiently familiar with the recipes for such formulations.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon^{®} SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulations. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following: mineral oil fractions of medium to high boiling point, such as kerosene and diesel oil, furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Suitable carriers include liquid and solid carriers. Liquid carriers include e.g. non-aqeuos solvents such as cyclic and aromatic hydrocarbons, e.g. paraffins, tetrahydronaphthalene, alkylated naphthalenes and their derivatives, alkylated benzenes and their derivatives, alcohols such as methanol, ethanol, propanol, butanol and cyclohexanol, ketones such as cyclohexanone, strongly polar solvents, e.g. amines such as N-methylpyrrolidone, and water as well as mixtures thereof. Solid carriers include e.g. mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF AG), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denaturated proteins, polysaccharides (e.g. methylcellulose), hydrophobically modified starches, polyvinyl alcohol (Mowiol types Clariant), polycarboxylates (BASF AG, Sokalan types), polyalkoxylates, polyvinylamine (BASF AG, Lupamine types), polyethyleneimine (BASF AG, Lupasol types), polyvinylpyrrolidone and copolymers thereof.

Powders, materials for broadcasting and dusts can be prepared by mixing or concomitant grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water. To prepare emulsions, pastes or oil dispersions, the herbicidal compositions, either as such or dissolved in an oil or solvent, can be homogenized in water by means of a wetting agent, tackifier, dispersant or emulsifier. Alternatively, it is also possible to prepare concentrates comprising active compound, wetting agent, tackifier, dispersant or emulsifier and, if desired, solvent or oil, which are suitable for dilution with water.

### Methods of controlling weeds

Herbicide-tolerant plants described herein may be used in conjunction with an herbicide to which they are tolerant. Herbicides may be applied to the plants using any techniques known to those skilled in the art. Herbicides may be applied at any point in the plant cultivation process. For example, herbicides may be applied pre-planting, at planting, pre-emergence, post-emergence or combinations thereof.

Herbicide compositions hereof can be applied, e.g., as foliar treatments, soil treatments, seed treatments, or soil drenches. Application can be made, e.g., by spraying, dusting, broadcasting, or any other mode known useful in the art.

Herbicides may be used to control the growth of weeds that may be found growing in the vicinity of the herbicide-tolerant plants. To this end an herbicide may be applied to a plot in which the herbicide-tolerant plants are growing in vicinity to weeds. An herbicide to which the herbicide-tolerant plant is tolerant may then be applied to the plot at a concentration sufficient to kill or inhibit the growth of the weed. Concentrations of herbicide sufficient to kill or inhibit the growth of weeds are known in the art.

Having now described the present invention in detail, the same will be more clearly understood by reference to the following examples, which are included herewith for purposes of illustration only and are not intended to be limiting of the invention.

### Use of Tissue Culture for Selection of Herbicide

Herbicide tolerant crops offer farmers additional options for weed management. Currently, there are genetically modified (GMO) solutions available in some crop systems. Additional, mutational techniques have been used to select for altered enzyme, activities or structures that confer herbicide resistance such as the current CLEARFIELD° solutions from BASF. In the US, CLEARFIELD Rice is the premier tool for managing red rice in infested areas (USDA-ARS, 2006); however, gene flow between red rice and CLEARFIELD Rice represents a considerable risk for the AHAS tolerance since out-crossing, has been reported at up to 170 F1 hybrids/ha (Shivrain et al, 2007). Stewardship guidelines including, amongst many other aspects, alternation non CLEARFIELD Rice can limit CLEARFIELD Rice market penetration. The generation of cultivated rice with tolerance to a different mode of action (MOA) graminicides would reduce these risks and provide more tools for weed management.

One enzyme that is already a target for many different graminaceous herbicides is acetyl CoA carboxylase (ACCase, EC 6.4.1.2), which catalyzes the first committed step in fatty acid (FA) biosynthesis. Aryloxyphenoxypropionate (APP or FOP) and cyclohexanedione (CHD or DIM) type herbicides are used post-emergence in dicot crops, with the exception of cyhalofop-butyl which is selective in rice to control grass weeds. Furthermore, most of these herbicides have relatively low persistence in soil and provide growers with flexibility for weed control and crop rotation. Mutations in this enzyme are known that confer tolerance to specific sets of FOPS and/or DIMS (Liu et al, 2007; Delye et a1, 2003, 2005).

Tissue culture offers an alternative approach in that single clumps of callus represent hundreds or even thousands of cells, each of which can be selected for a novel trait such as herbicide resistance (Jain, 2001). Mutations arising spontaneously in tissue culture or upon some kind of induction can be directly selected in culture and mutated events selected.

The exploitation of somaclonal variation that is inherent to *in vitro* tissue culture techniques has been a successful approach to selectively generate mutations that confer DIM and FOP tolerance in corn (Somers, 1996; Somers et al., 1994; Marshal et al., 1992; Parker et al., 1990) and in seashore paspalum (Heckart et al, 2009). In the case of maize, the efficiencies of producing regenerable events can be calculated. In Somers et al, 1994, sethoxydim resistant maize plants were obtained using tissue culture selection. They utilized 100 g of callus and obtained 2 tolerant lines following stepwise selection at 0.5, 1.0, 2.0, 5.0 and 10 µM sethoxydim. A calculated mutation rate in their protocol would be 2 lines/100 g of callus or 0.02 lines/g.

In the case of seashore paspalum, Heckert directly utilized a high level of sethoxydim and recovered 3 regenerable lines in approx 10,000 callus pieces or, essentially, a 0.03 % rate. While not comparable, these numbers will be later used for comparison with rice tissue culture mutagenesis. In the maize work, calli were constantly culled at each selection stage with only growing callus being transferred; however, in the case of seashore paspalum, all calli were transferred at each subculture.ACCase genes as selectable markers:

Plant transformation involves the use of selectable marker genes to identify the few transformed cells or individuals from the larger group of non-transformed cells or individuals. Selectable marker genes exist, but they are limited in number and availability. Alternative marker genes are required for stacking traits. In addition, the use of a selectable marker gene that confers an agronomic trait (i.e. herbicide resistance) is often desirable. ACCase genes are disclosed herein as selectable markers that can be added to the current limited suite of available selectable marker genes. Any of the mutants described herein can be introduced into a plasmid with a gene of interest and tranformed into the whole plant, plant tissue or plant cell for use as selectable markers. A detailed method is outlined in example 7 below. The selectable markers described herein may be utilized to produce events that confer field tolerance to a given group of herbicides and other where cross protection has been shown (i.e., FOP's).

Modern, high throughput plant transformation systems require an effective selectable marker system; however, there is a limited number available that are acceptable in the market. Therefore, selection systems which also convey a commercial trait are always valuable. The system described herein is an effective selection system in/for plant cells which also encode for an herbicide tolerance trait suitable for use in any monocotyledonous crop.

A method for selecting a tranformed plant is described herein which comprises introducing a nucleic acid molecule encoding a gene of interest into a plant cell, wherein the nucleic acid molecule further encodes a mutant acetyl-Coenzyme A carboxylase (ACCase) in which the amino acid sequence differs from an amino acid sequence of an ACCase of a corresponding wild-type rice plant at one amino acid position; and contacting the plant cells with an ACCase inhibitor to obtain the transformed plant, wherein said mutant ACCase confers upon the transformed plant increased herbicide tolerance as compared to the corresponding wild-type variety of the plant when expressed therein.

A method of marker-assisted breeding is described herein, the method comprising breeding a herbicide-tolerant plant as described herein with a second plant; and contacting progeny of the breeding step with an ACCase inhibitor to obtain the progeny comprising said mutant ACCase; wherein said mutant ACCase confers upon the progeny plant increased herbicide tolerance as compared to the second plant.

A single ACCase gene can be linked to a single gene of interest. The ACCase gene may be linked upstream or downstream of the gene of interest.

The use of ACCase nucleic acid and protein as described above in diagnostic assays is described herein. The diagnostic uses for selectable markers described herein can be employed to identify ACCase gene. Diagnostic methods can include PCR methodologies, proteins assays, labeled probes, and any other standard diagnostic methods known in the art.

### EXAMPLES

### EXAMPLE 1

### Tissue culture conditions

An *in vitro* tissue culture mutagenesis assay has been developed to isolate and characterize plant tissue (e.g., rice tissue) that is tolerant to acetyl-Coenzyme A carboxylase inhibiting herbicides, e.g., tepraloxydim, cycloxydim, and sethoxydim. The assay utilizes the somaclonal variation that is found in *in vitro* tissue culture. Spontaneous mutations derived from somaclonal variation can be enhanced by chemical mutagenesis and subsequent selection in a stepwise manner, on increasing concentrations of herbicide.

Tissue culture conditions for encouraging growth of friable, embryogenic rice callus that is regenerable are described herein. Calli were initiated from 4 different rice cultivars encompassing both Japonica (Taipei 309, Nipponbare, Koshihikari) and Indica (Indica 1) varieties. Dehusked seed were surface sterilized in 70% ethanol for approximately 1 min followed by 20% commercial Clorox bleach for 20 minutes. Seeds were rinsed with sterile water and plated on callus induction media. Various callus induction media were tested. The ingredient lists for the media tested are presented in Table 2.

**Table 2**

| **Ingredient** | **Supplier** | **R001M** | **R025M** | **R026M** | **R327M** | **R008M** | **MS711R** |
|---|---|---|---|---|---|---|---|
| B5 Vitamins | Sigma | | | | | 1.0 X | |
| MS salts | Sigma | | | 1.0 X | 1.0 X | 1.0 X | 1.0 X |
| MS Vitamins | Sigma | | | 1.0 X | 1.0 X | | |
| N6 salts | Phytotech | 4.0 g/L | 4.0g/L | | | | |
| N6 vitamins | Phytotech | 1.0 X | 1.0 X | | | | |
| L-Proline | Sigma | 2.9 g/L | 0.5 g/L | | | | 1.2 g/L |
| Casamino Acids | BD | 0.3 g/L | 0.3 g/L | 2 g/L | | | |
| Casein Hydrolysate | Sigma | | | | | | 1.0 g/L |
| L-Asp Monohydrate | Phytotech | | | | | | 150 mg/L |
| Nicotinic Acid | Sigma | | | | | | 0.5 mg/L |
| Pyridoxine HCl | Sigma | | | | | | 0.5 mg/L |
| Thiamine HCl | Sigma | | | | | | 1.0 mg/L |
| Myo-inositol | Sigma | | | | | | 100 mg/L |
| MES | Sigma | 500 mg/L | 500 mg/L | 500 mg/L | 500 mg/L | 500 mg/L | 500 mg/L |
| Maltose | VWR | 30 g/L | 30 g/L | 30 g/L | 30 g/L | | |
| Sorbitol | Duchefa | | | 30 g/L | | | |
| Sucrose | VWR | | | | | 10 g/L | 30 g/L |
| NAA | Duchefa | | | | | 50 µg/L | |
| 2,4-D | Sigma | 2.0 mg/L | | | | | 1.0 mg/L |
| MgCl_{2·}6H₂O | VWR | | | | | 750 mg/L | |
| →pH | | 5.8 | 5.8 | 5.8 | 5.8 | 5.8 | 5.7 |
| Gelrite | Duchefa | 4.0 g/L | | | | 2.5 g/L | |
| Agarose Type1 | Sigma | | 7.0 g/L | 10 g/L | 10 g/L | | |
| →Autoclave | | 15 min | 15 min | 15 min | 15 min | 15 min | 20 min |
| Kinetin | Sigma | | 2.0 mg/L | 2.0 mg/L | | | |
| NAA | Duchefa | | 1.0 mg/L | 1.0 mg/L | | | |
| ABA | Sigma | | 5.0 mg/L | | | | |
| Cefotaxime | Duchefa | | 0.1 g/L | 0.1 g/L | 0.1 g/L | | |
| Vancomycin | Duchefa | | 0.1 g/L | 0.1 g/L | 0.1 g/L | | |
| G418 Disulfate | Sigma | | 20 mg/L | 20 mg/L | 20 mg/L | | |

R001M callus induction media was selected after testing numerous variations. Cultures were kept in the dark at 30°C. Embryogenic callus was subcultured to fresh media after 10-14 days.

### EXAMPLE 2

### Selection of herbicide-tolerant calli

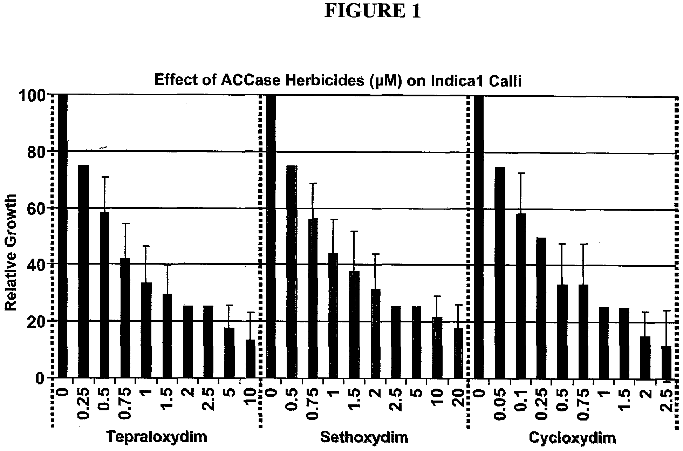

Once tissue culture conditions were determined, further establishment of selection conditions were established through the analysis of tissue survival in kill curves with cycloxydim, tepraloxydim, sethoxydim (Figure 1) or haloxyfop (not shown). Careful consideration of accumulation of the herbicide in the tissue, as well as its persistence and stability in the cells and the culture media was performed. Through these experiments, a sub-lethal dose has been established for the initial selection of mutated material.

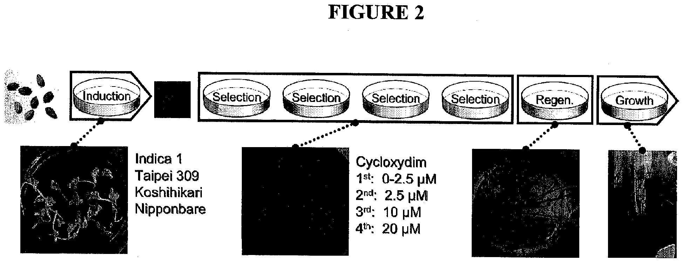

After the establishment of the starting dose of sethoxydim, cycloxydim, tepraloxydim, and haloxyfop in selection media, the tissues were selected in a step-wise fashion by increasing the concentration of the ACCase inhibitor with each transfer until cells are recovered that grew vigorously in the presence of toxic doses (see Figure 2). The resulting calli were further subcultured every 3-4 weeks to R001M with selective agent. Over 26,000 calli were subjected to selection for 4-5 subcultures until the selective pressure was above toxic levels as determined by kill curves and observations of continued culture. Toxic levels were determined to be 50 µM sethoxydim, 20 µM cycloxydim, 2.5 µM tepraloxydim (Figure 1) and 10 µM haloxyfop (not shown).

Alternatively, liquid cultures initiated from calli in MS711R (Table 2) with slow shaking and weekly subcultures. Once liquid cultures were established, selection agent was added directly to the flask at each subculture. Following 2-4 rounds of liquid selection, cultures were transferred to filters on solid R001M media for further growth.

### EXAMPLE 3

### Regeneration of plants

Tolerant tissue was regenerated and characterized molecularly for ACCase gene sequence mutations and/or biochemically for altered ACCase activity in the presence of the selective agent.

Following herbicide selection, calli were regenerated using a media regime of R025M for 10 - 14 days, R026M for ca. 2 weeks, R327M until well formed shoots were developed, and R008S until shoots were well rooted for transfer to the greenhouse (Table 2). Regeneration was carried out in the light. No selection agent was included during regeneration.

Once strong roots were established, M0 regenerants were transplant to the greenhouse in 4" square pots in a mixture of sand, NC Sandhills loamy soil, and Redi-earth (2:4:6) supplemented with gypsum. Transplants were maintained under a clear plastic cup until they were adapted to greenhouse conditions (ca. 1 week). The greenhouse was set to a day/night cycle of 27°C/21 °C (80°F/70°F) with 600W high pressure sodium lights supplementing light to maintain a 14 hour day length. Plants were watered 2-3 times a day depending in the weather and fertilized daily. Rice plants selected for seed increase were transplanted into one gallon pots. As plants approached maturity and prepared to bolt, the pots were placed in small flood flats to better maintain water and nutrient delivery. Plants were monitored for insects and plant health and managed under standard Integrated Pest Management practices.

### EXAMPLE 4

### Sequence analysis

Leaf tissue was collected from clonal plants separated for transplanting and analyzed as individuals. Genomic DNA was extracted using a Wizard® 96 Magnetic DNA Plant System kit (Promega, US Patent Nos. 6,027,945 & 6,368,800) as directed by the manufacturer. Isolated DNA was PCR amplified using one forward and one reverse primer.
Forward Primers:
   OsACCpU5142: 5'-GCAAATGATATTACGTTCAGAGCTG-3' (SEQ ID NO:7)
   OsACCpU5205: 5'-GTTACCAACCTAGCCTGTGAGAAG-3' (SEQ ID NO: 8)
Reverse Primers:
   OsACCpL7100: 5'-GATTTCTTCAACAAGTTGAGCTCTTC-3' (SEQ ID NO: 9)
   OsACCpL7054: 5'-AGTAACATGGAAAGACCCTGTGGC-3' (SEQ ID NO: 10)

PCR amplification was performed using Hotstar Taq DNA Polymerase (Qiagen) using touchdown thermocycling program as follows: 96°C for 15 min, followed by 35 cycles (96°C, 30 sec; 58°C - 0.2 °C per cycle, 30 sec; 72°C, 3 min and 30 sec), 10 min at 72°C.

PCR products were verified for concentration and fragment size via agarose gel electrophoresis. Dephosphorylated PCR products were analyzed by direct sequence using the PCR primers (DNA Landmarks). Chromatogram trace files (.scf) were analyzed for mutation relative to Os05g0295300 using Vector NTI Advance 10™ (Invitrogen). Based on sequence information, two mutations were identified in several individuals. I1,781 (Am)L and D2,078(*Am*)G were present in the heterozygous state. Sequence analysis was performed on the representative chromatograms and corresponding AlignX alignment with default settings and edited to call secondary peaks.

Samples inconsistent with an ACCase mutation were spray tested for tolerance and discarded as escapes. Surprisingly, most of the recovered lines were heterozygous for the I1,781(*Am*)L mutation and resistant events were generated in all tested genotypes using cycloxydim or sethoxydim: Indical (≥18 lines), Taipei 309 (≥14 lines), Nipponbare (≥3 lines), and Koshihikare (≥6 lines). One line was heterozygous for a D2,078(*Am*)G mutation. The D2,078(*Am*)G heterozygote line appeared stunted with narrow leaves, while the I1,781(*Am*)L heterozygotes varied in appearance, but most looked normal relative to their parental genotype. Several escapes were recovered and confirmed by sequencing and spray testing; however, sequencing results of the herbicide sensitive region of ACCase revealed that most tolerant mutants were heterozygous for an I1,781(*Am*)L, A to T mutation (See Table 3). One line, OsARWI010, was heterozygous for a D2,078(*Am*)G, A to G mutation. To date, all recovered plants lacking an ACCase mutation have been sensitive to herbicide application in the greenhouse.

**Table 3: Genotype of Rice Lines Recovered via Tissue Culture Selection**

| **Line** | **Parental Genotype** | **Rice Type** | **Mutation Identified** | **ATCC® Patent Deposit Designation** |
|---|---|---|---|---|
| OsARWI1 | Indica 1 | *indica* | I1781(*Am*)L | PTA-10568 |
| OsARWI3 | Indica 1 | *indica* | I1781(*Am*)L | PTA-10569 |
| OsARWI8 | Indica 1 | *indica* | I1781(*Am*)L | PTA-10570 |
| OsARWI10 | Indica 1 | *indica* | D2078(*Am*)G | NA, sterile |
| OsARWI15 | Indica 1 | *indica* | I1781(*Am*)L | NA |
| OsHPHI2 | Indica 1 | *indica* | I1781(*Am*)L | PTA-10267 |
| OsHPHI3 | Indica 1 | *indica* | I1781(*Am*)L | NA |
| OsHPHI4 | Indica 1 | *indica* | I1781(*Am*)L | NA |
| OsHPHK1 | Koshihikari | *japonica* | I1781(*Am*)L | NA |
| OsHPHK2 | Koshihikari | *japonica* | I1781(*Am*)L | NA |
| OsHPHK3 | Koshihikari | *japonica* | I1781(*Am*)L | NA |
| OsHPHK4 | Koshihikari | *japonica* | I1781(*Am*)L | NA |
| OsHPHK6 | Koshihikari | *japonica* | I1781(*Am*)L | NA |
| OsHPHN1 | Nipponbare | *japonica* | I1781(*Am*)L | PTA-10571 |
| OsHPHT1 | Taipei 309 | *japonica* | I1781(*Am*)L | NA |
| OsHPHT4 | Taipei 309 | *japonica* | I1781(*Am*)L | NA |
| OsHPHT6 | Taipei 309 | *japonica* | I1781(*Am*)L | NA |

### EXAMPLE 5

### Demonstration of herbicide-tolerance

Selected mutants and escapes were transferred to small pots. Wild-type cultivars and 3 biovars of red rice were germinated from seed to serve as controls.

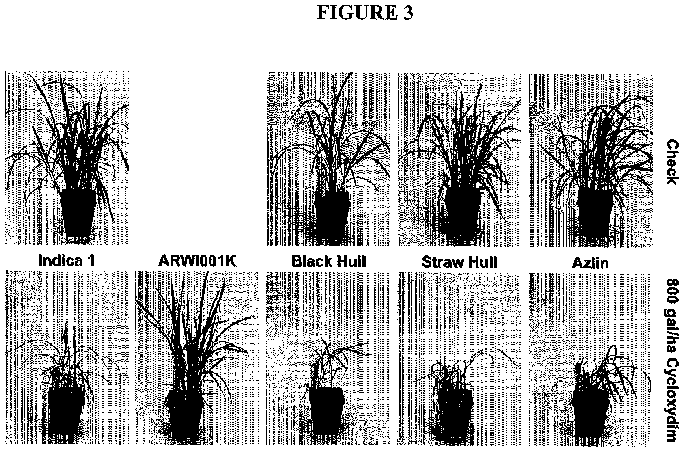

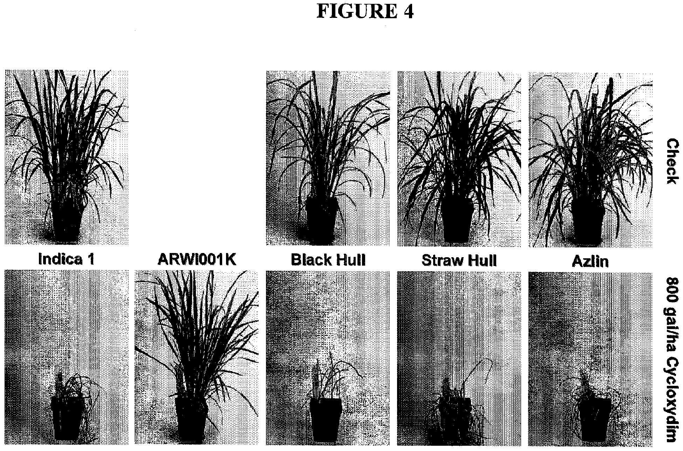

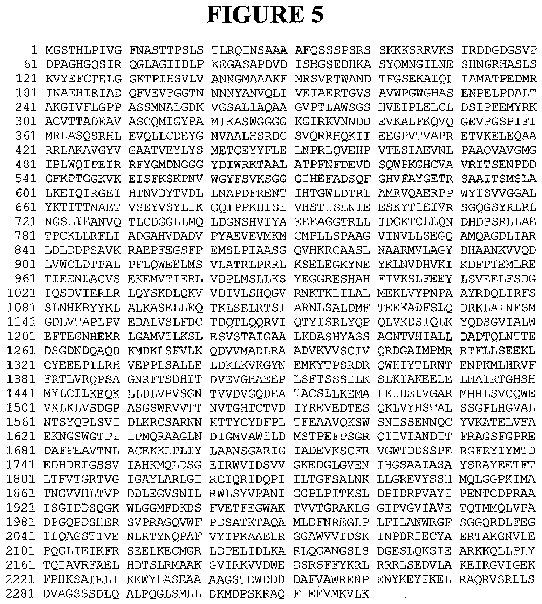

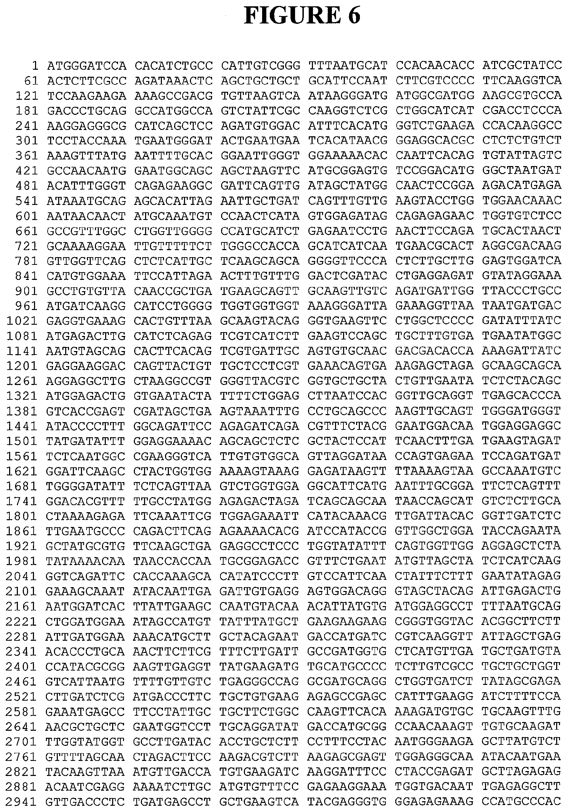

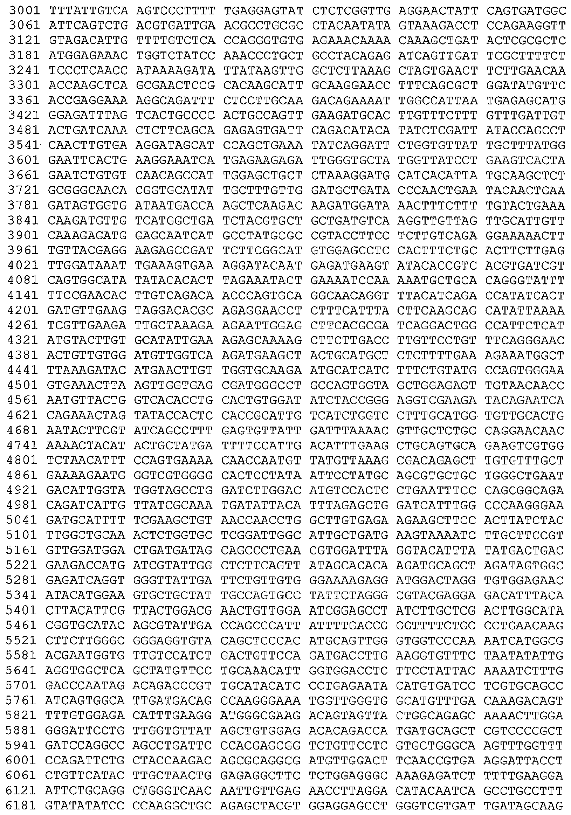

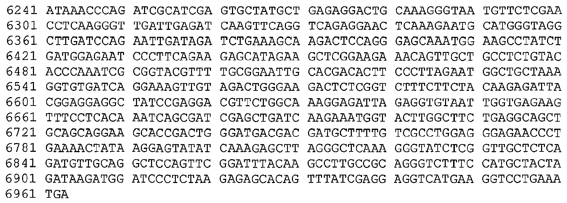

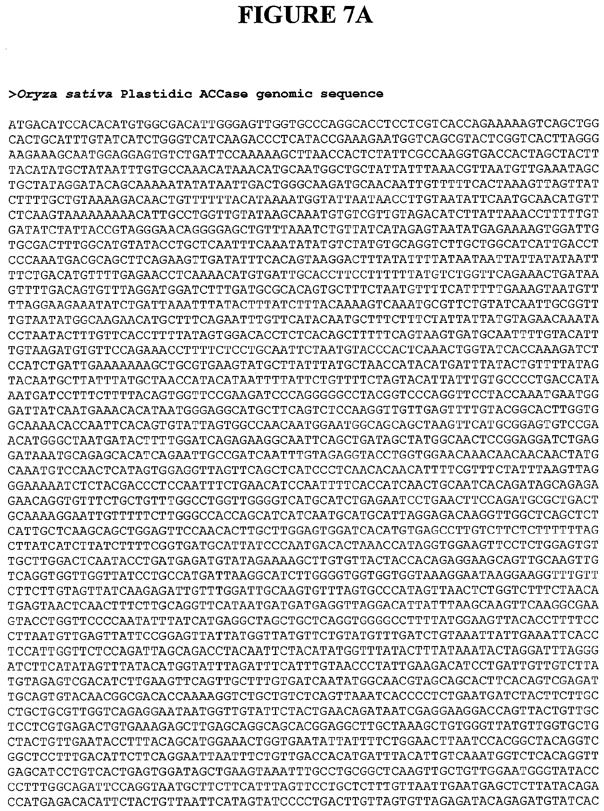

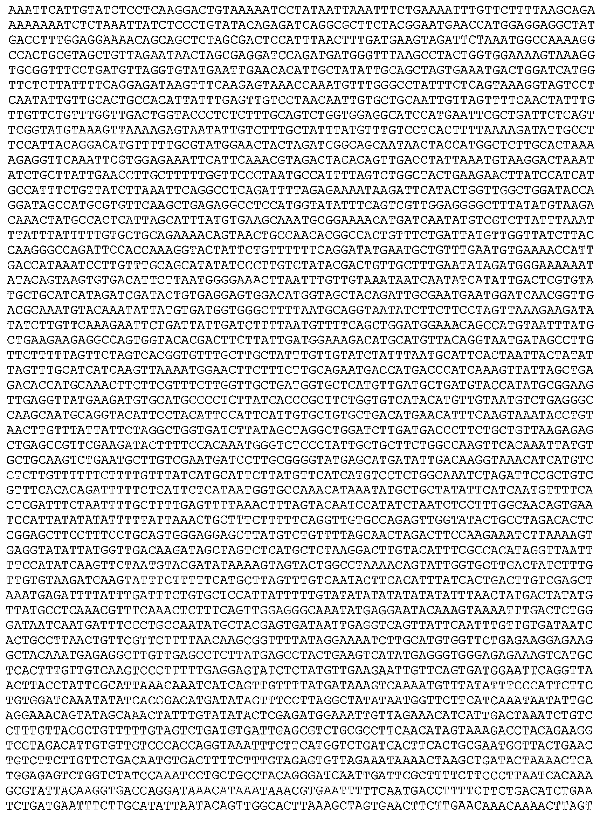

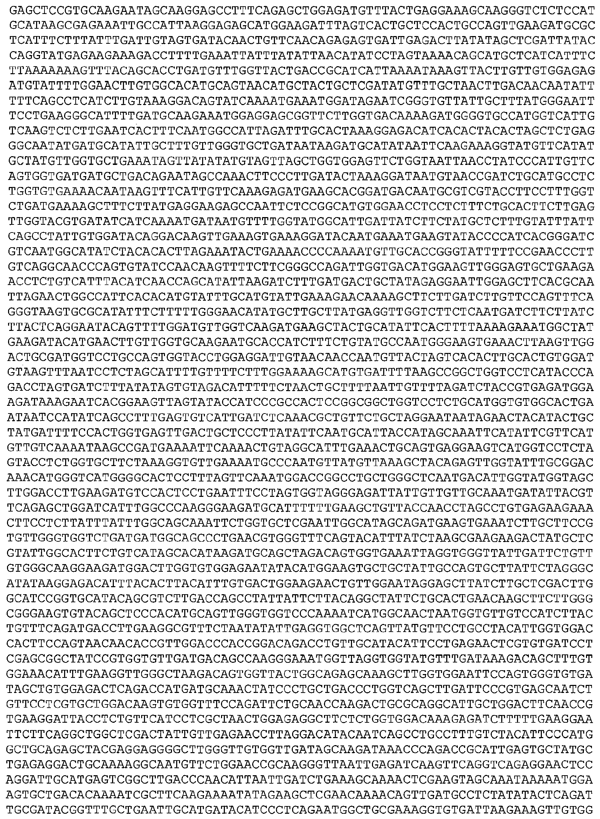

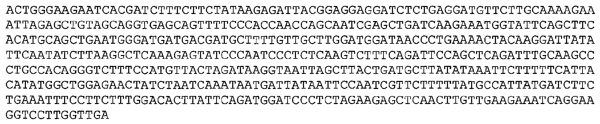

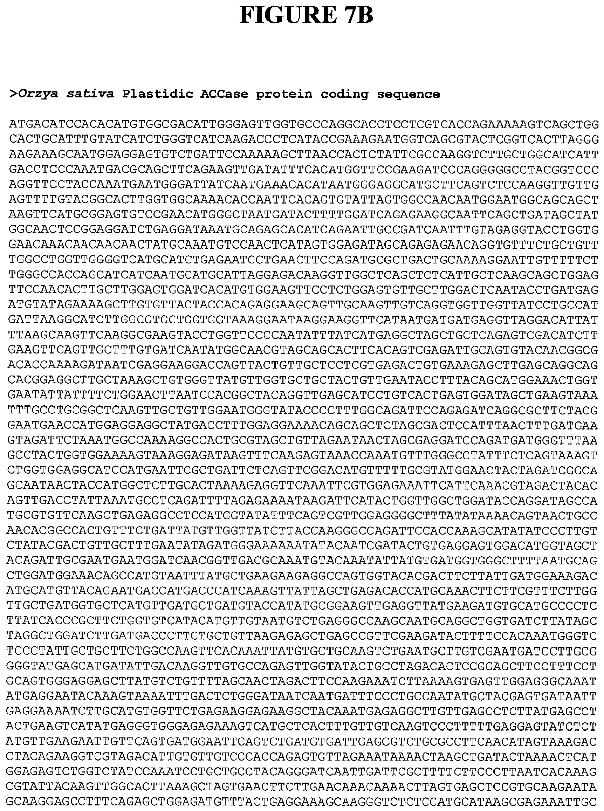

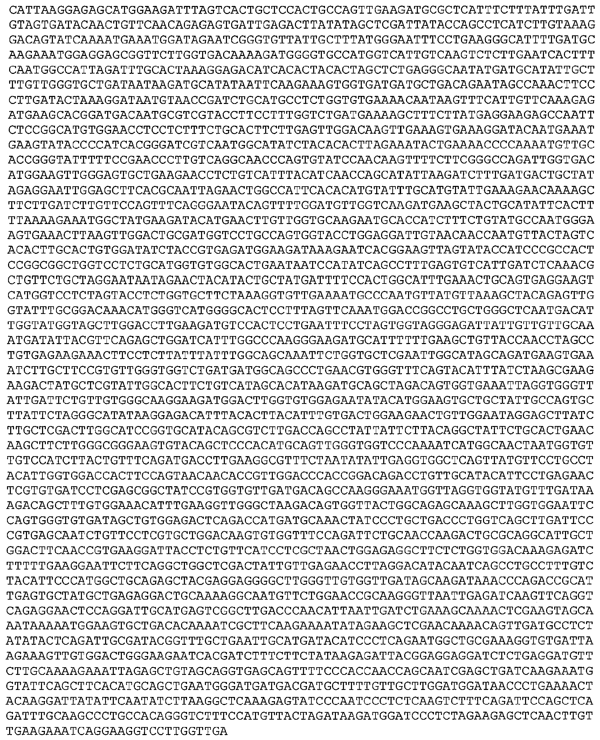

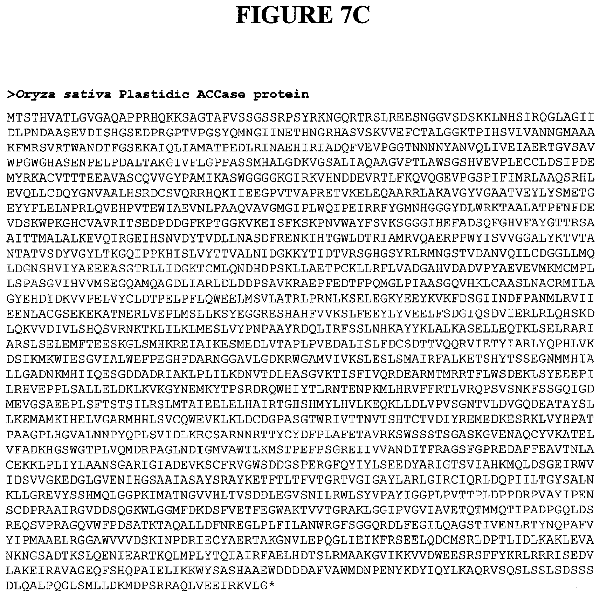

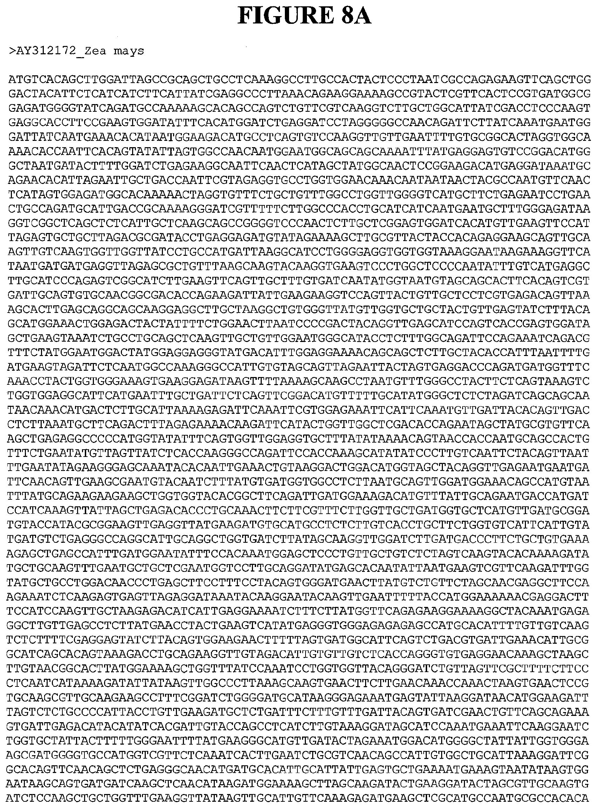

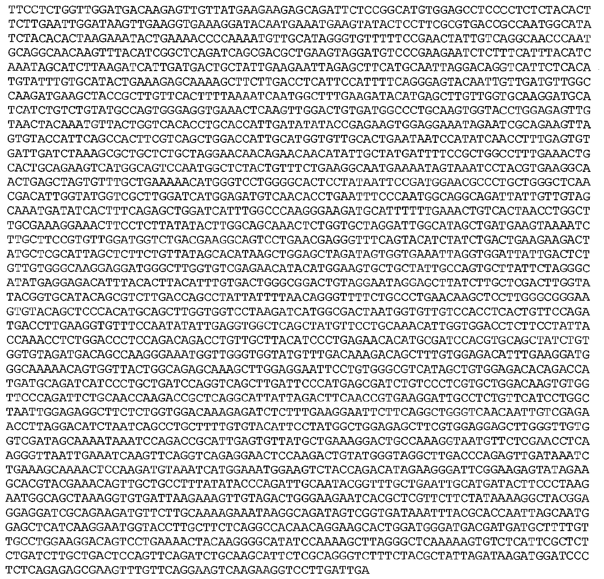

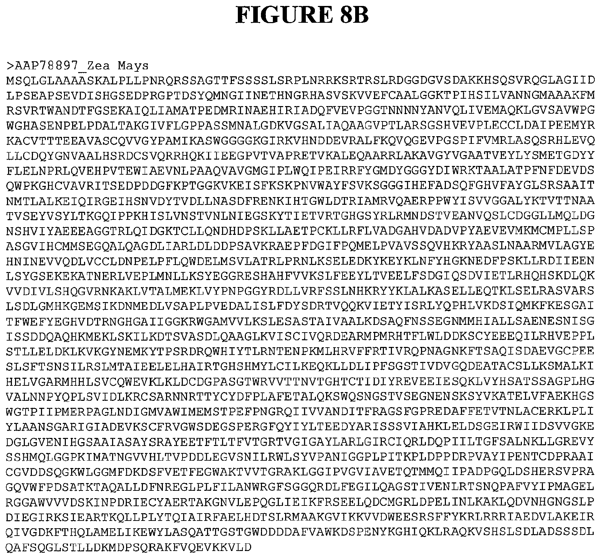

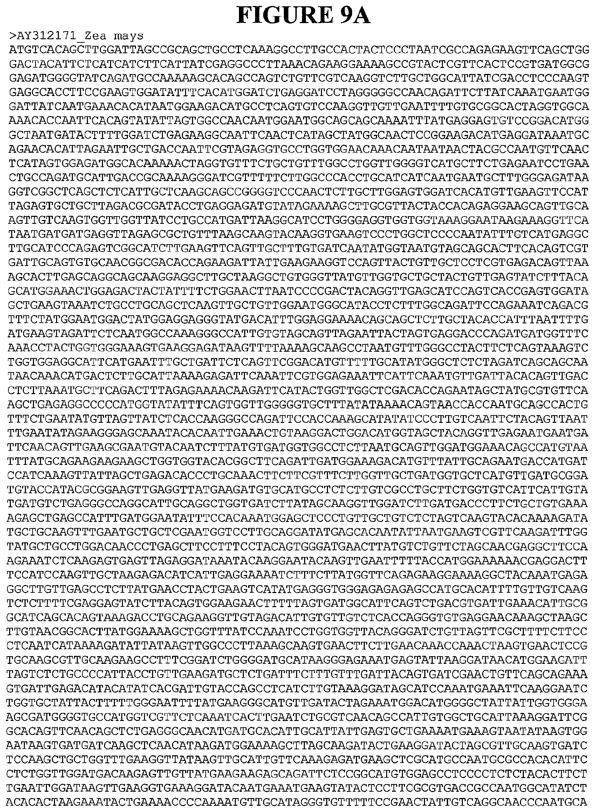

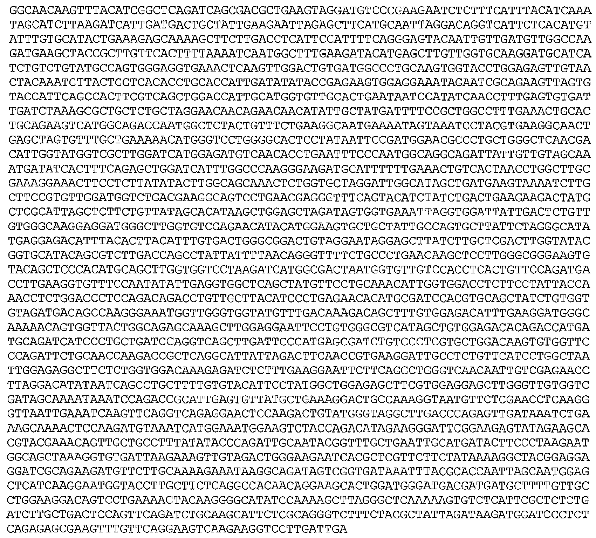

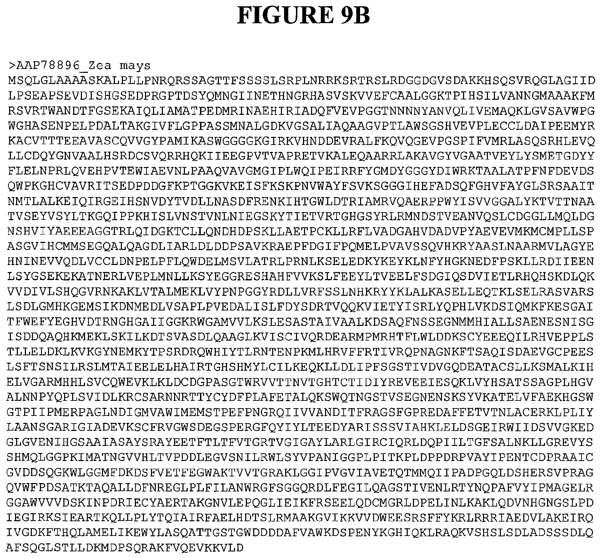

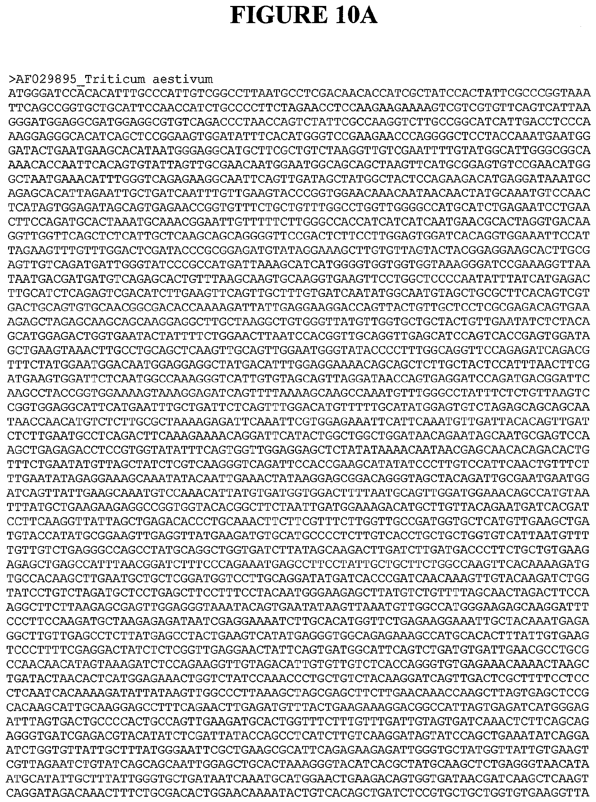

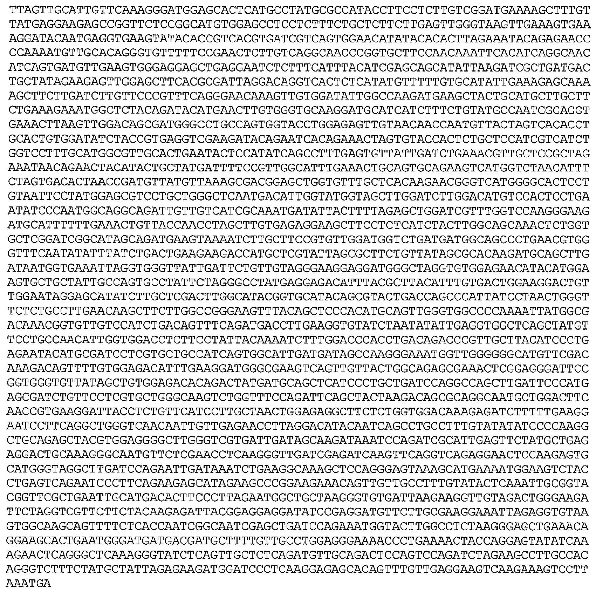

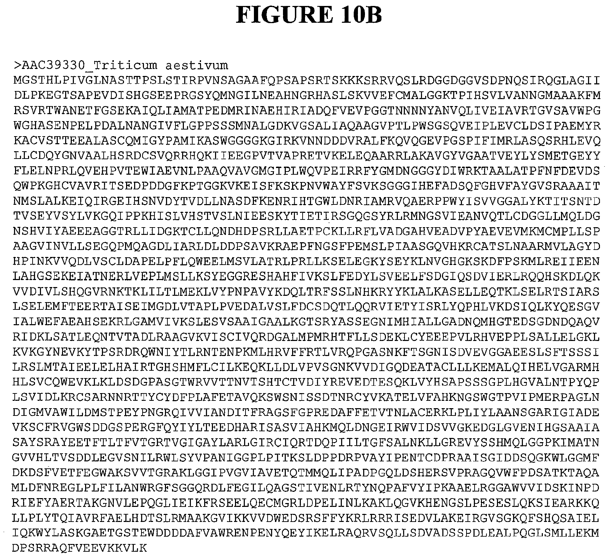

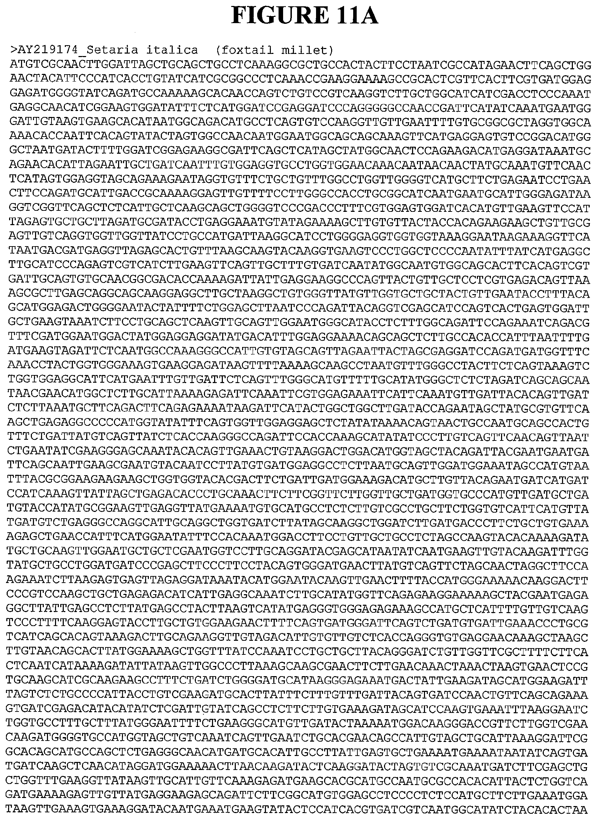

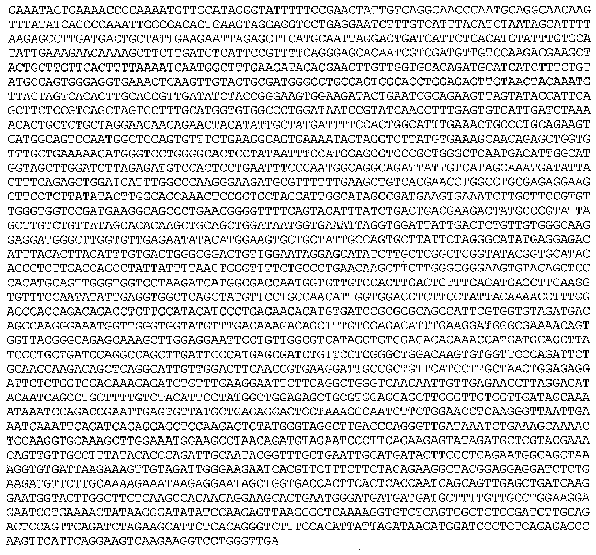

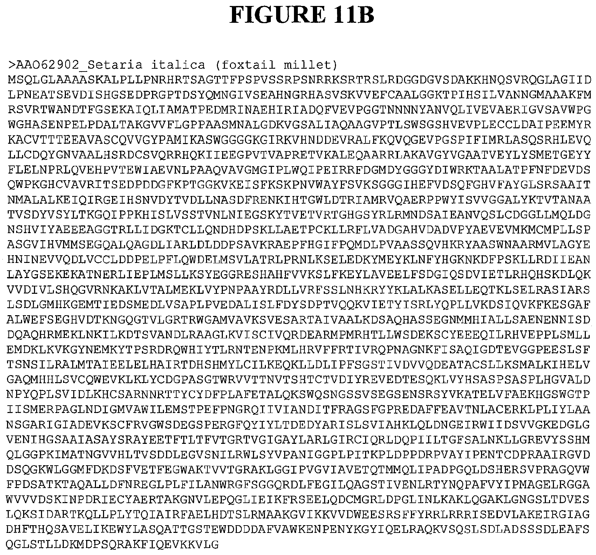

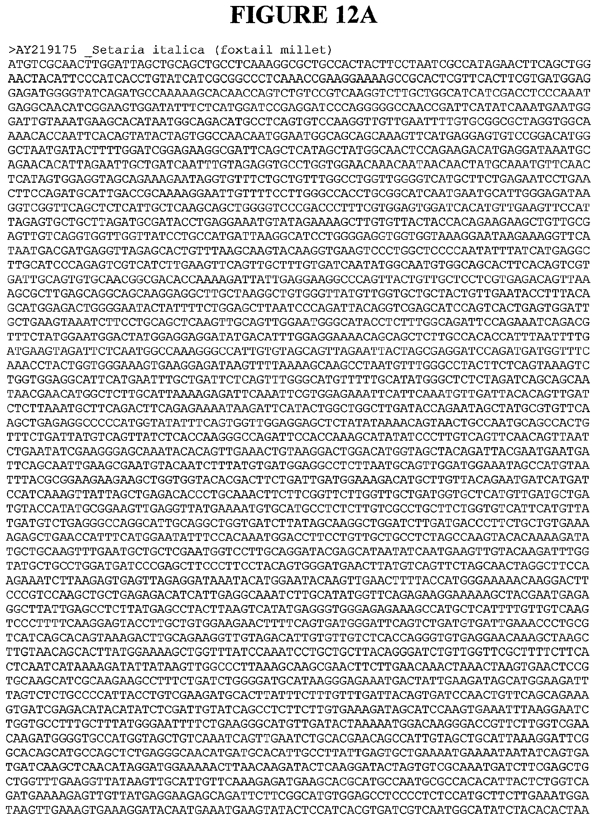

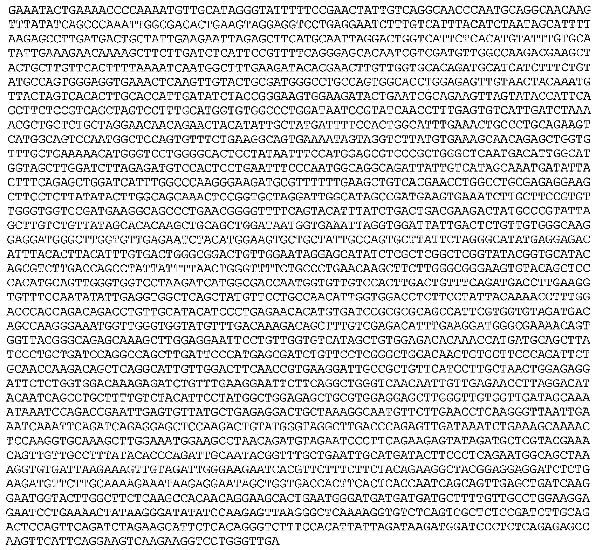

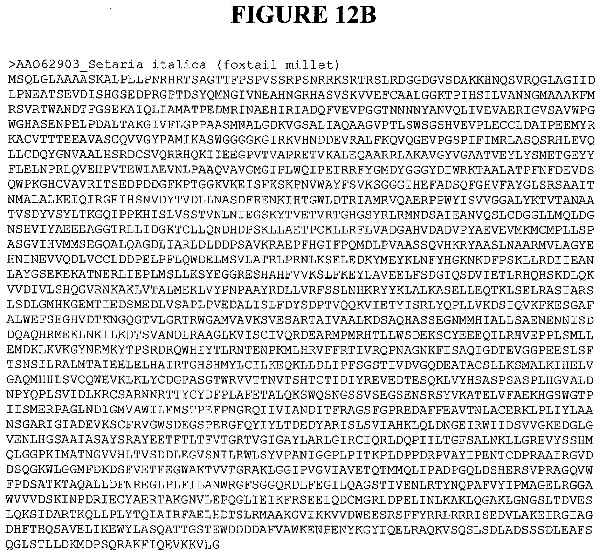

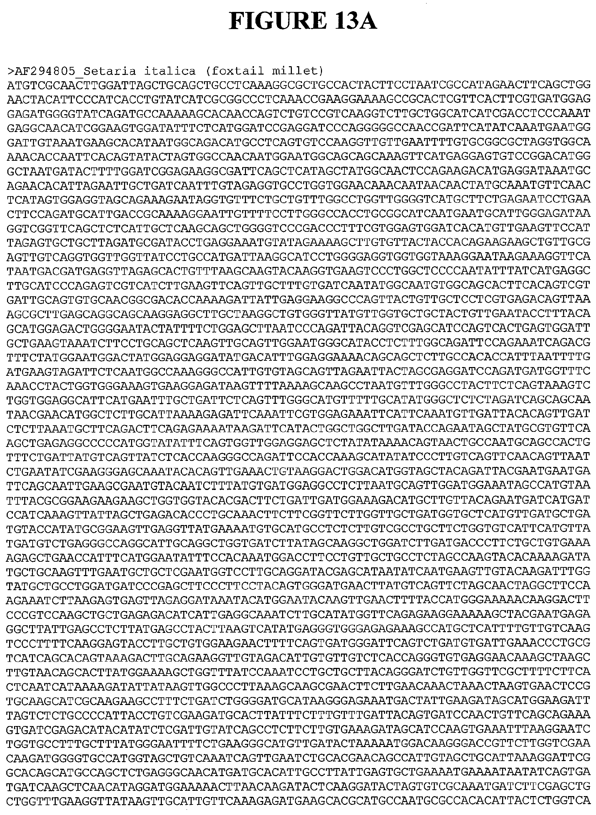

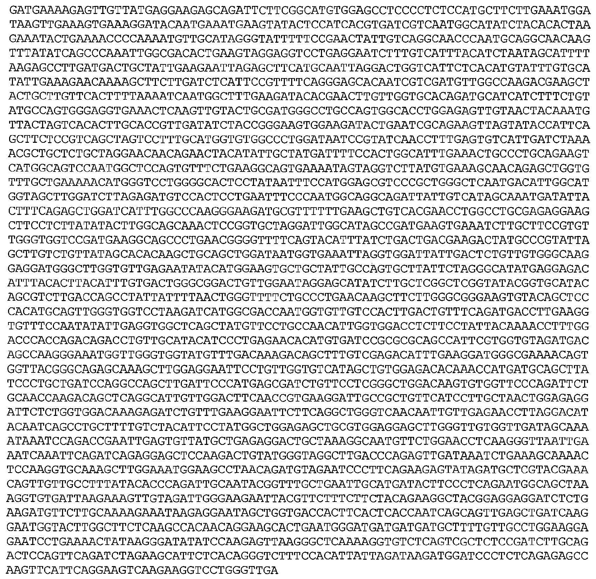

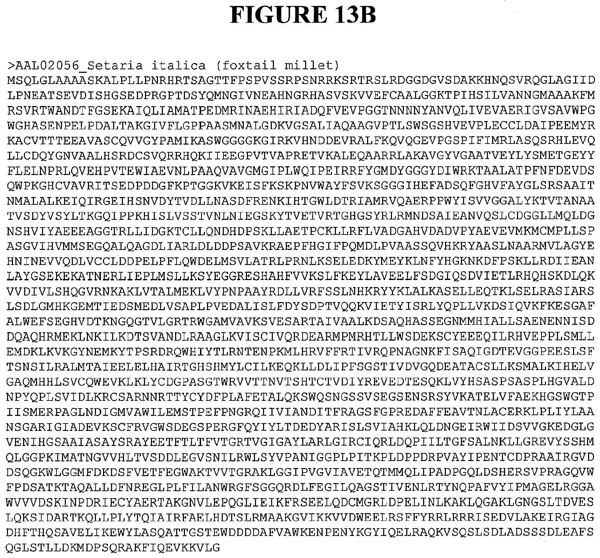

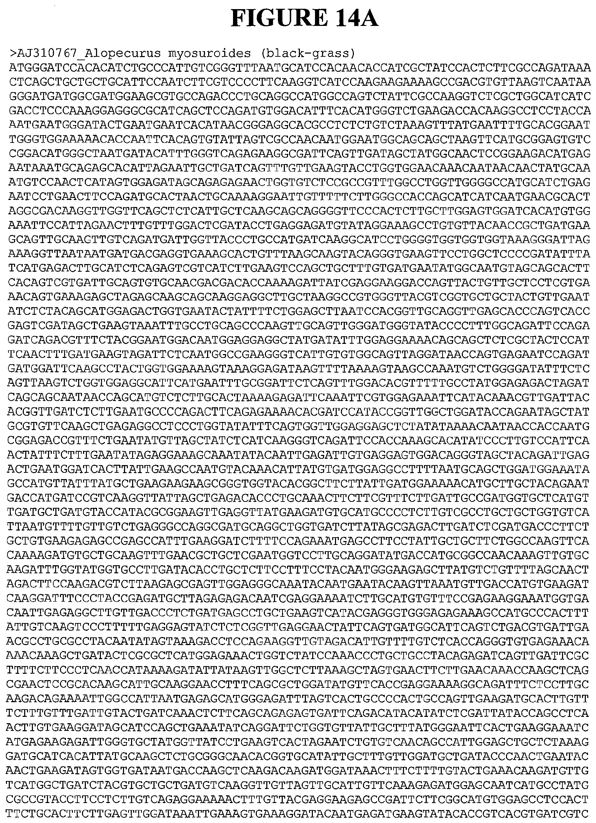

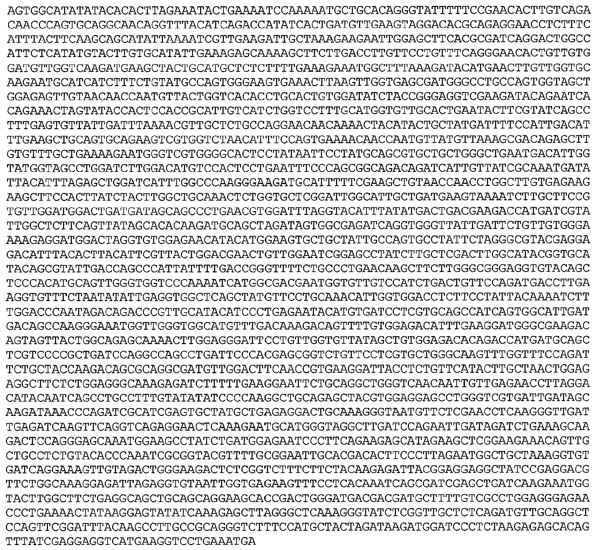

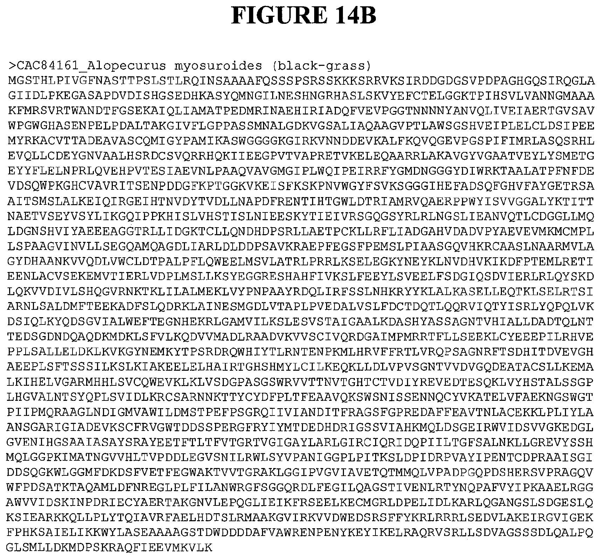

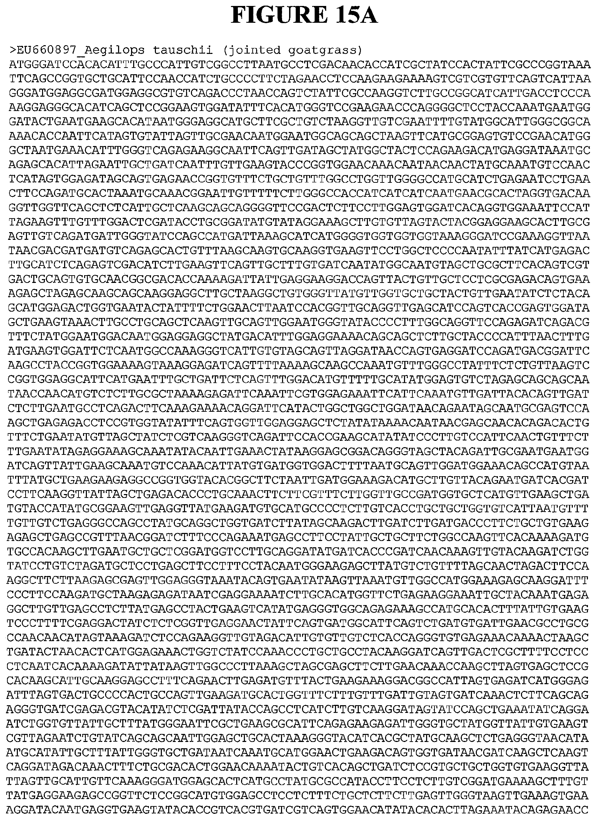

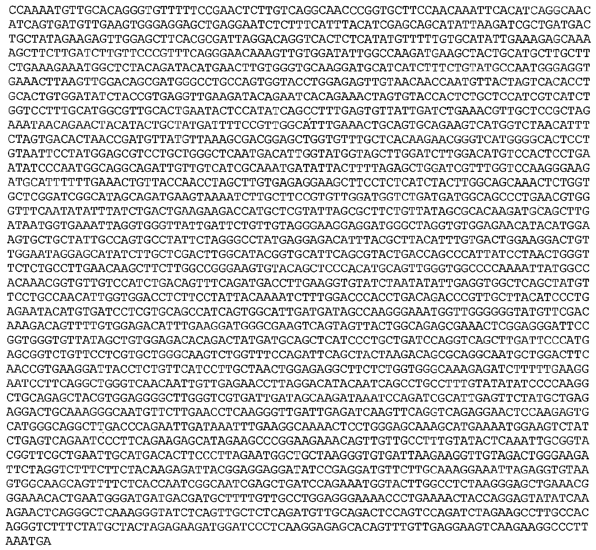

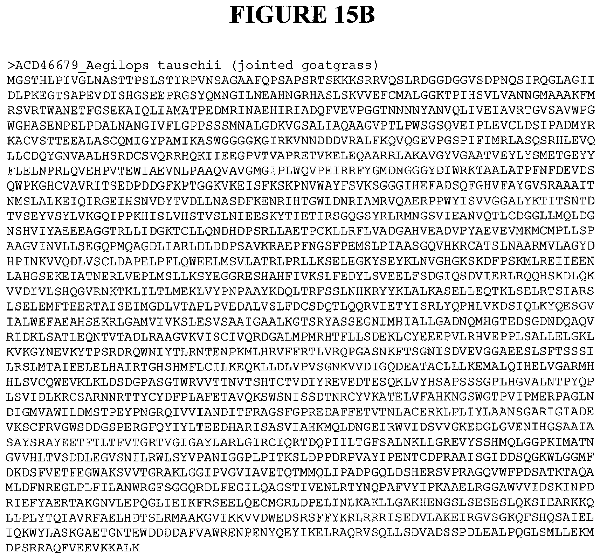

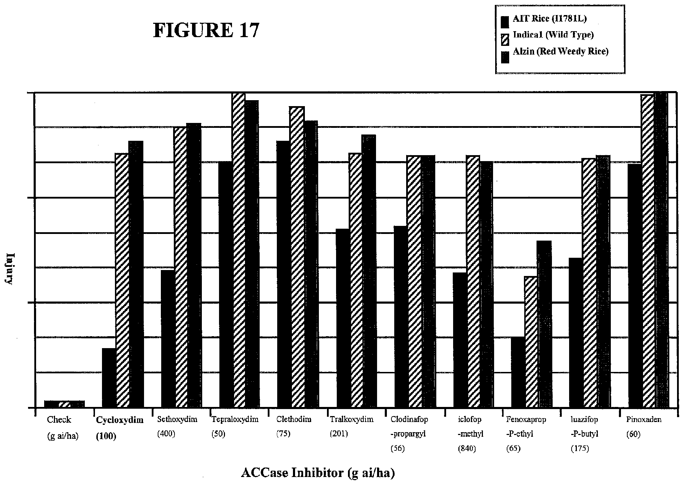

After ca. 3 weeks post-transplant, M0 regenerants were sprayed using a track sprayer with 400-1600 g ai/ha cycloxydim (BAS 517H) supplemented with 0.1% methylated seed oil. After the plants had adapted to greenhouse conditions, a subset were sprayed with 800 g ai/ha cycloxydim. Once sprayed, plants were kept on drought conditions for 24 hours before being watered and fertilized again. Sprayed plants were photographed and rated for herbicide injury at 1 (Figure 3) and 2 weeks after treatment (Figure 4). No injury was observed on plants containing the I1781(*Am*)L heterozygous mutation while control plants and tissue culture escapes (regenerated plants negative for the sequenced mutations) were heavily damaged after treatment (Figures 3 & 4). Figures 5-15 provide nucleic acid and/or amino acid sequences of acetyl-Coenzyme A carboxylase enzymes from various plants. Figure 17 provides a graph showing results for mutant rice versus various ACCase inhibitors.

### EXAMPLE 6

### Herbicide selection using tissue Culture

Media was selected for use and kill curves developed as specified above. For selection, different techniques were utilized. Either a step wise selection was applied, or an immediate lethal level of herbicide was applied. In either case, all of the calli were transferred for each new round of selection. Selection was 4-5 cycles of culture with 3-5 weeks for each cycle. Cali were placed onto nylon membranes to: facilitate transfer (200 micron pore sheets, Biodesign, Saco, Maine). Membranes were cut to fit 100x20 mm Petri dishes and were autoclaved prior to use 25-35 calli (average weight/calli being 22mg) were utilized in every plate. In addition, one set of calli were subjected to selection in liquid culture media with weekly subcultures followed by further selection on semi-solid media.

Mutant lines were selected using cycloxydim or sethoxydim in 4 different rice genotypes. Efficiencies of obtaining mutants was high either based on a percentage of calli that gave rise to a regenerable, mutant line or the number of lines as determined by the gram of tissue utilized. Overall, the mutation frequency compared to seashore paspalum is 5 fold and compared to maize is 2 fold. In some cases, this difference is much higher (>10 fold) as shown in Table 4 below.

**Table 4**

| Genotype | # Calli | Selection | Mutants | Rate | Weight (g) | # / gum callus |
|---|---|---|---|---|---|---|
| Indica 1 | 1865 | Cycloxidim | 3 | 0.161% | 41.04 | 0.07 |
| Indica 1 | 2640 | Sethoxydim | 3 | 0.114% | 58.08 | 0.05 |
| Koshi | 1800 | Cycloxidim | 6 | 0.333% | 39.6 | 0.15 |
| NB | 3400 | Cycloxidim | 1 | 0.029% | 74.8 | 0.01 |
| NB | 725 | Sethoxydim | 0 | 0.000% | 15.95 | 0.00 |
| T309 | 1800 | Cycloxidim | 8 | 0.444% | 36.9 | 0.20 |
| T309 | 1015 | Sethoxydim | 0 | 0.000% | 22.33 | 0.00 |
| Total | 13245 | | 21 | 0.159% | 291.39 | 0.07 |

If the data is analyzed using the criteria of selection, it is possible to see that cylcoxydim selection contributes to a higher rate of mutants isolated than sethoxydim, as shown in Table 5.

**Table 5**

| Genotype | # Calli | Selection | Mutants | Rate | Weight (g) | # / gm callus |
|---|---|---|---|---|---|---|
| Indica 1 | 1865 | Cycloxidim | 3 | 0.161% | 41.03 | 0.07 |
| Koshi | 1800 | Cycloxidim | 6 | 0.333% | 39.6 | 0.15 |
| NB | 3400 | Cycloxidim | 1 | 0.029% | 74.8 | 0.01 |
| T309 | 1800 | Cycloxidim | 8 | 0.444% | 39.6 | 0.20 |
| Total | 8865 | | 18 | 0.203% | 195.03 | 0.09 |
| | | | | | | |
| Indica 1 | 2640 | Sethoxydim | 3 | 0.114% | 58.08 | 0.05 |
| NB | 725 | Sethoxydim | 0 | 0.000% | 15.95 | 0.00 |
| T309 | 1015 | Sethoxydim | 0 | 0.000% | 22.33 | 0.00 |
| Total | 4380 | | 3 | 0.068% | 96.36 | 0.03 |

Using this analysis, the rate for cycloxydim is almost 10 fold higher than either of the previous reports using sethoxydim selection, whereas rates using sethoxydirn selection are similar to those previously reported. Further, 68% of the lines were confirmed as mutants when selection was on cycloxydim compared to 21% of the lines when selection was on sethoxydim. Increases seem to come from using cycloxydim instead of sethoxydim as a selection agent. Further, the use of membranes made transfer of callus significantly easier than moving each piece individually during subcultures. Over 20 mutants were obtained. Fertility appears to be high with the exception of one mutant that has a mutation known to cause a fitness penalty (D2,078(*Am*)G).

### EXAMPLE 7

### Use of mutant ACCase genes as selectable markers in plant transformation

### Methods:

Indical and Nipponbare rice callus transformation was carried out essentially as described in Hiei and Komari (2008) with the exception of media substitutions as specified (see attached media table for details). Callus was induced on R001M media for 4-8 weeks prior to use in transformation. Agrobacterium utilized was LBA4404(pSB1) (Ishida et al. 1996) transformed with RLM185 (L. Mankin, unpublished: contains DsRed and a mutant AHAS for selection), ACC gene containing I1781(*Am*)L, ACC gene containing I1781(*Am*)L and W2027C, ACC gene containing I1781(*Am*)L and I2041(*Am*)N, or ACC gene containing 11781(*Am*)A or wild type which also contains a mutant AHAS gene for selection. Agrobacterium grown for 1-3 days on solid media was suspended in M-LS-002 medium and the OD₆₆₀ adjusted to approximately 0.1. Callus was immersed in the Agrobacterium solution for approximately 30 minutes. Liquid was removed, and then callus was moved to filter paper for co-culture on semi-solid rice cc media. Co-culture was for 3 days in the dark at 24°C. Filters containing rice callus were directly transferred to R001M media containing Timentin for 1-2 weeks for recovery and cultured in the dark at 30°C. Callus was subdivided onto fresh R001M media with Timentin and supplemented with 100µMImazethapyr, 10µM Cycloxydim or 2.5µM Tepraloxydim. After 3-4 weeks, callus was transferred to fresh selection media. Following another 3-4 weeks, growing callus was transferred to fresh media and allowed to grow prior to Taqman analysis. Taqman analysis was for the Nos terminator and was conducted to provide for a molecular confirmation of the transgenic nature of the selected calli. Growth of transgenic calli was measured with various selection agents by subculturing calli on media containing either 10µM Cycloxydim or Haloxyfop, 2.5(µM Tepraloxydim or 100µM Imazethapry. Calli size was measured from scanned images following initial subculture and then after approximately 1 month of growth.

Transformation of maize immature embryos was carried out essentially as described by Lai et al (submitted). Briefly, immature embryos were co-cultured with the same *Agrobacterium* strains utilized for rice transformation suspended in M-LS-002 medium to an OD₆₆₀ of 1.0. Co-culture was on Maize CC medium for 3 days in the dark at 22°C. Embryos were removed from co-culture and transferred to M-MS-101 medium for 4-7 days at 27°C. Responding embryos were transferred to M-LS-202 medium for Imazethapyr selection or M-LS-213 media supplemented with either 1µM Cycloxydim or 0.75µM Tepraloxydim. Embryos were cultured for 2 weeks and growing callus was transferred to a second round of selection using the same media as previous except that Cycloxydim selection was increased to 5µM. Selected calli were transferred to M-LS-504 or M-LS-513 media supplemented with either 5µM Cycloxydim or 0.75µM of Tepraloxydim for and moved to the light (16hr/8hr day/night) for regeneration. Shoots appeared between 2-3 weeks and were transferred to plantcon boxes containing either M-LS-618 or M-LS-613 supplemented with either 5µM Cycloxydim or 0.75µM of Tepraloxydim for further shoot development and rooting. Leaf samples were submitted for Taqman analysis. Positive plants were transferred to soil for growth and seed generation. In the second set of experiments, conditions were identical except that Tepraloxydim selection was decreased to 0.5µM during regeneration and shoot and root formation. In the third set of experiments, Haloxyfop was also tested as a selection agent. In these experiments, 1µM was used throughout for selection

### Results and Discussion:

Transgenic calli were obtained from Indical rice transformation experiments using ACC gene containing I1781(*Am*)L and W2027(*Am*)C, and ACC gene containing I1781(*Am*)L and I2041(*Am*)N. One callus was obtained from ACC gene containing I1781(*Am*)L and W2027(*Am*)C following Tepraloxydim selection and 3 calli were obtained from ACC gene containing 11781(*Am*)L and I2041(*Am*)N. One callus was obtained from ACC gene containing I1781(*Am*)L and I2041(*Am*)N using Cycloxydim selection. Nos Taqman showed that all of these calli were transgenic. Calli were screened for growth under various selection agents including Imazethapry (Pursuit - P) for the mutant AHAS selectable marker.

As can be observed in Table 6, the double mutant constructs allowed for growth on both Cycloxydim and Tepraloxydim in addition to Haloxyfop. The levels utilized in these growth experiments are inhibitory for wild type material.

**Table 6: Growth of transgenic Indical callus on various selection media. Growth was measured as a % change in size following 1 month of culture on the selection media.**

| **Selection µM** | | | | |
|---|---|---|---|---|
| **Construct** | **H10** | **C10** | **T2.5** | **P100** |
| **I1781(*Am*)L, W2027(*Am*)C** | 1669% | 867% | 1416% | 739% |
| **I1781(*Am*)L, I2041(*Am*)N** | 1613% | 884% | 1360% | 634% |

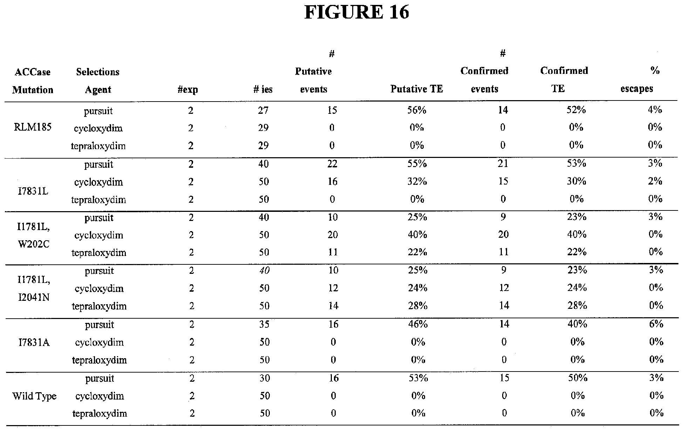

Results from the first set of maize experiments reveal that both the single of the double mutant can be used to select for Cycloxydim resistance or both Cylcoxydim or Tepraloxydim resistance at a relatively high efficiency (Figure 16).

Efficiencies between selection agents was relatively comparable in these experiments with maybe a slight decrease in the overall efficiency with the single mutant on Cycloxydim compared to Pursuit selection. However, the double mutant may have a slight increased efficiency. The escape rate - the percentage of non-confirmed putative events - was lower for Cycloxydim or Tepraloxydim. Further, under the conditions described, it was possible to differentiate between the single and double mutants using Tepraloxydim selection.

Similar results have been obtained in the second set of experiments (not shown). In the third set of experiments, Haloxyfop is also an efficient selectable marker for use in transformation with either the single or the double mutant (not shown).

The single mutant is useful for high efficiency transformation using Cycloxydim or Haloxyfop selection. It should also be useful for other related compounds such as Sethoxydim. The double mutant is useful for these selection agents with the addition that Tepraloxydim can be used. The single and the double mutant can be used in a two stage transformation in that the single mutant can be differentiated from the double with Tepraloxydim selection. In combination with other current BASF selection markers, these give two more options for high efficiency transformations of monocots and maize in particular.

Herbicide tolerance phenotypes as described herein have also been exhibited by ACCase-inhibitor tolerant rice plants hereof, in the field under 600 g/ha cycloxydim treatment (data not shown).

## Claims

1. A method for screening for herbicide-tolerant mutants of a monocot plastidic ACCase comprising:
a. providing plant cells or tissue from a monocot plant; and
b. culturing said plant cells or tissue in a tissue culture environment in the presence of cycloxydime;
wherein the cycloxydim-tolerant plant cells or tissues recovered after step (b) are cycloxydimtolerant due to a mutation in the monocot plastidic ACCase thereof, that was not present in the monocot plastidic ACCase prior to the culturing in step (b).

2. A method for screening for herbicide-tolerant mutants of a monocot plastidic ACCase comprising:
a. providing monocot host cells or tissue deficient in an endogenous plastidic ACCase activity, said host cells or tissue comprising an exogenous monocot plastidic ACCase; and
b. culturing said host cells or tissue in a tissue culture environment in the presence of cycloxydime;
wherein the exogenous Monocot plastidic ACCase of cycloxydim-tolerant plant cells or tissues recovered after step (b) is cycloxydim-tolerant due to a mutation therein that was not present in the exogenous monocot plastidic ACCase prior to the culturing in step (b).

3. The method according to claim 1 or 2, wherein the cycloxydim-tolerant mutational frequency is greater than 0.03 % cycloxydim-tolerant clones per number of individual cultures cultured in step (b).

4. The method according to any one of claims 1-3, wherein said tolerant plant cells or tissues comprise two or more mutations not present in the monocot plastidic ACCase prior to culturing in the presence of the herbicide.

5. The method according to any one of claims 1 to 4, wherein the culturing in the presence of cycloxydim in step (b) is performed in step-wise increases of cycloxydim concentrations.

6. The method according to any one of claims 1 to 5, wherein said method comprises culturing of cells on a membrane or in semi-solid media.

7. The method according to any one of claims 1 to 6, wherein said method further comprises identification of the at least one mutation not present in the Monocot plastidic ACCase prior to culturing in the presence of the cycloxydim.

8. The method according to any one of claims 1 to 7, wherein said monocot is a plant of the family *Poaceae.*

9. The method according to any one of claims 1 to 8, wherein said monocot is a BEP clade plant.

10. The method according to any one of claims 1 to 9, wherein said Monocot is rice.

11. The method according to claim 2, wherein said exogenous monocot plastidic ACCase of step (a) is a rice plastidic ACCase comprising the amino acid sequence of SEQ ID NO:2.

12. The method according to any one of claims 1 to 10, wherein the method further comprises the step of regenerating a plant from a cycloxydim-tolerant plant cell recovered after step (b).

## Patentansprüche

1. Verfahren zum Screenen auf herbizidtolerante Mutanten einer plastidären Monokotyledonen-ACCase, umfassend:
a. Bereitstellen von Pflanzenzellen oder -gewebe von einer monokotyledonen Pflanze; und
b. Kultivieren der Pflanzenzellen bzw. des Pflanzengewebes in einer Gewebekulturumwelt in Gegenwart von Cycloxydim;
wobei die nach Schritt (b) gewonnenen cycloxydimtoleranten Pflanzenzellen oder -gewebe aufgrund einer Mutation in ihrer plastidären Monokotyledonen-ACCase, die vor dem Kultivieren in Schritt (b) in der plastidären Monokotyledonen-ACCase nicht vorhanden war, cycloxydimtolerant sind.

2. Verfahren zum Screenen auf herbizidtolerante Mutanten einer plastidären Monokotyledonen-ACCase, umfassend:
a. Bereitstellen von Monokotylen-Wirtszellen oder -gewebe, denen/dem endogene plastidäre ACCase-Aktivität fehlt, wobei die Wirtszellen bzw. das Wirtsgewebe eine exogene plastidäre Monokotyledonen-ACCase umfassen/umfasst; und
b. Kultivieren der Wirtszellen bzw. des Wirtsgewebes in einer Gewebekulturumwelt in Gegenwart von Cycloxydim;
wobei die exogene plastidäre Monokotyledonen-ACCase von nach Schritt (b) gewonnenen cycloxydimtoleranten Pflanzenzellen oder -geweben aufgrund einer Mutation darin, die vor dem Kultivieren in Schritt (b) nicht in der exogenen plastidäre Monokotyledonen-ACCase vorhanden war, cycloxydimtolerant sind.

3. Verfahren nach Anspruch 1 oder 2, wobei die Mutationsfrequenz der Cycloxydimtoleranten höher als 0,03% cycloxydimtolerante Klone pro Anzahl in der in Schritt (b) kultivierten Einzelkulturen ist.

4. Verfahren nach einem der Ansprüche 1-3, wobei die toleranten Pflanzenzellen oder -gewebe zwei oder mehr Mutationen umfassen, die vor dem Kultivieren in Gegenwart des Herbizids nicht in der plastidären Monokotyledonen-ACCase vorhanden waren.

5. Verfahren nach einem der Ansprüche 1 bis 4, wobei das Kultivieren in Gegenwart von Cycloxydim in Schritt (b) mit schrittweisen Erhöhungen der Cycloxydimkonzentrationen durchgeführt wird.

6. Verfahren nach einem der Ansprüche 1 bis 5, wobei das Verfahren das Kultivieren der Zellen auf einer Membran oder in halbfesten Medien umfasst.

7. Verfahren nach einem der Ansprüche 1 bis 6, wobei das Verfahren weiterhin das Identifizieren der mindestens einen Mutation, die vor dem Kultivieren in Gegenwart des Cycloxydims in der plastidären Monokotyledonen-ACCase nicht vorhanden war, umfasst.

8. Verfahren nach einem der Ansprüche 1 bis 7, wobei es sich bei der Monokotylen um eine Pflanze der Familie Poaceae handelt.

9. Verfahren nach einem der Ansprüche 1 bis 8, wobei es sich bei der Monokotylen um eine Pflanze der Klade BEP handelt.

10. Verfahren nach einem der Ansprüche 1 bis 9, wobei es sich bei der Monokotylen um Reis handelt.

11. Verfahren nach Anspruch 2, wobei es sich bei der exogenen plastidären Monokotyledonen-ACCase von Schritt (a) um eine plastidäre Reis-ACCase umfassend die Aminosäuresequenz gemäß SEQ ID NO: 2 handelt.

12. Verfahren nach einem der Ansprüche 1 bis 10, wobei das Verfahren weiterhin den Schritt des Regenerierens einer Pflanze aus einer nach Schritt (b) gewonnenen cycloxydimtoleranten Pflanzenzellen umfasst.

## Revendications

1. Méthode de criblage, pour des mutants tolérants vis-à-vis d'herbicides, d'une ACCase plastidique de monocotylédone, comprenant :
a. la fourniture de cellules végétales ou de tissu végétal issus d'une plante monocotylédone ; et
b. la culture desdites cellules végétales ou dudit tissu végétal dans un environnement de culture tissulaire en présence de cycloxydime ;
où les cellules végétales ou les tissus végétaux tolérants vis-à-vis du cycloxydime récupérés après l'étape (b) sont tolérants vis-à-vis du cycloxydime grâce à une mutation dans l'ACCase plastidique de monocotylédone de ceux-ci, qui n'était pas présente dans l'ACCase plastidique de monocotylédone préalablement à la culture dans l'étape (b).

2. Méthode de criblage pour des mutants tolérants vis-à-vis d'herbicides d'une ACCase plastidique de monocotylédone, comprenant :
a. la fourniture de cellules hôtes ou de tissu hôte de monocotylédone déficients en une activité d'ACCase plastidique endogène, lesdites cellules hôtes ou ledit tissu hôte comprenant une ACCase plastidique de monocotylédone exogène ; et
b. la culture desdites cellules hôtes ou dudit tissu hôte dans un environnement de culture tissulaire en présence de cycloxydime ;
où l'ACCase plastidique de monocotylédone exogène des cellules végétales ou des tissus végétaux tolérants vis-à-vis du cycloxydime récupérés après l'étape (b) est tolérante vis-à-vis du cycloxydime grâce à une mutation dans celle-ci, qui n'était pas présente dans l'ACCase plastidique de monocotylédone exogène préalablement à la culture dans l'étape (b).

3. Méthode selon la revendication 1 ou 2, dans laquelle la fréquence mutationnelle de tolérance vis-à-vis du cycloxydime est supérieure à 0,03% des clones tolérants vis-à-vis du cycloxydime par nombre de cultures individuelles cultivées dans l'étape (b).

4. Méthode selon l'une quelconque des revendications 1-3, dans laquelle lesdites cellules végétales ou lesdits tissus végétaux tolérants comprennent deux, ou plus, mutations non présentes dans l'ACCase plastidique de monocotylédone préalablement à la culture en présence de l'herbicide.

5. Méthode selon l'une quelconque des revendications 1 à 4, dans laquelle la culture en présence de cycloxydime dans l'étape (b) est effectuée selon des augmentations par étapes de concentrations en cycloxydime.

6. Méthode selon l'une quelconque des revendications 1 à 5, où ladite méthode comprend la culture de cellules sur une membrane ou dans un milieu semi-solide.

7. Méthode selon l'une quelconque des revendications 1 à 6, où ladite méthode comprend en outre l'identification de la au moins une mutation non présente dans l'ACCase plastidique de monocotylédone préalablement à la culture en présence du cycloxydime.

8. Méthode selon l'une quelconque des revendications 1 à 7, dans laquelle ladite monocotylédone est une plante de la famille des *Poaceae.*

9. Méthode selon l'une quelconque des revendications 1 à 8, dans laquelle ladite monocotylédone est une plante de clade BEP.

10. Méthode selon l'une quelconque des revendications 1 à 9, dans laquelle ladite monocotylédone est le riz.

11. Méthode selon la revendication 2, dans laquelle ladite ACCase plastidique de monocotylédone exogène de l'étape (a) est une ACCase plastidique de riz comprenant la séquence d'acides aminés de SEQ ID n° 2.

12. Méthode selon l'une quelconque des revendications 1 à 10, où la méthode comprend en outre l'étape consistant à régénérer une plante à partir d'une cellule végétale tolérante vis-à-vis du cycloxydime récupérée après l'étape (b).
